(19) Europäisches Patentamt
European Patent Office
Office européen des brevets

(11) Veröffentlichungsnummer: 0 531 883 A1

(12) # EUROPÄISCHE PATENTANMELDUNG

(21) Anmeldenummer: **92115057.9**

(22) Anmeldetag: **03.09.92**

(51) Int. Cl.[5]: **C07D 235/18**, A61K 31/415, C07D 235/12, C07D 235/22, C07D 265/14, C07D 277/66, C07D 473/00, C07D 473/08, C07D 473/30, C07D 473/34, C07D 401/06, C07D 403/06, C07D 471/04, C07D 487/04, C07D 498/04

(30) Priorität: **06.09.91 DE 4129603**

(43) Veröffentlichungstag der Anmeldung:
**17.03.93 Patentblatt 93/11**

(84) Benannte Vertragsstaaten:
**AT BE CH DE DK ES FR GB GR IE IT LI LU NL PT SE**

(71) Anmelder: **Dr. Karl Thomae GmbH**
**Postfach 1755**
**W-7950 Biberach 1(DE)**

(72) Erfinder: **Austel, Volkhard, Dr. Dipl.-Chem.**
**Kapellenweg 7**
**W-7950 Biberach 1(DE)**
Erfinder: **Pieper, Helmut, Dr. Dipl.-Chem.**
**Kapellenweg 5**
**W-7950 Biberach 1(DE)**
Erfinder: **Himmelsbach, Frank, Dr. Dipl.-Chem.**
**Ahornweg 16**
**W-7951 Mittelbiberach(DE)**
Erfinder: **Linz, Günter, Dr. Dipl.-Chem.**
**Drosselweg 14**
**W-7951 Mittelbiberach(DE)**
Erfinder: **Müller, Thomas, Dr. Dipl.-Chem.**
**Gymnasiumstrasse 16**
**W-7950 Biberach 1(DE)**
Erfinder: **Weisenberger, Johannes, Dr. Dipl.-Chem.**
**Haydnweg 5**
**W-7950 Biberach 1(DE)**
Erfinder: **Seewaldt-Becker, Elke, Dr. Dipl.-Bio.**
**Hühnerfeldstrasse 26**
**W-7950 Biberach 1(DE)**

(54) **Kondensierte 5-gliedrige Heterocyclen, Verfahren zu ihrer Herstellung und diese Verbindungen enthaltende Arzneimittel.**

(57) Die Erfindung betrifft cyclische Iminoderivate der allgemeinen Formel

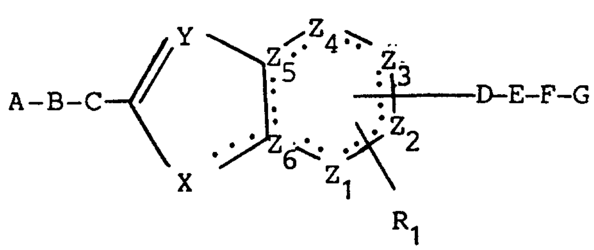

,(I)

in der
A bis G, $R_1$, $Z_1$ bis $Z_6$, X und Y wie im Anspruch 1 definiert sind, deren Tautomere, deren Stereoisomere, deren

EP 0 531 883 A1

Gemische und deren Salze, insbesondere deren physiologisch verträgliche Salze mit anorganischen oder organischen Säuren oder Basen, welche wertvolle pharmakologische Eigenschaften aufweisen, vorzugsweise aggregationshemmende Wirkungen, diese Verbindungen enthaltende Arzneimittel und Verfahren zu ihrer Herstellung.

Die Erfindung betrifft kondensierte 5-gliedrige Heterocyclen der allgemeinen Formel

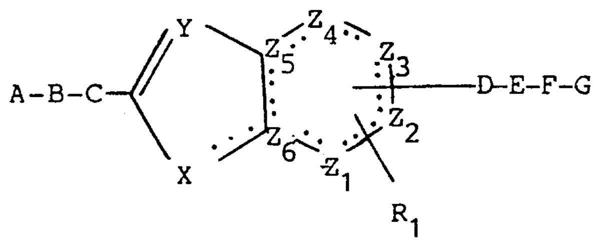

,(I)

deren Tautomere, deren Stereoisomere einschließlich deren Gemische und deren Salze, insbesondere deren physiologisch verträgliche Salze mit anorganischen oder organischen Säuren oder Basen, welche u.a. wertvolle pharmakologische Eigenschaften aufweisen, vorzugsweise aggregationshemmende Wirkungen, diese Verbindungen enthaltende Arzneimittel und Verfahren zu ihrer Herstellung.

In der obigen allgemeinen Formel I bedeutet

$R_1$ ein Wasserstoff-, Fluor-, Chlor- oder Bromatom, eine Alkyl-, Aralkyl-, Aryl-, Heteroaryl-, $R_3O$-, $(R_3)_2N$-, $R_4CO-NR_3$-, Alkylsulfonyl-$NR_3$-, Arylsulfonyl-$NR_3$-, $R_3S$-, $R_3SO$-, $R_3SO_2$- oder $R_5$-Gruppe, wobei

$R_3$ ein Wasserstoffatom, eine Alkylgruppe mit 1 bis 6 Kohlenstoffatomen, eine Aryl-, Heteroaryl-, Aralkyl-, Carboxyalkyl- oder Alkoxycarbonylalkylgruppe,

$R_4$ ein Wasserstoffatom, eine Alkyl- oder Alkoxygruppe mit jeweils 1 bis 6 Kohlenstoffatomen, eine Aryl-, Heteroaryl- oder Aralkylgruppe mit 1 bis 6 Kohlenstoffatomen im Alkylteil und

$R_5$ eine Azetidino-, Pyrrolidino-, Hexamethylenimino- oder Heptamethyleniminogruppe oder eine Piperidinogruppe, in der die Methylengruppe in 4-Stellung durch ein Sauerstoffatom, eine Sulfenyl-, Sulfinyl- oder Sulfonylgruppe oder durch eine durch eine $R_3$-, $R_4CO$-, Alkylsulfonyl- oder Arylsulfonylgruppe substituierte Iminogruppe ersetzt sein kann, wobei $R_3$ und $R_4$ wie vorstehend erwähnt definiert sind, darstellen,

X ein Sauerstoff-, Schwefel- oder Stickstoffatom oder eine -$NR_2$-Gruppe, wobei

$R_2$ ein Wasserstoffatom, eine geradkettige oder verzweigte Alkylgruppe mit 1 bis 15 Kohlenstoffatomen, eine geradkettige oder verzweigte Alkenyl- oder Alkinylgruppe mit jeweils 3 bis 10 Kohlenstoffatomen, wobei die Doppel- oder Dreifachbindung nicht unmittelbar an das Stickstoffatom anschließen kann, eine Cycloalkyl- oder Cycloalkylalkylgruppe mit jeweils 3 bis 7 Kohlenstoffatomen im Cycloalkylteil, eine Aryl- oder Heteroarylgruppe, eine Alkylgruppe mit 2 bis 6 Kohlenstoffatomen, die ab der $\beta$-Position zu dem Stickstoffatom der -$NR_2$-Gruppe durch eine $R_3O$-, $(R_3)_2N$-, $R_4CO-NR_3$-, Alkylsulfonyl-$NR_3$-, Arylsulfonyl-$NR_3$-, Alkylsulfenyl-, Alkylsulfinyl-, Alkylsulfonyl- oder $R_5$-Gruppe substituiert ist, oder eine Alkylgruppe mit 1 bis 6 Kohlenstoffatomen, die durch eine oder zwei Arylgruppen, durch eine Heteroaryl-, $R_6OCO$-, $(R_3)_2NCO$-, $R_5CO$-, $R_3O$-CO-alkylen-$NR_3$-CO-, $(R_3)_2N$-CO-alkylen-$NR_3$-CO- oder $R_5CO$-alkylen-$NR_3$-CO-gruppe substituiert ist, in denen $R_3$ und $R_5$ wie vorstehend definiert sind und $R_6$ ein Wasserstoffatom, eine Alkylgruppe mit 1 bis 6 Kohlenstoffatomen, eine Cycloalkylgruppe mit 5 bis 7 Kohlenstoffatomen oder eine Aralkylgruppe darstellt,

Y eine NO-Gruppe, ein Stickstoffatom oder eine gegebenenfalls durch eine Alkylgruppe substituierte Methingruppe,

$Z_1$, $Z_2$, $Z_3$ und $Z_4$, die gleich oder verschieden sein können, Methingruppen, Kohlenstoffatome, Iminogruppen oder Stickstoffatome, wobei mindestens einer der Reste $Z_1$ bis $Z_4$ ein Kohlenstoffatom enthalten muß und eine oder zwei zu einem Stickstoffatom benachbarte Methingruppen jeweils durch Carbonylgruppen ersetzt sein können,

$Z_5$ und $Z_6$ jeweils ein Kohlenstoffatom oder auch einer der Reste $Z_5$ oder $Z_6$ ein Stickstoffatom und der andere der Reste $Z_5$ oder $Z_6$ ein Kohlenstoffatom,

A eine Cyanogruppe, eine Aminogruppe, eine geradkettige oder verzweigte Aminoalkylgruppe mit 1 bis 4 Kohlenstoffatomen, eine Amidino-, Guanidino- oder Guanidinoalkylgruppe, wobei in den vorstehend erwähnten Amino-, Aminoalkyl-, Amidino-, Guanidino- oder Guanidinoalkylgruppen jeweils an einem der Stickstoffatome ein oder zwei Wasserstoffatome durch eine oder zwei Alkylgruppen mit jeweils 1 bis 4 Kohlenstoffatomen oder ein Wasserstoffatom durch eine Alkoxycarbonylgruppe mit insgesamt 2 bis 5 Kohlenstoffatomen, durch eine Alkenyloxycarbonylgruppe mit insgesamt 4 bis 6 Kohlenstoffatomen, durch eine Alkylcarbonylgruppe mit insgesamt 2 bis 5 Kohlenstoffatomen, durch eine Arylcarbonyl-, Aryloxycarbonyl-, Aralkoxycarbonyl-, Alkanoyloxymethoxycarbonyl-, Cycloalkanoyloxymethoxycarbonyl-, Aralkanoyloxymethoxycarbonyl-, Aroyloxymethoxycarbonyl-, Phosphono-, Dialkylphosphoryl- oder O-Alkyl-phosphonogruppe ersetzt sein kann, in denen jeweils die Alkanoylteile insgesamt 2 bis 7 Kohlenstoffatome und die Cycloalkanoylteile insgesamt 4 bis 8 Kohlenstoffatome enthalten sowie der Methoxyteil jeweils durch eine Cycloalkylgruppe mit 3 bis 6 Kohlenstoffatomen, durch eine Aralkyl-, Aryl- oder Alkylgruppe oder durch zwei Alkylgruppen, die zusammen mit dem Methylenkohlenstoffatom auch einen 5- oder 6-gliedrigen Ring bilden können, substituiert sein kann, oder, falls B ein cyclisches Imin mit 4 bis 7 Ringgliedern darstellt, auch ein Wasserstoffatom oder eine Alkylgruppe, die jeweils an den Iminostickstoff gebunden sind,

B eine Phenylengruppe, die durch Fluor-, Chlor- oder Bromatome, durch Alkyl-, Hydroxy-, Alkoxy-, Alkylsulfenyl-, Alkylsulfinyl-, Alkylsulfonyl-, Nitro-, Amino-, Alkylamino-, Dialkylamino-, Alkylcarbonylamino-, Alkylsulfonylamino- oder Trifluormethylgruppen mono- oder disubstituiert sein kann, wobei die Substituenten gleich oder verschieden sein können und gleichzeitig eine oder zwei Methingruppen in den vorstehend erwähnten Phenylengruppen jeweils durch ein oder zwei Stickstoffatome ersetzt sein können, oder eine Cycloalkylengruppe mit 3 bis 7 Kohlenstoffatomen, wobei in einem 4- oder 5-gliedrigen Cycloalkylenring ein Ringglied ein Stickstoffatom und in einem 6- oder 7-gliedrigen Cycloalkylenring ein oder zwei Ringglieder jeweils ein Stickstoffatom darstellen können und gleichzeitig die Verbindung mit Kohlenstoffatomen der benachbarten Reste über die gegebenenfalls vorhandenen Stickstoffatome erfolgen kann,

eine Indanylen- oder 1,2,3,4-Tetrahydronaphthylengruppe, in denen jeweils der gesättigte Ring an den Rest A und der aromatische Ring an den Rest C oder an den kondensierten 5-gliedrigen Heterocyclus gebunden ist,

C eine Bindung, eine Alkylen-, Arylen-, -O-alkylen-, -S-alkylen-, -NH-alkylen-, -N(Alkyl)-alkylen-, -Alkylen-NH-, -Alkylen-N(Alkyl)-, -SO-alkylen- oder $-SO_2$-alkylengruppe,

D eine Bindung oder eine Alkylengruppe,

E eine Alkylengruppe mit 1 bis 7 Kohlenstoffatomen, eine Alkenylen- oder Alkinylengruppe mit jeweils 2 bis 7 Kohlenstoffatomen, wobei die Doppel- oder Dreifachbindung nicht direkt an ein Stickstoffatom der $-Z_1-Z_2-Z_3-Z_4$-Gruppe gebunden sein kann, oder, sofern E nicht unmittelbar an ein Stickstoffatom der $-Z_1-Z_2-Z_3-Z_4$-Gruppe gebunden ist, eine -O-, -S-, -SO-, $-SO_2$-, $-NR_3$-, $-N(COR_4)$-, -CO-, $-NR_3$-CO-, $-CO-NR_3$-, $-SO_2-NR_3$-, Alkylsulfonylimino- oder Arylsulfonyliminogruppe, wobei $R_3$ und $R_4$ wie vorstehend erwähnt definiert ist, oder eine Cycloalkylengruppe mit 4 bis 7 Kohlenstoffatomen, wobei in einem 4- oder 5-gliedrigen Cycloalkylenring ein Ringglied ein Stickstoffatom und in einem 6- oder 7-gliedrigen Cycloalkylenring ein oder zwei Ringglieder jeweils ein Stickstoffatom darstellen können und zusätzlich eine zu einem Stickstoffatom benachbarte Methylengruppe durch eine Carbonylgruppe ersetzt sein kann, wobei gleichzeitig die Verbindung mit Kohlenstoffatomen der benachbarten Reste über die gegebenenfalls vorhandenen Stickstoffatome erfolgen kann,

F eine Bindung, eine geradkettige oder verzweigte Alkylengruppe mit 1 bis 6 Kohlenstoffatomen, eine geradkettige oder verzweigte Alkenylen- oder Alkinylengruppe mit jeweils 2 bis 6 Kohlenstoffatomen, wobei die Doppel- oder Dreifachbindung nicht unmittelbar an ein Heteroatom oder eine Dreifachbindung des Restes E anschließen kann, und die vorstehend erwähnten Alkylen-, Alkenylen- und Alkinylengruppen jeweils durch eine Aryl-, $-COOR_6$-, $-CON(R_3)_2$- oder $-CO-N(R_3)$-alkyl-Gruppe substituiert sein können, wobei die Reste $R_3$ und $R_6$ wie vorstehend erwähnt definiert sind und der Alkylteil der $-CO-N(R_3)$-alkyl-Gruppe, der 1 bis 6 Kohlenstoffatome enthalten kann, zusätzlich durch die Reste $R_7$ und $R_8$ substituiert sein kann, wobei $R_7$ und $R_8$, die gleich oder verschieden sein können, jeweils ein Wasserstoffatom, eine Aryl- oder eine $-COOR_6$-gruppe darstellen, wobei $R_6$ wie vorstehend erwähnt definiert ist,

eine Cycloalkylen-, Alkylen-cycloalkylen- oder Cycloalkylenalkylen-gruppe mit jeweils 4 bis 6 Kohlenstoffatomen im Cycloalkylenteil, in denen jeweils eine im Ring befindliche CH-Gruppe durch ein Stickstoffatom ersetzt sein und die Verknüpfung zum benachbarten Rest E jeweils auch über das Stickstoffatom erfolgen kann, sofern die Bindung über ein Kohlenstoffatom des Restes E erfolgt, wobei, falls D eine Bindung, E ein Sauerstoffatom und F eine Alkylgruppe darstellen, A keine direkt an einen Phenylring gebundene Amino- oder Acylaminogruppe sein kann, und, für den Fall, daß gleichzeitig noch X ein Schwefelatom und Y ein Stickstoffatom darstellt, die Gruppe A-B-C keine 4-Acetamino-piperazinogruppe sein kann, und

G eine nicht an ein Heteroatom des Restes E gebundene Carbonylgruppe, die durch eine Hydroxygruppe, durch eine Arylalkenyloxygruppe mit 3 bis 4 Kohlenstoffatomen im Alkenylteil, durch eine Alkoxygruppe mit 1 bis 8 Kohlenstoffatomen, in der ein Alkoxyrest mit 1 bis 5 Kohlenstoffatomen durch eine Arylgruppe oder ein Alkoxyrest mit 1 bis 3 Kohlenstoffatomen in 1-, 2- oder 3-Stellung durch eine Heteroaryl- oder Cycloalkylgruppe mit 4 bis 8 Kohlenstoffatomen oder in 2- oder 3-Stellung durch eine Pyrrolidin-2-on-1-yl-, Morpholino-, Thiomorpholino- oder 1-Oxido-thiomorpholinogruppe substituiert sein kann, durch eine gegebenenfalls durch 1 bis 3 Alkylgruppen substituierte Cycloalkoxygruppe mit 4 bis 8

Kohlenstoffatomen, durch eine gegebenenfalls durch 1 bis 3 Methylgruppen substituierte Benzocycloalkoxy-, Benzocycloalkylalkoxy-, Bicycloalkoxy- oder Bicycloalkylalkoxygruppe mit jeweils 4 bis 8 Kohlenstoffatomen im Cycloalkylteil und 6 bis 8 Kohlenstoffatomen im Bicycloalkylteil, durch eine Alkanoyloxymethoxygruppe mit insgesamt 2 bis 7 Kohlenstoffatomen im Alkanoylteil, durch eine Cycloalkanoyloxymethoxygruppe mit insgesamt 4 bis 8 Kohlenstoffatomen im Cycloalkanoylteil, durch eine Alkoxycarbonyloxymethoxygruppe mit 1 bis 6 Kohlenstoffatomen im Alkylteil, durch eine Cycloalkoxycarbonyloxymethoxygruppe mit 3 bis 7 Kohlenstoffatomen im Cycloalkylteil, durch eine Aroyloxymethoxy-, Aralkanoyloxymethoxy-, Aryloxycarbonyloxymethoxy- oder Aralkoxycarbonyloxymethoxygruppe, in denen der Methoxyteil jeweils durch eine Alkylgruppe mit 1 bis 6 Kohlenstoffatomen, durch eine Cycloalkylgruppe mit 3 bis 7 Kohlenstoffatomen, durch eine Aralkyl- oder Arylgruppe substituiert sein kann, substituiert ist, eine Sulfo-, Phosphono-, O-Alkylphosphono- oder Tetrazol-5-yl-gruppe darstellen,

wobei, soweit nichts anderes erwähnt wurde,

die vorstehend erwähnten Alkyl-, Alkylen- oder Alkoxyteile jeweils 1 bis 3 Kohlenstoffatome enthalten können, sowie

unter "eine Arylgruppe" eine gegebenenfalls durch Fluor-, Chlor-, Brom- oder Jodatome, durch Alkyl-, Trifluormethyl-, Nitro-, Amino-, Alkylamino-, Dialkylamino-, Alkanoylamino-, Alkylsulfonylamino-, Aminosulfonyl-, Alkylaminosulfonyl-, Dialkylaminosulfonyl-, Hydroxy-, Alkoxy-, Aralkoxy-, Carboxy-, Alkoxycarbonyl-, Aminocarbonyl-, Alkylaminocarbonyl-, Dialkylaminocarbonyl-, Alkylsulfenyl-, Alkylsulfinyl- oder Alkylsulfonylgruppen mono-, di- oder trisubstituierte Phenylgruppe, wobei die Substituenten gleich oder verschieden sein können, oder eine Naphthylgruppe und

unter "eine Heteroarylgruppe" ein 5-gliedriger heteroaromatischer Ring, der eine Iminogruppe, ein Sauerstoff- oder Schwefelatom, ein bis zwei Stickstoffatome und ein Sauerstoff- oder Schwefelatom oder eine Iminogruppe und ein bis drei Stickstoffatome enthält, oder ein 6-gliedriger heteroaromatischer Ring, der 1, 2 oder 3 Stickstoffatome enthält, wobei an die vorstehend erwähnten Ringe ein Phenylring ankondensiert sein kann und zusätzlich die vorstehend erwähnten Ringe durch ein Fluor-, Chlor- oder Bromatom, durch eine Alkyl-, Alkoxy-, Hydroxy-, Amino-, Dialkylamino-, Alkylcarbonylamino-, Alkylsulfonylamino- oder Trifluormethylgruppe oder durch eine Alkylaminogruppe mit 1 bis 4 Kohlenstoffatomen mono- oder disubstituiert sein können, zu verstehen sind.

Bevorzugte Verbindungen der obigen allgemeinen Formel I sind jedoch diejenigen, in denen

$R_1$ ein Wasserstoff-, Fluor-, Chlor- oder Bromatom, eine Alkoxycarbonylamino- oder N-Alkoxycarbonylalkylaminogruppe mit jeweils 1 bis 4 Kohlenstoffatomen im Alkoxyteil, eine Alkyl-, Hydroxy-, Alkoxy-, Phenylalkoxy-, Amino-, Alkylamino-, Dialkylamino-, Pyrrolidino-, Piperidino-, Alkylcarbonylamino- oder Alkylsulfonylaminogruppe, wobei soweit nichts anderes erwähnt wurde, der Alkyl- und Alkoxyteil jeweils 1 bis 3 Kohlenstoffatome enthalten kann, und zusätzlich die Alkylteile der Alkylamino- und Dialkylaminogruppen durch eine Carboxy- oder Alkoxycarbonylgruppe mit 1 bis 3 Kohlenstoffatomen im Alkylteil substituiert sein können,

X ein Sauerstoff-, Schwefel- oder Stickstoffatom oder eine $-NR_2$-Gruppe, wobei

$R_2$ ein Wasserstoffatom, eine geradkettige oder verzweigte Alkylgruppe mit 1 bis 14 Kohlenstoffatomen, eine Alkenyl- oder Alkinylgruppe mit jeweils 3 oder 4 Kohlenstoffatomen, wobei die Doppel- oder Dreifachbindung nicht unmittelbar an das Stickstoffatom anschließen kann, eine Alkylgruppe mit 1 bis 5 Kohlenstoffatomen, die durch eine Carboxy-, Alkoxycarbonyl-, Aminocarbonyl-, Alkylaminocarbonyl-, Dialkylaminocarbonyl-, Phenylalkylaminocarbonyl-, Pyrrolidinocarbonyl-, Carboxyalkylaminocarbonyl-, Alkoxycarbonylalkylaminocarbonyl-, 2- oder 4-Imidazolyl- oder Pyridylgruppe, durch eine Piperidinocarbonylgruppe, in der die Methylengruppe in 4-Stellung durch eine Sauerstoffatom, durch eine Sulfenyl-, Sulfinyl-, Sulfonyl-, Imino-, Alkylimino- oder Phenylalkyliminogruppe ersetzt sein kann, oder durch eine gegebenenfalls durch Chlor- oder Bromatome, durch Amino-, Hydroxy-, Alkoxy- oder Alkylgruppen mono- oder disubstituierte Phenylgruppe oder durch zwei Phenylgruppen substituiert ist, eine Cycloalkylgruppe mit 3 bis 7 Kohlenstoffatomen, eine Pyridylgruppe, eine gegebenenfalls durch Fluor-, Chlor- oder Bromatome, durch Alkyl-, Alkoxy-, Alkylsulfenyl-, Alkylsulfinyl- oder Alkylsulfonylgruppen mono- oder disubstituierte Phenylgruppe, wobei die Substiuenten gleich oder verschieden sein können,

eine Alkylgruppe mit 2 bis 4 Kohlenstoffatomen, die in 2-, 3- oder 4-Stellung durch eine Hydroxy-, Alkoxy-, Alkylsulfenyl-, Alkylsulfinyl-, Alklylsulfonyl-, 1-Imidazolyl- oder Pyrrolidinogruppe oder durch eine Piperidinogruppe, in der die Methylengruppe in 4-Stellung durch ein Sauerstoffatom, eine Sulfenyl-, Sulfinyl-, Sulfonyl-, Imino-, Alkylimino- oder Phenylalkyliminogruppe ersetzt sein kann, substituiert ist, oder

eine Alkylgruppe mit 2 bis 6 Kohlenstoffatomen, die in 2-, 3-, 4-, 5- oder 6-Stellung durch eine Amino-, Alkylamino- oder Dialkylaminogruppe substituiert ist, wobei, soweit nichts anderes erwähnt wurde, die vorstehend erwähnten Alkyl- und Alkoxyteile jeweils 1 bis 3 Kohlenstoffatome enthalten können,

Y eine NO-Gruppe, ein Stickstoffatom oder eine gegebenenfalls durch eine Alkylgruppe mit 1 bis 3

Kohlenstoffatomen substituierte Methingruppe,

$Z_1$, $Z_2$, $Z_3$ und $Z_4$, die gleich oder verschieden sein können, Methingruppen, Kohlenstoffatome, Iminogruppen oder Stickstoffatome, wobei mindestens einer der Reste $Z_1$ bis $Z_4$ ein Kohlenstoffatom enthalten muß und eine oder zwei zu einem Stickstoffatom benachbarte Methingruppen jeweils durch Carbonylgruppen ersetzt sein können,

$Z_5$ und $Z_6$ jeweils ein Kohlenstoffatom oder auch einer der Reste $Z_5$ oder $Z_6$ ein Stickstoffatom und der andere der Reste $Z_5$ oder $Z_6$ ein Kohlenstoffatom,

A eine Cyanogruppe, eine Aminogruppe, eine geradkettige oder verzweigte Aminoalkylgruppe mit 1 bis 4 Kohlenstoffatomen, eine Amidino-, Guanidino- oder Guanidinoalkylgruppe, wobei in den vorstehend erwähnten Amino-, Aminoalkyl-, Amidino-, Guanidino- oder Guanidinoalkylgruppen jeweils an einem der Stickstoffatome ein Wasserstoffatom durch eine Alkylgruppe mit jeweils 1 bis 4 Kohlenstoffatomen, durch eine Alkoxycarbonylgruppe mit insgesamt 2 bis 5 Kohlenstoffatomen, durch eine Phenylalkoxycarbonyl-, Phenyloxycarbonyloxy- oder Benzoylgruppe oder durch eine Alkanoyloxymethoxycarbonylgruppe, in der der Alkanoylteil insgesamt 2 bis 4 Kohlenstoffatome enthalten kann, oder durch eine Phosphono-, Dialkylphosphoryl- oder O-Alkyl-phosphonogruppe ersetzt sein kann, oder, falls B ein cyclisches Imin darstellt, auch ein Wasserstoffatom- oder eine Alkylgruppe mit 1 bis 3 Kohlenstoffatomen, die jeweils an den Iminostickstoff gebunden sind,

B eine Phenylengruppe, die durch Fluor-, Chlor- oder Bromatome, durch Alkyl-, Hydroxy-, Alkoxy-, Alkylsulfenyl-, Alkylsulfinyl-, Alkylsulfonyl-, Nitro-, Amino-, Alkylamino-, Dialkylamino-, Alkylcarbonylamino-, Alkylsulfonylamino- oder Trifluormethylgruppen mono- oder disubstituiert sein kann, wobei die Substituenten gleich oder verschieden sein können sowie die Alkyl- und Alkoxyteile jeweils 1 bis 3 Kohlenstoffatome enthalten können, eine Pyridinylen- oder Cycloalkylengruppe mit 3 bis 6 Kohlenstoffatomen, eine Piperidinylen- oder Piperazinylengruppe, die auch über die Stickstoffatome an Kohlenstoffatome der benachbarten Reste gebunden sein können,

eine Indanylen- oder 1,2,3,4-Tetrahydronaphthylengruppe, in denen jeweils der gesättigte Ring an den Rest A und der aromatische Ring an den Rest C oder den kondensierten 5-gliedrigen Heterocyclus gebunden ist,

C eine Bindung, eine Methylen-, Ethylen-, Phenylen-, -O-methylen-, -S-methylen-, -SO-methylen-, $-SO_2-$methylen- oder -N(Alkyl)-methylen-gruppe, die jeweils mit dem Heteroatom an den Rest B gebunden sind, oder eine -Methylen-N(Alkyl)-gruppe, wobei der Alkylteil jeweils 1 bis 3 Kohlenstoffatome enthalten kann,

D eine Bindung, eine Methylen- oder Ethylengruppe,

E eine Alkylengruppe mit 1 bis 5 Kohlenstoffatomen, eine Alkenylen- oder Alkinylengruppe mit jeweils 3 bis 5 Kohlenstoffatomen, wobei die Doppel- oder Dreifachbindung nicht direkt an ein Stickstoffatom der $-Z_1-Z_2-Z_3-Z_4-$Gruppe gebunden sein kann, oder, sofern E nicht unmittelbar an ein Stickstoffatom der $-Z_1-Z_2-Z_3-Z_4-$Gruppe gebunden ist, eine -O-, -S-, -SO-, $-SO_2-$, -NH-, -N(Alkyl)-, -N(COAlkyl)-, -CO-, -NH-CO-, -N(Alkyl)-CO-, -CO-NH-, -CO-N(Alkyl)-, $-SO_2-$NH-, $-SO_2-$N(Alkyl)- oder Alkylsulfonyliminogruppe, wobei der Alkylteil jeweils 1 bis 3 Kohlenstoffatome enthalten kann, eine Cyclohexylengruppe, eine Piperidinylen- oder Piperazinylengruppe, in denen zusätzlich eine zu einem Stickstoffatom benachbarte Methylengruppe durch eine Carbonylgruppe ersetzt sein kann, wobei gleichzeitig die Verbindung mit Kohlenstoffatomen der benachbarten Reste über die Stickstoffatome erfolgen kann,

F eine Bindung, eine Alkylengruppe mit 1 bis 4 Kohlenstoffatomen, die durch eine Phenyl-, Carboxy-, Alkoxycarbonyl-, Phenylalkoxycarbonyl- oder Alkylaminocarbonylgruppe substituiert sein kann, wobei die Alkylaminocarbonylgruppe im Alkylteil, der 1 bis 5 Kohlenstoffatome enthalten kann, durch eine Phenyl-, Hydroxyphenyl-, Methoxyphenyl-, Benzyloxyphenyl-, Carboxy-, Alkoxycarbonyl- oder Phenylalkoxycarbonylgruppe oder zusätzlich durch eine weitere Carboxy-, Alkoxycarbonyl- oder Phenylalkoxycarbonylgruppe substituiert sein kann, eine Cyclohexylen-, Cyclohexylen-alkylen- oder Alkylen-cyclohexylengruppe mit jeweils 1 bis 3 Kohlenstoffatomen in den Alkyl-, Alkylen- und Alkoxyteilen, eine Piperidinylen- oder Pyrrolidinylengruppe, die jeweils auch über das Stickstoffatom an den benachbarten Rest E gebunden sein können, sofern die Bindung über einen Kohlenstoffatom des Restes E erfolgt, wobei, falls D eine Bindung, E ein Sauerstoffatom und F eine Alkylgruppe darstellen, A keine direkt an einen Phenylring gebundene Amino- oder Acylaminogruppe sein kann, und

G eine nicht an ein Heteroatom des Restes E gebundene Carbonylgruppe, die durch eine Hydroxygruppe, durch eine Cinnamyloxygruppe, durch eine Alkoxygruppe mit 1 bis 6 Kohlenstoffatomen, in der ein Alkoxyrest mit 1 bis 5 Kohlenstoffatomen durch eine Phenylgruppe oder ein Alkoxyrest mit 1 bis 3 Kohlenstoffatomen in 1-, 2- oder 3-Stellung durch eine Naphthylgruppe, durch eine Cycloalkylgruppe mit 5 bis 6 Kohlenstoffatomen, durch eine Pyridylgruppe oder in 2- oder 3-Stellung durch eine Pyrrolidin-2-on-1-yl-gruppe substituiert sein kann, durch eine Cycloalkoxygruppe mit 5 bis 8 Kohlenstoffatomen,

durch eine Indanyloxy-, 1,2,3,4-Tetrahydronaphthyloxy-, Bicycloheptyloxy- oder Bicycloheptylmethoxygruppe, wobei die Bicycloheptylgruppe jeweils durch 1 bis 3 Methylgruppen substituiert sein kann, durch eine Alkanoyloxymethoxygruppe mit insgesamt 2 bis 5 Kohlenstoffatomen im Alkanoylteil, durch eine Alkoxycarbonyloxymethoxygruppe mit 1 bis 2 Kohlenstoffatomen im Alkylteil oder durch eine Cycloalkoxycarbonyloxymethoxygruppe mit 5 oder 6 Kohlenstoffatomen im Cycloalkylteil, in denen der Methoxyteil jeweils durch eine Alkylgruppe substituiert sein kann, substituiert ist, eine O-Alkylphosphonogruppe mit 1 bis 3 Kohlenstoffatomen, eine Sulfo-, Phosphono- oder Tetrazol-5-ylgruppe darstellen,
deren Tautomere, deren Stereoisomere einschließlich deren Gemische und deren Salze, insbesondere deren physiologisch verträgliche Salze mit anorganischen oder organischen Säuren oder Basen.

Besonders bevorzugte Verbindungen der obigen allgemeinen Formel I sind jedoch diejenigen, in denen
$R_1$ ein Wasserstoff-, Fluor-, Chlor- oder Bromatom, eine Alkoxy- oder Phenylalkoxygruppe mit jeweils 1 bis 3 Kohlenstoffatomen im Alkoxyteil, eine Alkoxycarbonylamino- oder N-Alkoxycarbonyl-methylamino-gruppe mit jeweils 1 bis 4 Kohlenstoffatomen im Alkoxyteil, eine Methyl-, Hydroxy-, Amino-, Methylamino-, Dimethylamino-, N-Carboxymethyl-amino-, N-Carboxymethyl-methylamino-, N-Methoxycarbonylmethyl-methylamino-, Acetylamino-, Piperidino- oder Methylsulfonylaminogruppe,
X ein Sauerstoff-, Schwefel- oder Stickstoffatom oder eine $-NR_2$-Gruppe, wobei
$R_2$ ein Wasserstoffatom, eine geradkettige oder verzweigte Alkylgruppe mit 1 bis 14 Kohlenstoffatomen, eine Allyl- oder Propargylgruppe,
eine Alkylgruppe mit 1 bis 3 Kohlenstoffatomen, die durch eine Carboxy-, Methoxycarbonyl-, Ethoxycarbonyl-, Aminocarbonyl-, Methylaminocarbonyl-, Dimethylaminocarbonyl-, Benzylaminocarbonyl-, Phenylethylaminocarbonyl-, Carboxymethylaminocarbonyl-, Methoxycarbonylmethylaminocarbonyl- oder Pyridylgruppe oder durch eine Piperidinocarbonylgruppe, in der die Methylengruppe in 4-Stellung durch eine Sauerstoffatom, durch eine Sulfenyl-, Sulfinyl-, Sulfonyl-, Imino- oder Benzyliminogruppe ersetzt sein kann, substituiert ist,
eine Alkylgruppe mit 1 bis 4 Kohlenstoffatomen, die durch eine gegebenenfalls durch Brom- oder Chloratome, durch Amino-, Hydroxy-, Methoxy- oder Methylgruppen mono- oder disubstituierte Phenylgruppe oder durch zwei Phenylgruppen substituiert ist,
eine Cycloalkylgruppe mit 3 bis 6 Kohlenstoffatomen, eine Pyridylgruppe, eine gegebenenfalls durch Chloratome, durch Methyl-, Methoxy-, Methylsulfenyl-, Methylsulfinyl- oder Methylsulfonylgruppen mono- oder disubstituierte Phenylgruppe, wobei die Substituenten gleich oder verschieden sein können,
eine Alkylgruppe mit 2 oder 3 Kohlenstoffatomen, die in 2- oder 3-Stellung durch eine Hydroxy-, Methoxy-, Methylsulfenyl-, Methylsulfinyl-, Methylsulfonyl-, Imidazolyl- oder Pyrrolidinogruppe oder durch eine Piperidinogruppe, in der die Methylengruppe in 4-Stellung durch ein Sauerstoffatom, durch eine Sulfenyl-, Sulfinyl-, Sulfonyl-, Imino- oder Benzyliminogruppe ersetzt sein kann, substituiert ist, oder
eine Alkylgruppe mit 2 bis 6 Kohlenstoffatomen, die in 2-, 3-, 4-, 5- oder 6-Stellung durch eine Aminogruppe substituiert ist,
Y eine NO-Gruppe, ein Stickstoffatom oder eine gegebenenfalls durch eine Methylgruppe substituierte Methingruppe,
$Z_1$, $Z_2$, $Z_3$ und $Z_4$, die gleich oder verschieden sein können, Methingruppen, Kohlenstoffatome, Iminogruppen oder Stickstoffatome, wobei mindestens einer der Reste $Z_1$ bis $Z_4$ ein Kohlenstoffatom enthalten muß und eine oder zwei Zu einem Stickstoffatom benachbarte Methingruppen jeweils durch Carbonylgruppen ersetzt sein können,
$Z_5$ und $Z_6$ jeweils ein Kohlenstoffatom oder auch einer der Reste $Z_5$ oder $Z_6$ ein Stickstoffatom und der andere der Reste $Z_5$ oder $Z_6$ ein Kohlenstoffatom,
A eine Aminogruppe, eine geradkettige oder verzweigte Aminoalkylgruppe mit 1 bis 3 Kohlenstoffatomen, eine Amidino-, Guanidino- oder Guanidinomethylgruppe, wobei in den vorstehend erwähnten Amino-, Aminoalkyl-, Amidino-, Guanidino- oder Guanidinomethylgruppen jeweils an einem der Stickstoffatome ein Wasserstoffatom durch eine Alkylgruppe mit jeweils 1 bis 4 Kohlenstoffatomen, durch eine Alkoxycarbonylgruppe mit insgesamt 2 bis 5 Kohlenstoffatomen, durch eine Benzyloxycarbonyl-, Acetyloxymethoxycarbonyl-, Dimethylphosphoryl- oder Diethylphosphorylgruppe ersetzt sein kann,
B eine Phenylengruppe, die durch ein Fluor-, Chlor- oder Bromatom, durch eine Methyl-, Hydroxy- oder Methoxygruppe substituiert sein kann, eine Pyridinylengruppe oder eine Cycloalkylengruppe mit 3 bis 6 Kohlenstoffatomen, eine Indanylen- oder 1,2,3,4-Tetrahydronaphthylengruppe, in denen jeweils der gesättigte Ring an den Rest A und der aromatische Ring an den Rest C oder den kondensierten 5-gliedrigen Heterocyclus gebunden ist, eine Piperidinylen- oder Piperazinylengruppe, die auch über die Stickstoffatome an Kohlenstoffatome der benachbarten Reste gebunden sein können,
C eine Bindung, eine Phenylen- oder eine -O-methylen-Gruppe,
D eine Bindung oder eine Methylengruppe,

E eine Alkylengruppe mit 3 bis 5 Kohlenstoffatomen, eine Alkenylengruppe mit jeweils 3 bis 5 Kohlenstoffatomen, wobei die Doppelbindung nicht direkt an ein Stickstoffatom der $-Z_1-Z_2-Z_3-Z_4$-Gruppe gebunden sein kann, oder, sofern E nicht unmittelbar an ein Stickstoffatom der $-Z_1-Z_2-Z_3-Z_4$-Gruppe gebunden ist, eine -O-, -S-, -SO-, $-SO_2-$, -NH-, -N(Methyl)-, -N(Acetyl)-, $-N(SO_2CH_3)-$, -CO-, -NH-CO-, $-N(CH_3)-CO-$, -CO-NH-, $-CO-N(CH_3)-$, $-SO_2-NH-$ oder $-SO_2-N(CH_3)$-gruppe, eine Piperidinylen-, Piperazinylen- oder Oxo-piperazinylengruppe, wobei die Stickstoffatome auch an Kohlenstoffatome der benachbarten Reste gebunden sein können,

F eine Bindung, eine Alkylengruppe mit 1 bis 4 Kohlenstoffatomen, die durch eine Phenyl-, Carboxy-, Methoxycarbonyl-, Benzyloxycarbonyl-, Phenylethylaminocarbonyl-, (Methoxyphenyl)ethylaminocarbonyl- oder Alkylaminocarbonylgruppe, wobei die Alkylaminocarbonylgruppe im Alkylteil, der 1 bis 4 Kohlenstoffatome enthalten kann, durch eine Phenyl-, Hydroxyphenyl-, Methoxyphenyl-, Carboxy- oder Benzyloxycarbonylgruppe oder zusätzlich durch eine weitere Carboxy- oder Benzyloxycarbonylgruppen substituiert sein kann, substituiert sein kann,

eine Piperidinylen- oder Pyrrolidinylengruppe, die jeweils auch über das Stickstoffatom an den benachbarten Rest E gebunden sein können, sofern die Bindung über ein Stickstoffatom des Restes E erfolgt,

eine Cyclohexylen- oder Cyclohexylen-methylengruppe, wobei, falls D eine Bindung, E ein Sauerstoffatom und F eine Alkylgruppe darstellen, A keine direkt an einen Phenylring gebundene Amino- oder Acylaminogruppe sein kann,

G eine nicht an ein Heteroatom des Restes E gebundene Carbonylgruppe, die durch eine Hydroxygruppe, durch eine Alkoxygruppe mit 1 bis 6 Kohlenstoffatomen, die in 1 bis 5-Stellung durch eine Phenylgruppe oder in 1- oder 2-Stellung durch eine Cyclohexyl-, Naphthyl- oder Pyridylgruppe oder in 2-Stellung durch eine Pyrrolidin-2-on-1-yl-gruppe substituiert sein kann, durch eine Cinnamyloxygruppe, durch eine Cycloalkoxygruppe mit 6 bis 8 Kohlenstoffatomen, durch eine Indanyloxy-, Norbornyloxy- oder Norbornylmethyloxygruppe, durch eine Alkanoyloxymethoxygruppe mit insgesamt 2 bis 5 Kohlenstoffatomen im Alkanoylteil, durch eine Alkoxycarbonyloxymethoxygruppe mit 1 bis 3 Kohlenstoffatomen im Alkoxyteil oder durch eine Cyclohexyloxycarbonyloxymethoxygruppe, in denen der Methoxyteil jeweils durch eine Methylgruppe substituiert sein kann, substituiert ist, eine Sulfo-, Phosphono-, O-Methyl-phosphono- oder Tetrazol-5-ylgruppe darstellen,

insbesondere diejenigen Verbindungen, in denen

$R_1$ ein Wasserstoff-, eine Methyl-, Methoxy-, Methylamino-, Dimethylamino-, N-Butyloxycarbonyl-methylamino-, N-Isobutyloxycarbonyl-methylamino-, N-Carboxymethyl-amino-, N-Carboxymethyl-methylamino-, N-Methoxycarbonylmethyl-amino- oder N-Methoxycarbonylmethyl-methylaminogruppe,

X ein Sauerstoff- oder Stickstoffatom oder eine $-NR_2$-Gruppe, wobei

$R_2$ ein Wasserstoffatom, eine geradkettige oder verzweigte Alkylgruppe mit 1 bis 14 Kohlenstoffatomen,

eine Alkylgruppe mit 1 bis 3 Kohlenstoffatomen, die durch eine Carboxy- oder Methoxycarbonylgruppe substituiert ist,

eine Alkylgruppe mit 1 bis 4 Kohlenstoffatomen, die durch eine gegebenenfalls durch Bromatome, Amino- oder Methoxygruppen mono- oder disubstituierte Phenylgruppe durch zwei Phenylgruppen oder durch eine Pyridylgruppe substituiert ist,

eine Alkylgruppe mit 2 oder 3 Kohlenstoffatomen, die in 2- oder 3-Stellung durch eine Amino- oder Piperidinogruppe, in der die Methylengruppe in 4-Stellung durch eine Imino-, Benzylimino-, Sulfenyl-, Sulfinyl- oder Sulfonylgruppe ersetzt ist, substituiert ist,

Y eine NO-Gruppe, ein Stickstoffatom oder eine Methingruppe,

$Z_1$, $Z_2$, $Z_3$ und $Z_4$, die gleich oder verschieden sein können, Methingruppen, Kohlenstoffatome, Iminogruppen oder Stickstoffatome, wobei mindestens zwei der Reste $Z_1$ bis $Z_4$ ein Kohlenstoffatom enthalten müssen und eine oder zwei zu einem Stickstoffatom benachbarte Methingruppen jeweils durch Carbonylgruppen ersetzt sein können,

$Z_5$ und $Z_6$ jeweils ein Kohlenstoffatom oder auch einer der Reste $Z_5$ oder $Z_6$ ein Stickstoffatom und der andere der Reste $Z_5$ oder $Z_6$ ein Kohlenstoffatom,

A eine Aminomethyl- oder Amidinogruppe, in denen jeweils an einem der Stickstoffatome ein Wasserstoffatom durch eine Methoxycarbonylgruppe ersetzt sein kann,

B eine Phenylengruppe,

C eine Bindung oder eine -O-methylen-Gruppe,

D eine Bindung,

E eine Alkylengruppe mit 3 oder 4 Kohlenstoffatomen oder, sofern E nicht unmittelbar an ein Stickstoffatom der $-Z_1-Z_2-Z_3-Z_4$-Gruppe gebunden ist, eine -O-, -NH-CO- oder -CO-NH-gruppe,

F eine Bindung oder eine Alkylengruppe mit 1 bis 3 Kohlenstoffatomen und

G eine Carboxygruppe, eine Alkoxycarbonylgruppe mit insgesamt 2 bis 5 Kohlenstoffatomen oder eine Cyclohexyloxygruppe darstellen,

deren Tautomere, deren Stereoisomere einschließlich deren Gemische und deren Salze, insbesondere deren physiologisch verträgliche Salze mit anorganischen oder organischen Säuren oder Basen.

Erfindungsgemäß erhält man die neuen Verbindungen der allgemeinen Formel I beispielsweise nach folgenden an und für sich bekannten Verfahren:

a) Zur Herstellung von Verbindungen der allgemeinen Formel I, in der G eine Carboxylgruppe darstellt: Überführung einer Verbindung der allgemeinen Formel

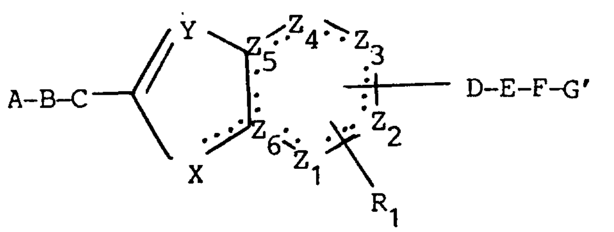

,(II)

in der

A bis F, $R_1$, $Z_1$ bis $Z_6$, X und Y wie eingangs definiert sind und

G', das an ein Kohlenstoffatom gebunden ist, eine mittels Hydrolyse, Behandlung mit Säuren, Thermolyse oder Hydrogenolyse in eine Carboxylgruppe überführbare Gruppe darstellt.

Beispielsweise können funktionelle Derivate der Carboxylgruppe wie deren unsubstituierte oder substituierte Amide, Ester, Thioester, Trimethylsilylester, Orthoester, Iminoester, Amidine oder Anhydride, oder die Nitrilgruppe mittels Hydrolyse in eine Carboxylgruppe, Ester mit tertiären Alkoholen, z.B. der tert. Butylester, mittels Behandlung mit einer Säure oder Thermolyse in eine Carboxylgruppe, Ester mit Aralkanolen, z.B. der Benzylester, mittels Hydrogenolyse in eine Carboxylgruppe, und Bis-(alkoxycarbonyl)methylgruppen mittels Hydrolyse oder Behandlung mit einer Säure in eine Bis-(hydroxycarbonyl)methylgruppe, welche anschließend decarboxyliert wird, übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure, in Gegenwart einer Base wie Lithiumhydroxid, Natriumhydroxid oder Kaliumhydroxid in einem geeigneten Lösungsmittel wie Wasser, Methanol, Wasser/Methanol, Ethanol, Wasser/Ethanol, Wasser/Isopropanol, Wasser/Tetrahydrofuran oder Wasser/Dioxan bei Temperaturen zwischen -10 °C und 120 °C, z.B. bei Temperaturen zwischen Raumtemperatur und der Siedetemperatur des Reaktionsgemisches, durchgeführt. Bei der Behandlung mit einer organischen Säure wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet G' in einer Verbindung der Formel II eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50 °C in die Carboxylgruppe übergeführt werden.

Bedeutet G' in einer Verbindung der Formel II beispielsweise die tert. Butyloxycarbonylgruppe, so kann die tert. Butylgruppe auch durch Behandlung mit einer Säure wie Trifluoressigsäure, Ameisensäure, p-Toluolsulfonsäure, Schwefelsäure, Phosphorsäure oder Polyphosphorsäure gegebenenfalls in einem inerten Lösungsmittel wie Methylenchlorid, Chloroform, Benzol, Toluol, Tetrahydrofuran oder Dioxan vorzugsweise bei Temperaturen zwischen -10 °C und 120 °C, z.B. bei Temperaturen zwischen 0 und 60 °C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 °C und 100 °C, abgespalten werden.

Bedeutet G' in einer Verbindung der Formel II beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureethylester, Dioxan oder Dimethylformamid vorzugsweise bei Temperaturen zwischen 0 und 50 °C, z.B. bei Raumtemperatur, und einem Wasserstoffdruck von 1 bis 10 bar abgespalten werden. Bei der Hydrogenolyse können gleichzeitig andere Reste, z.B. eine Nitrogruppe zur Aminogruppe, oder eine

Benzyloxygruppe zur Hydroxygruppe mitreduziert werden.

b) Zur Herstellung von Benzimidazolen der allgemeinen Formel I:

Cyclisierung einer Verbindung der allgemeinen Formel

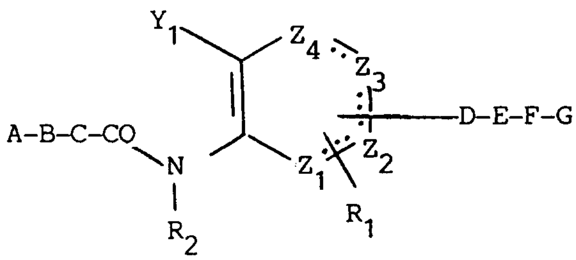

,(III)

in der

A bis G, $R_1$, $R_2$ und $Z_1$ bis $Z_4$ wie eingangs definiert sind und

$Y_1$ eine Aminogruppe darstellt.

Die Cyclisierung wird zweckmäßigerweise in einem Lösungsmittel- oder Lösungsmittelgemisch wie Methanol, Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel III verwendeten Acylierungsmittel, z.B. in der entsprechenden Säure oder dem entsprechenden Nitril, Anhydrid, Säurehalogenid, Ester oder Amid, beispielsweise bei Temperaturen zwischen 0 und 250 °C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Methansulfonsäure, Salzsäure, Phosphorsäure, Polyphosphorsäure, Essigsäureanhydrid oder gegebenenfalls auch in Gegenwart einer Base wie Kaliumäthylat oder Kaliumtert.butylat durchgeführt. Die Cyclisierung kann jedoch auch ohne Lösungsmittel und/oder Kondensationsmittel durchgeführt werden.

Besonders vorteilhaft wird die Umsetzung jedoch in der Weise durchgeführt, daß eine Verbindung der allgemeinen Formel III im Reaktionsgemisch durch Acylierung einer entsprechenden Diaminoverbindung oder durch Reduktion einer entsprechenden o-Nitro-acylaminoverbindung hergestellt wird. Bei Abbruch der Reduktion der Nitrogruppe auf der Hydroxylaminstufe erhält man bei der anschließenden Cyclisierung das N-Oxid einer Verbindung der allgemeinen Formel I. Das so erhaltene N-Oxid kann anschließend mittels Reduktion in eine entsprechende Verbindung der allgemeinen Formel I übergeführt werden.

Die anschließende Reduktion des erhaltenen N-Oxids der Formel I wird vorzugsweise in einem Lösungsmittel wie Wasser, Wasser/Ethanol, Methanol, Eisessig, Essigsäureethylester oder Dimethylformamid mit Wasserstoff in Gegenwart eines Hydrierungskatalysators wie Raney-Nickel, Platin oder Palladium/Kohle, mit Metallen wie Eisen, Zinn oder Zink in Gegenwart einer Säure wie Essigsäure, Salzsäure oder Schwefelsäure, mit Salzen wie Eisen(II)sulfat, Zinn(II)chlorid oder Natriumdithionit, mit Derivaten des dreiwertigen Phosphors wie Triphenylphosphin, Triethylphosphit oder Phosphortrichlorid, oder mit Hydrazin in Gegenwart von Raney-Nickel bei Temperaturen zwischen 0 und 50 °C, vorzugsweise jedoch bei Raumtemperatur durchgeführt.

c) Zur Herstellung von Verbindungen der allgemeinen Formel I, in der A eine gegebenenfalls durch eine Alkylgruppe substituierte Amidinogruppe darstellt:

Umsetzung einer gegebenenfalls im Reaktionsgemisch gebildeten Verbindung der allgemeinen Formel

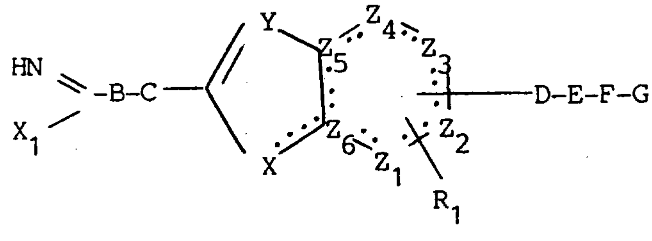

,(IV)

in der

B bis G, $R_1$, $Z_1$ bis $Z_6$, X und Y wie eingangs definiert sind und
$X_1$ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe oder eine Aminogruppe darstellt, mit einem Amin der allgemeinen Formel

$R_a$ - $NH_2$ ,(V)

in der
$R_a$ ein Wasserstoffatom oder eine Alkylgruppe mit 1 bis 4 Kohlenstoffatomen bedeutet, oder mit deren Säureadditionssalzen.

Die Umsetzung wird zweckmäßigerweise in einem Lösungsmittel wie Methanol, Ethanol, n-Propanol, Wasser, Methanol/Wasser, Tetrahydrofuran oder Dioxan bei Temperaturen zwischen 0 und 150 °C, vorzugsweise bei Temperaturen zwischen 20 und 120 °C, mit einem entsprechenden freien Amin oder mit einem entsprechenden Säureadditionssalz wie beispielsweise Ammoniumcarbonat oder Ammoniumacetat durchgeführt.

Eine Verbindung der allgemeinen Formel IV erhält man beispielsweise durch Umsetzung eines entsprechenden Nitrils mit einem entsprechenden Alkohol wie Methanol, Ethanol, n-Propanol, Isopropanol oder Benzylalkohol in Gegenwart einer Säure wie Salzsäure oder in Gegenwart eines entsprechenden Alkoholats wie Natriummethylat oder Natriumethylat oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen -10 und 50 °C, vorzugsweise jedoch bei Temperaturen zwischen 0 und 20 °C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid.

d) Zur Herstellung von Verbindungen der allgemeinen Formel I, in der A eine Aminoalkylgruppe darstellt:
Reduktion einer Verbindung der allgemeinen Formel

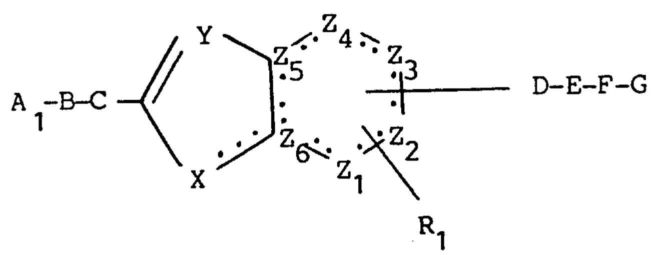

,(VI)

in der
B bis G, $R_1$, $Z_1$ bis $Z_6$, X und Y wie eingangs definiert sind und
$A_1$ eine Cyano- oder Cyanoalkylgruppe darstellt.

Die Reduktion wird vorzugsweise in einem geeigneten Lösungsmittel wie Methanol, Methanol/Wasser, Methanol/Ammoniak, Methanol/Wasser/Ammoniak, Methanol/Salzsäure, Ethanol, Ether, Tetrahydrofuran, Dioxan, Dimethylformamid oder Eisessig in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Lithiumborhydrid oder Lithiumaluminiumhydrid bei Temperaturen zwischen 0 und 100 °C, vorzugsweise bei Temperaturen zwischen 20 und 80 °C, durchgeführt.

e) Zur Herstellung von Verbindungen der allgemeinen Formel I, die eine Sulfinylgruppe oder eine Sulfonylgruppe enthalten, die nicht mit einem Stickstoffatom verknüpft ist:
Oxidation einer Verbindung der allgemeinen Formel

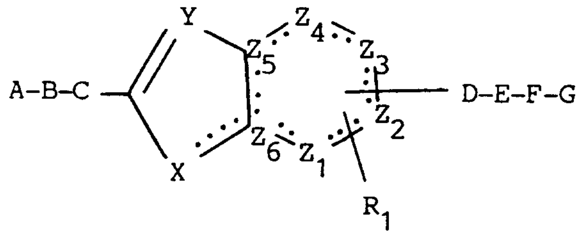

, (VII)

in der
A bis G, $R_1$, $Z_1$ bis $Z_6$, X und Y mit der Maßgabe wie eingangs definiert sind, daß mindestens einer der Reste $R_1$, B, C, E, G oder X eine Sulfenyl- oder Sulfinylgruppe enthält, die nicht mit einem Stickstoffatom verknüpft ist.

Die Oxidation wird vorzugsweise in einem Lösungsmittel oder Lösungsmittelgemisch, z.B. in Wasser, Wasser/Pyridin, Aceton, Methylenchlorid, Eisessig, Eisessig/Acetanhydrid, verdünnter Schwefelsäure oder Trifluoressigsäure, je nach dem verwendeten Oxidationsmittel zweckmäßigerweise bei Temperaturen zwischen -80 und 100 °C durchgeführt.

Zur Herstellung einer entsprechenden S-Oxidverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20 °C oder in Aceton bei 0 bis 60 °C, mit einer Persäure wie Perameisensäure in Eisessig oder Trifluoressigsäure bei 0 bis 50 °C oder mit m-Chlorperbenzoesäure in Methylenchlorid oder Chloroform bei -20 bis 60 °C, mit Natriummetaperjodat in wäßrigem Methanol oder Ethanol bei -15 bis 25 °C, mit Brom in Eisessig oder wäßriger Essigsäure gegebenenfalls in Gegenwart einer schwachen Base wie Natriumacetat, mit N-Brom-succinimid in Ethanol, mit tert.Butyl-hypochlorit in Methanol bei -80 bis -30 °C, mit Jodbenzodichlorid in wäßrigem Pyridin bei 0 bis 50 °C, mit Salpetersäure in Eisessig bei 0 bis 20 °C, mit Chromsäure in Eisessig oder in Aceton bei 0 bis 20 °C und mit Sulfurylchlorid in Methylenchlorid bei -70 °C, der hierbei erhaltene Thioether-Chlor-Komplex wird zweckmäßigerweise mit wäßrigem Ethanol hydrolysiert.

Zur Herstellung einer S,S-Dioxidverbindung der allgemeinen Formel I wird die Oxidation ausgehend von einer entsprechenden Alkylsulfinylverbindung zweckmäßigerweise mit einem oder mehr Äquivalenten des verwendeten Oxidationsmittels oder ausgehend von einer entsprechenden Alkylsulfenylverbindung zweckmäßigerweise mit zwei oder mehr Äquivalenten des verwendeten Oxidationsmittels durchgeführt, z.B. mit Wasserstoffperoxid in Eisessig/Acetanhydrid, Trifluoressigsäure oder in Ameisensäure bei 20 bis 100 °C oder in Aceton bei 0 bis 60 °C, mit einer Persäure wie Perameisensäure oder m-Chlorperbenzoesäure in Eisessig, Trifluoressigsäure, Methylenchlorid oder Chloroform bei Temperaturen zwischen 0 und 60 °C, mit Salpetersäure in Eisessig bei 0 bis 20 °C, mit Chromsäure oder Kaliumpermanganat in Eisessig, Wasser/Schwefelsäure oder in Aceton bei 0 bis 20 °C.

f) Zur Herstellung von Verbindungen der allgemeinen Formel I, in der A eine durch eine Alkoxycarbonylgruppe mit insgesamt 2 bis 5 Kohlenstoffatomen, durch eine Arylmethoxycarbonyl-, Arylethoxycarbonyl-, Aryloxycarbonyl-, Alkylcarbonyl-, Aralkylcarbonyl- oder Arylcarbonylgruppe substituierte Amino-, Aminoalkyl-, Amidino-, Guanidino- oder Guanidinoalkylgruppe darstellt:
Umsetzung einer Verbindung der allgemeinen Formel

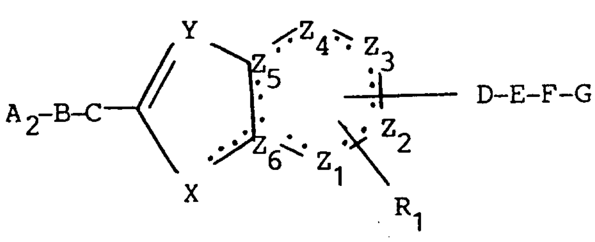

, (VIII)

in der
B bis G, $R_1$, $Z_1$ bis $Z_6$, X und Y wie eingangs definiert sind und
$A_2$ eine Amino-, Aminoalkyl-, Amidino-, Guanidino- oder Guanidinoalkylgruppe darstellt, mit einer Verbindung der allgemeinen Formel

$X_2$ - $COR_b$ ,(IX)

in der
$R_b$ eine Alkoxygruppe mit 1 bis 4 Kohlenstoffatomen, eine Arylmethoxy-, Arylethoxy-, Aryloxy-, Alkyl-, Aralkyl- oder Arylgruppe und
$X_2$ eine nukleophile Austrittsgruppe wie ein Halogenatom, z. B. ein Chlor- oder Bromatom, darstellen.

Die Umsetzung wird zweckmäßigerweise in einem Lösungsmittel wie Tetrahydrofuran, Methylenchlorid, Chloroform oder Dimethylformamid zweckmäßigerweise in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100 ° C, vorzugsweise jedoch bei Temperaturen zwischen -10 und 80 ° C, durchgeführt.

g) Zur Herstellung von Verbindungen der allgemeinen Formel I, in der G eine nicht an ein Heteroatom des Restes E gebundene Carbonylgruppe darstellt, die durch eine Cinnamyloxygruppe oder durch eine Alkoxygruppe mit 1 bis 6 Kohlenstoffatomen substituiert ist, in der ein Alkoxyrest mit 1 bis 5 Kohlenstoffatomen durch eine Phenylgruppe oder ein Alkoxyrest mit 1 bis 3 Kohlenstoffatomen in 1-, 2- oder 3-Stellung durch eine Naphthylgruppe, durch eine Cycloalkylgruppe mit 5 bis 6 Kohlenstoffatomen, durch eine Pyridylgruppe oder in 2- oder 3-Stellung durch eine Pyrrolidin-2-on-1-yl-gruppe substituiert sein kann, durch eine Cycloalkoxygruppe mit 5 bis 8 Kohlenstoffatomen, durch eine Indanyloxy-, 1,2,3,4-Tetrahydronaphthyloxy-, Bicycloheptyloxy- oder Bicycloheptylmethoxygruppe, durch eine Alkanoyloxymethoxygruppe mit insgesamt 2 bis 5 Kohlenstoffatomen im Alkanoylteil, durch eine Alkoxycarbonyloxymethoxygruppe mit 1 bis 2 Kohlenstoffatomen im Alkylteil oder durch eine Cycloalkoxycarbonyloxymethoxygruppe mit 5 oder 6 Kohlenstoffatomen im Cycloalkylteil, in denen der Methoxyteil jeweils durch eine Alkylgruppe substituiert sein kann, substituiert ist:
Umsetzung einer Verbindung der allgemeinen Formel

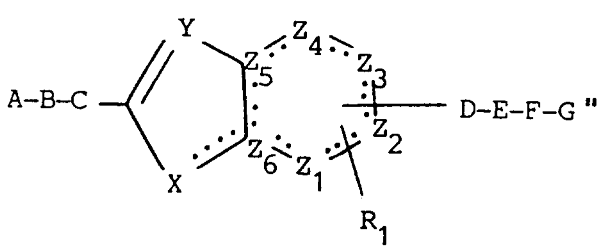 ,(X)

in der
A bis F, $R_1$, $Z_1$ bis $Z_6$, X und Y wie eingangs definiert sind und
G" eine Carboxy- oder Alkoxycarbonylgruppe darstellt, mit einem Alkohol der allgemeinen Formel

$X_3$ - $R_c$ ,(XI)

in der
$X_3$ eine Hydroxygruppe oder eine nukleophile Austrittsgruppe wie ein Halogenatom, z.B. ein Chlor-, Brom- oder Jodatom, und
$R_c$ eine Cinnamylgruppe oder eine Alkylgruppe mit 1 bis 6 Kohlenstoffatomen, in der ein Alkylrest mit 1 bis 5 Kohlenstoffatomen durch eine Phenylgruppe oder ein Alkylrest mit 1 bis 3 Kohlenstoffatomen in 1-, 2- oder 3-Stellung durch eine Naphthylgruppe, durch eine Cycloalkylgruppe mit 5 bis 6 Kohlenstoffatomen, durch eine Pyridylgruppe oder in 2- oder 3-Stellung durch eine Pyrrolidin-2-on-1-yl-gruppe substituiert sein kann, eine Cycloalkylgruppe mit 5 bis 8 Kohlenstoffatomen, eine Indanyl-, 1,2,3,4-Tetrahydronaphthyl-, Bicycloheptyl- oder Bicycloheptylmethylgruppe, eine Alkanoyloxymethylgruppe mit insgesamt 2 bis 5 Kohlenstoffatomen im Alkanoylteil, eine Alkoxycarbonyloxymethylgruppe mit 1 bis 2 Kohlenstoffatomen im Alkylteil oder eine Cycloalkoxycarbonyloxymethylgruppe mit 5 oder 6 Kohlenstoffatomen im Cycloalkylteil, in denen der Methylteil jeweils durch eine Alkylgruppe substituiert sein kann, darstellen.

Die Umsetzung wird zweckmäßigerweise in einem Lösungsmittel oder Lösungsmittelgemisch wie Methylenchlorid, Dimethylformamid, Dimethylsulfoxid, Toluol, Chlorbenzol, Tetrahydrofuran,

Toluol/Tetrahydrofuran oder Dioxan gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Titantetrachlorid, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol, N,N'-Carbonyldiimidazol- oder N,N'-Thionyldiimidazol oder Triphenylphosphin/Tetrachlorkohlenstoff oder in Gegenwart einer Base wie Natriumcarbonat, Kaliumcarbonat, Natronlauge, Triethylamin, N-Ethyl-diisopropylamin, N-Methyl-morpholin, Pyridin oder 4-Dimethylamino-pyridin zweckmäßigerweise bei Temperaturen zwischen 0 und 150 ° C, vorzugsweise bei Temperaturen zwischen 0 und 50 ° C, durchgeführt.

Bedeutet $X_3$ eine nukleophile Austrittsgruppe, so wird die Umsetzung zweckmäßigerweise in einem Lösungsmittel wie Methylenchlorid, Tetrahydrofuran, Dioxan, Dimethylsulfoxid oder Dimethylformamid gegebenenfalls in Gegenwart eines Reaktionsbeschleunigers wie Natrium- oder Kaliumjodid und vorzugsweise in Gegenwart einer Base wie Natriumcarbonat, Kaliumcarbonat oder Natronlauge oder in Gegenwart einer tertiären organischen Base wie N-Ethyl-diisopropylamin oder N-Methyl-morpholin, welche gleichzeitig auch als Lösungsmittel dienen können, oder gegebenenfalls in Gegenwart von Silberkarbonat oder Silberoxid bei Temperaturen zwischen -30 und 100 ° C, vorzugsweise jedoch bei Temperaturen zwischen -10 und 80 ° C, durchgeführt.

Bedeutet $X_3$ eine Hydroxygruppe, so wird die Veresterung zweckmäßigerweise in einem Lösungsmittel oder Lösungsmittelgemisch wie Methylenchlorid, Dimethylformamid, Dimethylsulfoxid, Toluol, Chlorbenzol, Tetrahydrofuran, Toluol/Tetrahydrofuran oder Dioxan gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Titantetrachlorid, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol, N,N'-Carbonyldiimidazol- oder N,N'-Thionyldiimidazol oder Triphenylphosphin/Tetrachlorkohlenstoff bei Temperaturen zwischen -30 und 100 ° C, vorzugsweise jedoch bei Temperaturen zwischen -10 und 60 ° C, durchgeführt.

h) Zur Herstellung von Verbindungen der allgemeinen Formel I, in der A eine Guanidinoalkylgruppe oder eine Amidinogruppe darstellt, welche an das Stickstoffatom einer cyclischen Iminogruppe gebunden ist:
Umsetzung einer Verbindung der allgemeinen Formel

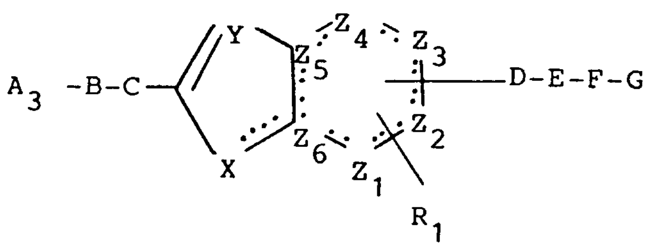

,(XII)

in der
B bis G, $R_1$, $Z_1$ bis $Z_6$, X und Y wie eingangs definiert sind und
$A_3$ eine Aminoalkylgruppe darstellt, oder eine Verbindung der allgemeinen Formel

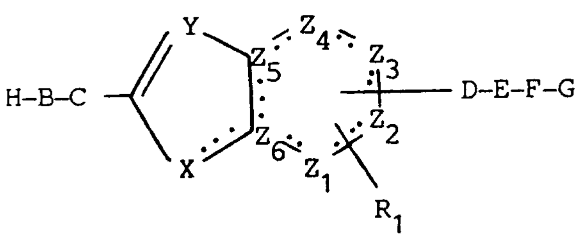

,(XIII)

in der
B bis G, $R_1$, $Z_1$ bis $Z_6$, X und Y mit der Maßgabe wie eingangs definiert sind, daß der Rest B eine cyclische Iminogruppe enthält, mit einem S-Alkyl-isothioharnstoff.

Die Umsetzung wird zweckmäßigerweise in einem Lösungsmittel wie Dimethylformamid und vorzugsweise in Gegenwart einer Base wie Natriumcarbonat bei erhöhten Temperaturen, z.B. bei Temperaturen zwischen 80 und 120°C, durchgeführt.

i) Zur Herstellung von Verbindungen der allgemeinen Formel I, in der A eine an einen aromatischen Ring gebundene Guanidinogruppe darstellt:

Umsetzung einer Verbindung der allgemeinen Formel

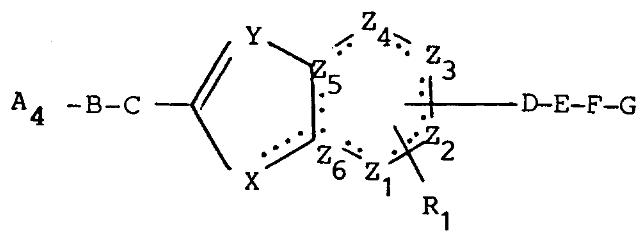

,(XIV)

in der

B bis G, $R_1$, $Z_1$ bis $Z_6$, X und Y mit der Maßgabe wie eingangs definiert sind, daß B einen aromatischen oder heteroaromatischen Ring darstellt, und

$A_4$ eine Aminogruppe darstellt, mit Cyanamid oder dessen Säureadditionssalz.

Die Umsetzung wird zweckmäßigerweise in einem Lösungsmittel wie Dioxan, Dioxan/Wasser oder Tetrahydrofuran vorzugsweise bei Temperaturen zwischen 60 und 120°C, z.B. bei der Siedetemperatur des Reaktionsgemisches, durchgeführt.

j) Zur Herstellung von Verbindungen der allgemeinen Formel I, in der D eine Bindung und E eine $-NR_3-CO$-Gruppe darstellt:

Umsetzung einer Verbindung der allgemeinen Formel

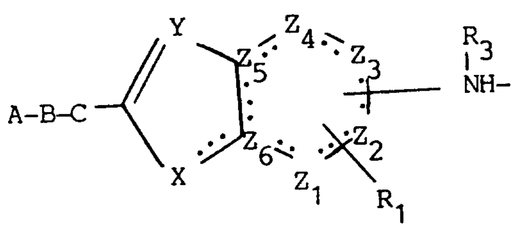

,(XV)

in der

A bis C, $R_1$, $R_3$, $Z_1$ bis $Z_6$, X und Y wie eingangs definiert sind, mit einer Verbindung der allgemeinen Formel

$X_4$ - CO - F - G ,(XVI)

in der

F und G wie eingangs definiert sind und

$X_4$ eine nucleophile Austrittsgruppe wie eine Hydroxy- oder Alkoxygruppe oder ein Halogenatom, z. B. ein Chlor-, Brom- oder Jodatom oder eine Methoxy- oder Ethoxygruppe, darstellt, oder mit deren reaktionsfähigen Derivaten.

Die Umsetzung wird zweckmäßigerweise in einem Lösungsmittel wie Methylenchlorid, Chloroform, Tetrachlorkohlenstoff, Ether, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, gegebenenfalls in Gegenwart einer anorganischen Base wie Natriumcarbonat oder einer tertiären organischen Base wie Triethylamin, Pyridin oder 4-Dimethylamino-pyridin, welche gleichzeitig als Lösungsmittel dienen können, bei Temperaturen zwischen -25 und 150°C, vorzugsweise jedoch bei Temperaturen zwischen -10°C und der Siedetemperatur des verwendeten Lösungsmittels, durchgeführt. Die Acylierung

wird jedoch wie vorstehend beschrieben vorzugsweise mit einem entsprechenden Säurehalogenid oder Säureanhydrid durchgeführt, wobei diese auch ohne Lösungsmittel durchgeführt werden kann.

k) Zur Herstellung von Verbindungen der allgemeinen Formel I, in der A eine Amino- oder Aminoalkylgruppe darstellt:

Umsetzung einer Verbindung der allgemeinen Formel

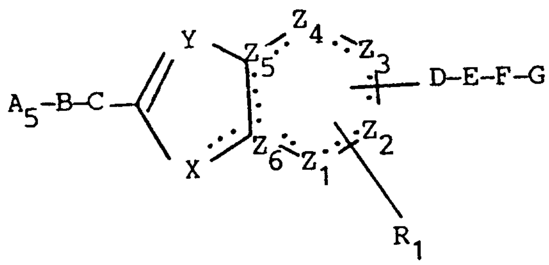

,(XVII)

in der

$R_1$, B bis G, X, Y und $Z_1$ bis $Z_6$ wie eingangs definiert sind und

$A_5$ eine $H_2N$-CO-T-Gruppe darstellt, in der T eine Bindung oder eine Alkylengruppe mit 1 bis 4 Kohlenstoffatomen darstellt, mit einer Phenyljod(III)verbindung der allgemeinen Formel

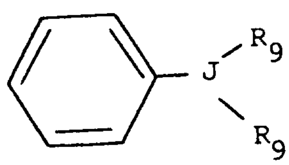

,(XVIII)

in der

$R_9$ jeweils den Acylrest einer organischen Carbonsäure wie die Acetoxy- oder Trifluoracetoxygruppe darstellt.

Die Umsetzung wird vorzugsweise in einem wäßrigen Lösungsmittel wie Wasser oder Wasser/Acetonitril bei Temperaturen zwischen 0 und 50 °C, vorzugsweise jedoch bei Raumtemperatur, durchgeführt.

1) Zur Herstellung von Verbindungen der allgemeinen Formel I, in der A eine Aminoalkylgruppe, in der die Aminogruppe nicht an ein quartäres Kohlenstoffatom gebunden ist, oder eine Aminogruppe darstellt, die an eine CH- oder $CH_2$-Gruppe des Restes B oder C gebunden ist, darstellt:

Reduktion einer Verbindung der allgemeinen Formel

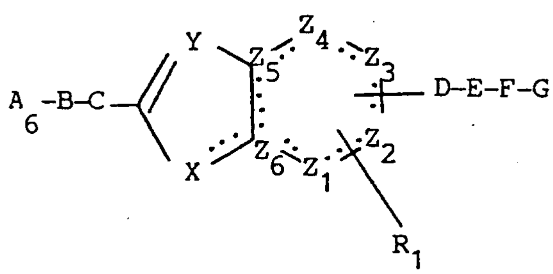

,(XIX)

in der

$R_1$, B bis G, X, Y und $Z_1$ bis $Z_6$ wie eingangs definiert sind und

$A_5$ eine Hydroxyimino- oder Hydroxyiminoalkylgruppe mit 1 bis 4 Kohlenstoffatomen darstellt.

Die Reduktion wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid, gegebenenfalls unter Zusatz einer Säure wie Salzsäure in Gegenwart von katalytisch angeregtem Wasserstoff, z.B. von Wasserstoff in Gegenwart von Raney-Nickel, Platin oder Palladium/Kohle, bei Temperaturen zwischen 0 und 100 °C, vorzugsweise bei Temperaturen zwischen 20 und 80 °C, durchgeführt.

m) Zur Herstellung von Verbindungen der allgemeinen Formel I, in der A eine durch eine Dialkylphosphorylgruppe mit jeweils 1 bis 3 Kohlenstoffatomen im Alkylteil substituierte Amino-, Aminoalkyl-, Amidino-, Guanidino- oder Guanidinoalkylgruppe darstellt:

Umsetzung einer Verbindung der allgemeinen Formel

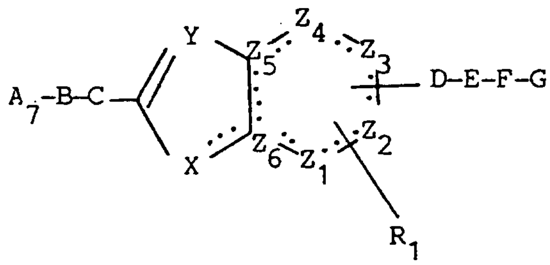

,(XX)

in der

$R_1$, B bis G, X, Y und $Z_1$ bis $Z_6$ wie eingangs definiert sind und

$A_7$ eine Aminogruppe, eine geradkettige oder verzweigte Aminoalkylgruppe mit 1 bis 4 Kohlenstoffatomen, eine Amidino-, Guanidino- oder Guanidinoalkylgruppe mit 1 bis 3 Kohlenstoffatomen im Alkylteil darstellt, mit einer Verbindung der allgemeinen Formel

$X_5$ - $PO(OR_{10})_2$ ,(XXI)

in der

$R_{10}$ eine Alkylgruppe mit 1 bis 3 Kohlenstoffatomen und

$X_5$ eine nukleophile Austrittsgruppe wie eine Cyanogruppe, ein Chlor- oder Bromatom darstellen.

Die Umsetzung wird zweckmäßigerweise in einem Lösungsmittel wie Dimethylformamid bei Temperaturen zwischen 0 und 100 ° C, vorzugsweise bei Temperaturen zwischen 15 und 50 ° C, durchgeführt.

n) Zur Herstellung von Verbindungen der allgemeinen Formel I, in der E eine -CO-$NR_3$-Gruppe darstellt:

Umsetzung einer Verbindung der allgemeinen Formel

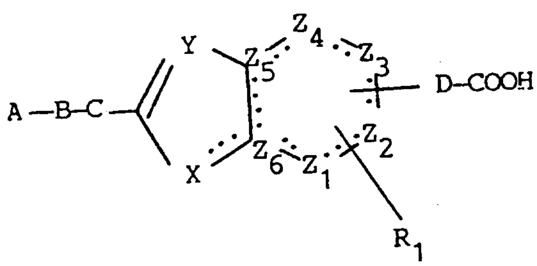

,(XXII)

in der

A bis D, X, Y und $Z_1$ bis $Z_6$ wie eingangs definiert sind, mit einer Verbindung der allgemeinen Formel

$HNR_3$ - F - G ,(XXIII)

in der

F, G und $R_3$ wie eingangs definiert sind.

Die Umsetzung wird zweckmäßigerweise in einem Lösungsmittel wie Methylenchlorid, Chloroform, Tetrachlorkohlenstoff, Ether, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureethylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Dicyclohexylcarbodiimid/1-Hydroxybenztriazol, 2-(1H-Benztriazol-1-yl)-1,1,3,3-tetramethyluronium-tetrafluorborat, N,N'-Carbonyldiimidazol oder N,N'-Thionyldiimidazol oder Triphenylphosphin/Tetrachlorkohlenstoff, gegebenenfalls in Gegenwart einer anorganischen Base wie Natriumcarbonat oder einer tertiären organischen Base wie Triethylamin, N-Methyl-morpholin, Pyridin oder 4-Dimethylaminopyridin, welche gleichzeitig

als Lösungsmittel dienen können, bei Temperaturen zwischen -25 und 150 ° C, vorzugsweise jedoch bei Temperaturen zwischen -10 ° C und der Siedetemperatur des verwendeten Lösungsmittels, durchgeführt.

Erhält man erfindungsgemäß eine Verbindung der allgemeinen Formel I, in der $R_1$ eine der eingangs erwähnten Amino- oder Aminoalkylgruppen darstellt, so kann diese mittels Alkylierung in eine entsprechende Alkylamino- oder Dialkylaminoverbindung der allgemeinen Formel I übergeführt werden.

Die nachträgliche Alkylierung wird zweckmäßigerweise in einem Lösungsmittel oder Lösungsmittelgemisch wie Methylenchlorid, Dimethylformamid, Dimethylsulfoxid, Toluol, Chlorbenzol, Tetrahydrofuran, Toluol/Tetrahydrofuran oder Dioxan gegebenenfalls in Gegenwart einer Base wie Natriumcarbonat, Kaliumcarbonat, Natronlauge, Kalium-tert.butylat oder N-Ethyl-diisopropylamin bei Temperaturen zwischen 0 und 150 ° C, vorzugsweise bei Temperaturen zwischen 0 und 50 ° C, durchgeführt.

Bei den vorstehend beschriebenen Umsetzungen können gegebenenfalls vorhandene reaktive Gruppen wie Hydroxy-, Carboxy-, Phosphono-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,
als Schutzreste für eine Carboxylgruppe die Trimethylsilyl-, Methyl-, Ethyl-, tert.Butyl-, Benzyl- oder Tetrahydropyranylgruppe,
als Schutzrest für eine Phosphonogruppe die Trimethylsilyl-, Methyl-, Ethyl- oder Benzylgruppe und
als Schutzrest für eine Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure, Bromwasserstoffsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100 ° C, vorzugsweise bei Temperaturen zwischen 10 und 50 ° C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 ° C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50 ° C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung nur eines Alkylrestes von einer O,O'-Dialkylphosphonogruppe erfolgt beispielsweise mit Natriumjodid in einem Lösungsmittel wie Aceton, Ethylmethylketon, Acetonitril oder Dimethylformamid bei Temperaturen zwischen 40 und 150 ° C, vorzugsweise jedoch bei Temperaturen zwischen 60 und 100 ° C.

Die Abspaltung beider Alkylreste von einer O,O'-Dialkylphosphonogruppe erfolgt beispielsweise mit Jodtrimethylsilan, Bromtrimethylsilan oder Chlortrimethylsilan/Natriumjodid in einem Lösungsmittel wie Methylenchlorid, Chloroform oder Acetonitril bei Temperaturen zwischen 0 ° C und der Siedetemperatur des Reaktionsgemisches, vorzugsweise jedoch bei Temperaturen zwischen 20 und 60 ° C.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50 ° C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit

mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihren aktivierten Derivaten oder Alkoholen, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Essigsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxylgruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Ammoniak, Cyclohexylamin, Äthanolamin, Diäthanolamin und Triäthanolamin in Betracht.

Die als Ausgangsstoffe verwendeten Verbindungen sind teilweise literaturbekannt oder man erhält diese nach literaturbekannten Verfahren wie diese in den Beispielen I bis XXIII beschrieben werden.

Wie bereits eingangs erwähnt, weisen die neuen kondensierten 5-gliedrigen Heterocyclen der allgemeinen Formel I und deren Additionssalze, insbesondere deren physiologisch verträgliche Additionssalze mit anorganischen oder organischen Säuren oder Basen, wertvolle Eigenschaften auf. So weisen die neuen Verbindungen der allgemeinen Formel I, in denen A eine gegebenenfalls am Stickstoff substituierte Amino-, Aminoalkyl-, Amidino-, Guanidino- oder Guanidinoalkylgruppe darstellt oder eine gegebenenfalls in vivo in eine gegebenenfalls am Stickstoff substituierte Amino-, Amidino- oder Guanidinogruppe überführbare Gruppe, z.B. eine durch eine Alkoxycarbonyl-, Alkenyloxycarbonyl-, Aralkoxycarbonyl-, Alkylcarbonyl-, Arylcarbonyl-, Aryloxycarbonyl-, Alkanoyloxymethoxycarbonyl-, Cycloalkanoyloxymethoxycarbonyl-, Aralkanoyloxymethoxycarbonyl-, Aryloxymethoxycarbonyl-, Phosphono-, Dialkylphosphoryl- oder O-Alkylphosphonogruppe am Stickstoff substituierte Amino-, Aminoalkyl-, Amidino-, Guanidino- oder Guanidinoalkylgruppe, enthält oder B eine am Stickstoffatom gegebenenfalls alkylierte cyclische Iminogruppe darstellt und -D-E-F Carboxyl-, Sulfo-, Phosphono-, O-Alkyl-phosphono- oder 5-Tetrazolylgruppen oder in vivo in Carboxyl-, Sulfo-, Phosphono-, O-Alkyl-phosphono- oder Tetrazolylgruppen überführbare Gruppen, z.B. durch eine Alkoxy-, Aralkoxy-, Aralkenyloxy-, Cycloalkylalkoxy-, Heteroarylalkoxy-, Pyrrolidin-2-on-1-yl-alkoxy-, Morpholinoalkoxy-, Thiomorpholinoalkoxy-, 1-Oxido-thiomorpholinoalkoxy-, Cycloalkoxy-, Benzocycloalkoxy-, Bicycloalkoxy-, Bicycloalkylalkoxy-, Alkanoyloxymethoxy-, Cycloalkylalkanoyloxymethoxy-, Alkoxycarbonyloxymethoxy-, Aroyloxymethoxy-, Aralkanoyloxymethoxy-, Aryloxycarbonyloxymethoxy- oder Aralkoxycarbonyloxymethoxygruppe substituierte Carbonylgruppen, enthält, wertvolle pharmakologische Eigenschaften auf, neben einer entzündungshemmenden und den Knochenabbau hemmenden Wirkung insbesondere antithrombotische, antiaggregatorische und tumor- bzw. metastasenhemmende Wirkungen.

Die Verbindungen der allgemeinen Formel I, in der A eine Cyanogruppe darstellt, stellen wertvolle Zwischenprodukte zur Herstellung der entsprechenden Aminomethyl- und Amidinoverbindungen der allgemeinen Formel I dar.

Beispielsweise wurden die Verbindungen der allgemeinen Formel I auf ihre biologischen Wirkungen wie folgt untersucht:

1. Fibrinogen-Bindung an Humanthrombozyten

Das durch Punktion einer Antekubitalvene gewonnene Blut wird mit Trinatriumcitrat (Endkonzentration: 13 mM) antikoaguliert und 10 Minuten bei 170 *g zentrifugiert. Das überstehende plättchenreiche Plasma

wird auf eine Sepharose 2B-Säule (Pharmacia) gegeben und mit einer Lösung aus 90 mM Kochsalz, 14 mM Trinatriumcitrat, 5 mM Glucose und 50 mM Tris(hydroxymethyl)aminomethan, eingestellt auf pH 7,4, eluiert. Die vor den Plasmaproteinen erscheinenden gelfiltrierten Plättchen (GFP) werden für die Bindungsversuche verwendet.

50 µl einer 60 mM Calziumchlorid-Lösung, 50 µl einer 0,6 mM Adenosindiphosphat-Lösung, 100 µl Substanzlösung bzw. Lösungsmittel und 50 µl Fibrinogenlösung (enthaltend 3 µg $^{125}$J-Fibrinogen) werden zu 750 µl GFP gegeben und bei Raumtemperatur 20 Minuten inkubiert. Die unspezifische Bindung wird in Gegenwart von 3 mg/ml kaltem Fibrinogen bestimmt.

900 µl des Inkubates werden vorsichtig auf 250 µl Silikonöl (AP 38: AR 20, 1:2 v/v, Wacker Chemie) in Eppendorf-Gefäße pipettiert und 2 Minuten bei 10 000 *g zentrifugiert. Der wäßrige Überstand und ein Teil des Öls werden abgezogen, die Gefäßspitze mit dem Plättchenpellet abgeschnitten und im Gamma-Zähler die Menge des gebundenen Fibrinogens bestimmt. Aus einer Konzentrationsreihe wird die Substanzkonzentration ermittelt, welche die Fibrinogenbindung zu 50 % hemmt und als $IC_{50}$ angegeben.

2. Antithrombotische Wirkung

Methodik

Die Thrombozytenaggregation wird nach der Methode von Born und Cross (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München. Vor der Collagen-Zugabe wird das Plasma jeweils 10 Minuten mit der Substanz bei 37° C inkubiert.

Aus den erhaltenen Meßzahlen wird graphisch eine $EC_{50}$ bestimmt, die sich auf eine 50%ige Änderung der "optical density" im Sinne einer Aggregationshemmung bezieht.

Die nachfolgende Tabelle enthält die gefundenen Ergebnisse:

| Substanz (Beispiel Nr.) | Fibrinogen-Bindungstest $IC_{50}$[nM] | Hemmung der Plättchenaggregation $EC_{50}$[nM] |
|---|---|---|
| 1 | 33 000 | 27 000 |
| 1(1) | 600 | 6 600 |
| 1(2) | 720* | 3 300 |
| 1(3) | 37 | 70 |
| 1(62) | 38* | |
| 1(73) | 49* | 160 |
| 1(74) | 120* | 310 |
| 1(75) | 230* | 1 100 |
| 1(76) | 28 | 1 150 |
| 1(79) | 41 | 80 |
| 1(135) | 800 | 7 600 |
| 1(136) | 30 | 80 |
| 1(137) | 1 800* | 7 000 |
| 1(138) | 1 500* | 3 600 |
| 1(139) | 410* | 1 500 |
| 1(140) | 220* | 750 |
| 1(160) | 7,5* | 70 |
| 3 | | 1 200 |
| 3(19) | 280* | 330 |
| 3(48) | 7 000* | 6 400 |
| 3(52) | 4 800* | 9 500 |
| 3(59) | 2 000* | 3 800 |
| 3(119) | 4 600* | 39 000 |
| 3(120) | 1 300* | 2 000 |
| 3(121) | 15 000* | 13 000 |
| 3(122) | 4 000* | 8 600 |
| 3(123) | 25 000* | 12 000 |
| 3(124) | 17 000* | 2 800 |
| 3(125) | 2 000 | 250 |

| Substanz (Beispiel Nr.) | Fibrinogen-Bindungstest $IC_{50}$[nM] | Hemmung der Plättchenaggregation $EC_{50}$[nM] |
|---|---|---|
| 3(126) | 2 200 | 9 900 |
| 3(130) | 14 000 | 160 |
| 3(141) | 140 | 80 |
| 5 | 15 000* | 43 000 |
| 5(6) | 5 500* | 18 000 |
| 6 | 4 000 | 3 700 |
| 13 | 1 700* | 5 700 |
| 13(3) | 1 400* | 4 300 |
| 13(6) | 130* | 980 |
| 15 | 28 | 40 |
| 15(1) | 2 500* | 4 100 |
| 17 | 1 000 | 3 800 |
| 17(1) | 700 | 1 530 |
| 17(2) | 22* | 80 |
| 17(3) | 170* | 430 |
| 17(4) | 1 900* | >100 000 |
| 17(5) | 370* | 2 130 |
| 17(6) | 1 800* | 30 000 |
| 17(7) | 5,4* | 290 |
| 17(8) | 47* | 350 |
| 17(9) | 9,5* | 1 140 |
| 17(10) | 26 000 | 27 000 |
| 17(13) | 20* | 40 |

* $^{125}I$ Fibrinogen wurde durch 3H-(3S,5S)-5-[(4'-Amidino-4-biphenylyl)-oxymethyl]-3-carboxymethyl-2-pyrrolidon ersetzt.

Die erfindungsgemäßen Verbindungen sind gut verträglich, da beispielsweise bei intravenöser Gabe von 30 mg/kg an jeweils drei Mäusen bei der Verbindung des Beispiels 1(3) kein Tier gestorben war.

Auf Grund ihrer Hemmwirkung auf Zell-Zell- bzw. Zell-Matrix-Wechselwirkungen eignen sich die neuen kondensierten 5-gliedrigen Stickstoffheterocyclen der allgemeinen Formel I und ihre physiologisch verträglichen Additionssalze zur Bekämpfung bzw. Verhütung von Krankheiten, bei denen kleinere oder größere Zell-Aggregate auftreten oder Zell-Matrixinteraktionen eine Rolle spielen, z. B. bei der Bekämpfung bzw. Verhütung von venösen und arteriellen Thrombosen, von zerebrovasculären Erkrankungen, von Lungenembolien, des Herzinfarkts, der Arteriosklerose, der Osteoporose und der Metastasierung von Tumoren und der

Therapie genetisch bedingter oder auch erworbener Störungen der Interaktion von Zellen untereinander oder mit soliden Strukturen. Weiterhin eignen sich diese bei der Begleittherapie bei der Thrombolyse mit Fibrinolytica oder Gefäßinterventionen wie transluminaler Angioplastie oder auch bei der Therapie von Schockzuständen, der Psoriasis, des Diabetes und von Entzündungen.

Für die Bekämpfung bzw. Verhütung der vorstehend erwähnten Krankheiten liegt die Dosis zwischen 0,1 µg und 30 mg/kg Körpergewicht, vorzugsweise bei 1 µg bis 15 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromboxan-Rezeptor-Antagonisten und Thromboxansynthesehemmer oder deren Kombinationen, Serotonin-Antagonisten, α-Rezeptorantagonisten, Alkylnitrate wie Glycerintrinitrat, Phosphodiesterasehemmer, Prostacyclin und dessen Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter Gerinnungsfaktoren, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Stearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Sprays oder Zäpfchen einarbeiten.

Die nachfolgenden Beispiele sollen die Erfindung näher erläutern:

Beispiel I

4-[N-(4-Amidino-benzoyl)-methylamino]-3-nitro-benzoesäure-[N-(3-methoxycarbonyl-propyl)-amid]

Hergestellt analog Beispiel 3 aus 4-[4-[N-(4-Cyan-benzoyl)-methylamino]-3-nitro-benzoesäure-[N-(3-methoxycarbonyl-propyl)-amid].
$R_f$-Wert: 0,11 (Kieselgel; Essigester/Ethanol = 7:3).
Analog wird folgende Verbindung erhalten:
(1) 4-[N-(4-Amidino-benzoyl)-methylamino]-3-nitro-benzoesäure-[N-(2-methoxycarbonyl-ethyl)-amid].
$R_f$-Wert: 0,11 (Kieselgel; Essigester/Ethanol = 7:3).

Beispiel II

4-[N-(4-Cyan-benzoyl)-methylamino]-3-nitro-benzoesäure-[N-(3-methoxycarbonyl-propyl)-amid]

Zu einer Mischung aus 3,2 g 4-[N-(4-Cyan-benzoyl)-methylamino]-3-nitro-benzoylchlorid, 1,68 g 4-Aminobuttersäuremethylester-hydrochlorid und 50 ml Methylenchlorid werden unter Kühlung auf 0°C 2,84 g N-Ethyl-diisopropylamin gegeben. Man rührt 64 Stunden, wobei man auf Raumtemperatur kommen läßt. Das Methylenchlorid wird abgedampft und der Rückstand über Kieselgel gereinigt (Elutionsmittel: Essigester/Ethanol = 200:1).
Ausbeute: 3,9 g (100 % der Theorie),
$R_f$-Wert: 0,62 (Kieselgel; Essigester/Ethanol = 9:1)
Analog werden folgende Verbindungen erhalten:
(1) 4-[N-(4-Cyan-benzoyl)-methylamino]-3-nitro-benzoesäure-[N-(2-methoxycarbonyl-ethyl)-amid]
$R_f$-Wert: 0,75 (Kieselgel; Essigester/Ethanol = 9:1)
(2) 4-[(4-Cyan-phenyl)-oxymethylcarbonylamino]-3-nitrophenoxy]-essigsäure-ethylester
Man verwendet 4-Dimethylamino-pyridin als Base.
Schmelzpunkt: 151-153°C
(3) 4-[2-(4-Benzyl-piperazino)-ethylamino]-3-(4-cyan-benzoylamino)-benzoesäure-[N-(2-methoxycarbonyl-ethyl)-amid]
Man arbeitet in Pyridin bei Raumtemperatur.
$R_f$-Wert: 0,73 (Kieselgel; Methylenchlorid/Methanol = 9:1, nach zweimaliger Entwicklung)
(4) 5-(4-Cyan-benzoylamino)-2,4-bis-methylamino-benzoesäure-[N-(2-methoxycarbonyl-ethyl)-amid]
Man arbeitet mit 4-Dimethylamino-pyridin als Base.
Schmelzpunkt: 208-210°C
$R_f$-Wert: 0,71 (Kieselgel; Methylenchlorid/Methanol/konz. Ammoniak = 9:1:0,1, nach dreimaliger Entwicklung)

(5) 4-Chlor-2-methoxy-5-nitro-benzoesäure-[N-(2-methoxycarbonyl-ethyl)-amid]
Man kocht eine Mischung aus 4-Chlor-2-methoxy-5-nitro-benzoyl-chlorid, β-Alanin-methylester-hydrochlorid und Toluol 48 Stunden unter Rückfluß.
Schmelzpunkt: 74-76 °C
$R_f$-Wert: 0,43 (Kieselgel; Methylenchlorid/Methanol = 30:1)
(6) 6-Methylamino-5-nitro-nicotinsäure-[N-(2-carboxy-ethyl)-amid]
Man arbeitet mit 1N Natronlauge bei Raumtemperatur, wobei das 6-Methylamino-5-nitro-nicotinsäurechlorid (hergestellt aus der Säure und Thionylchlorid) in Methylenchlorid gelöst zugegeben wird.
$R_f$-Wert: 0,47 (Reversed-Phase-Platte RP18; Methanol/5%ige Natriumchloridlösung = 6:4)
(7) 4-Chlor-3-nitro-benzoesäure-(2-carboxy-pyrrolidid)
Man verfährt wie unter (6).
$R_f$-Wert: 0,43 (Kieselgel; Essigester/Ethanol/Eisessig = 8:2:0,1)
(8) 4-Chlor-3-nitro-benzoesäure-(3-carboxy-piperidid)
Man verfährt wie unter (6).
Schmelzpunkt: 211-213 °C
$R_f$-Wert: 0,69 (Kieselgel; Essigester/Ethanol/Eisessig = 8:2:0,1)

## Beispiel III

### 4-[N-(4-Cyan-benzoyl)-methylamino]-3-nitro-benzoylchlorid

Eine Mischung bestehend aus 6,2 g 4-[N-(4-Cyan-benzoyl)-methylamino]-3-nitro-benzoesäure und 20 ml Thionylchlorid wird solange unter Rückfluß erhitzt, bis eine klare Lösung entstanden ist. Überschüssiges Thionylchlorid wird im Vakuum abgedampft und der Rückstand ohne zusätzliche Reinigung weiterverwendet.

Analog wird folgende Verbindung erhalten:
(1) 4-Chlor-2-methoxy-5-nitro-benzoylchlorid
Man kocht 18 Stunden unter Rückfluß. Das Produkt wird ohne Reinigung weiterverwendet.

## Beispiel IV

### 4-[N-(4-Cyan-benzoyl)-methylamino]-3-nitro-benzoesäure

Man löst 9,9 g 4-[N-(4-Cyan-benzoyl)-methylamino]-3-nitrobenzoesäure-methylester unter leichtem Erwärmen in einer Mischung aus 290 ml Tetrahydrofuran und 290 ml Wasser und fügt dann unter Rühren bei Raumtemperatur 29 ml 1N Lithiumhydroxid-Lösung zu. Man rührt noch 25 Minuten weiter, fügt 29 ml 1N Salzsäure zu und destilliert das Tetrahydrofuran im Vakuum ab, wobei das Produkt teilweise ausfallt. Die zurückbleibende wäßrige Phase wird salzsauer gestellt und mit Essigester extrahiert, wobei man nach Abdampfen des Essigesters eine weitere Fraktion erhält. Das Produkt wird durch Aufnehmen in heißem Tetrahydrofuran und Fällen mit Petrolether gereinigt.
Ausbeute: 7,5 g (79 % der Theorie),
Schmelzpunkt: 220-222 °C
$R_f$-Wert: 0,22 (Kieselgel; Essigester/Ethanol = 9:1)

## Beispiel V

### 4-[N-(4-Cyan-benzoyl)-methylamino]-3-nitro-benzoesäuremethylester

Eine Mischung aus 13,1 g 4-Methylamino-3-nitro-benzoesäuremethylester, 10,3 g 4-Cyan-benzoylchlorid und 75 ml Phosphoroxychlorid wird 8 Stunden bei 100 °C gerührt. Nach dem Abkühlen zersetzt man mit Wasser und extrahiert die wäßrige Phase mit Essigester. Die Essigesterphasen werden mit Sodalösung gewaschen und eingedampft. Der Rückstand wird in wenig Aceton gelöst und langsam mit Ether versetzt. Die anfänglich ausgefallenen Mengen (etwa 1,2 g) werden verworfen. Nach Zusatz von weiterem Ether fällt die gewünschte Substanz aus.
Ausbeute: 10,2 g (48 % der Theorie),
$R_f$-Wert: 0,54 (Kieselgel; Essigester/Petrolether = 7:3)

Beispiel VI

5(6)-Amino-2-(4-cyan-phenyl)-benzimidazol

4,8 g 2-(4-Cyan-phenyl)-5(6)-nitro-benzimidazol werden in einer Mischung aus 150 ml Ethanol und 20 ml Dimethylformamid gelöst, mit 1 g 5%iger Palladiumkohle versetzt und mit Wasserstoff von 5 bar bei Raumtemperatur behandelt. Nach 4 und 21 Stunden wird jeweils noch 1 g Katalysator zugesetzt. Nach insgesamt 24 Stunden filtriert man vom Katalysator ab, engt ein, nimmt den Rückstand in Essigester auf und extrahiert mit 0,1 N Salzsäure. Die wäßrigen Phasen werden mit Natriumbicarbonat alkalisch gestellt und mit Essigester extrahiert. Der nach dem Eindampfen der Essigesterphasen verbleibende Rückstand wird mit Wasser kristallin gerieben.
Ausbeute: 1,6 g (40 % der Theorie),
Schmelzpunkt: 128-130 °C (sintert ab 118 °C)
Analog werden folgende Verbindung erhalten:

(1) 3-Amino-4-[(4-Cyan-phenyl)-oxymethylcarbonylamino]-phenoxy-essigsäure-ethylester
Als Lösungsmittel wird ein 1:3,5-Gemisch aus Essigester und Tetrahydrofuran verwendet.
$R_f$-Wert: 0,59 (Kieselgel; Methylenchlorid/Methanol/Eisessig: 50:1:0,1 nach zweimaliger Entwicklung)

(2) 3-Amino-4-(3-thiomorpholino-propylamino)-benzoesäure-[N-(2-methoxycarbonyl-ethyl)-amid]-hydrochlorid
Man arbeitet in Methanol mit 10%iger Palladiumkohle.
$R_f$-Wert: 0,67 (Kieselgel; Methylenchlorid/Methanol/konz. Ammoniak = 9:1:0,1, nach zweimaliger Entwicklung)

(3) 3-Amino-4-(3-amino-propylamino)-benzoesäure-[N-(2-methoxycarbonyl-ethyl)-amid]-hydrochlorid-hydrogenacetat
Man arbeitet in Eisessig mit 10%iger Palladiumkohle.
$R_f$-Wert: 0,23 (Kieselgel; Methylenchlorid/Methanol/Eisessig = 2:1:0,1, nach zweimaliger Entwicklung)

(4) 3-Amino-4-methylamino-benzoesäure-[N-(2-methoxycarbonylethyl)-amid]
Man verfährt wie unter (2).
$R_f$-Wert: 0,28 (Kieselgel; Essigester/Ethanol = 50:2)

(5) 3-Amino-4-[2-(3,4-dimethoxy-phenyl)-ethylamino]-benzoesäure-[N-(2-methoxycarbonyl-ethyl)-amid]
Man verfährt wie unter (2).
$R_f$-Wert: 0,53 (Kieselgel; Essigester/Ethanol = 50:2)

(6) 3-Amino-4-(3-pyridylmethylamino)-benzoesäure-[N-(2-methoxycarbonyl-ethyl)-amid]
Man verfährt wie unter (2).
$R_f$-Wert: 0,07 (Kieselgel; Essigester/Ethanol = 9:1)

(7) 3-Amino-4-n-tetradecylamino-benzoesäure-[N-(2-methoxycarbonyl-ethyl)-amid]
Man verfährt wie unter (2).
$R_f$-Wert: 0,55 (Kieselgel; Essigester/Ethanol = 50:2)

(8) 3-Amino-4-(3-methoxycarbonyl-propylamino)-benzoesäure-[N-(2-methoxycarbonyl-ethyl)-amid]-hydrochlorid
Man verfährt wie unter (2) unter Zusatz von etherischer Salzsäure.
$R_f$-Wert: 0,65 (Kieselgel; Methylenchlorid/Methanol = 9:1)

(9) 3-Amino-4-(4-phenyl-butylamino)-benzoesäure-[N-(2-methoxycarbonyl-ethyl)-amid]
Man verfährt wie unter (2).
$R_f$-Wert: 0,68 (Kieselgel; Essigester/Ethanol = 9:1)

(10) 5-Amino-2,4-bis-methylamino-benzoesäure-[N-(2-methoxycarbonyl-ethyl)-amid]-hydrochlorid
Man arbeitet in Tetrahydrofuran mit 10%iger Palladiumkohle.
Schmelzpunkt: 138-140 °C (Zers.)
$R_f$-Wert: 0,40 (Kieselgel; Methylenchlorid/Methanol/konz. Ammoniak = 19:1:0,1, nach zweimaliger Entwicklung)

(11) 3-Amino-4-[(3,3-diphenyl-propyl)-amino]-benzoesäure-[N-(2-methoxycarbonyl-ethyl)-amid]
Man verfährt wie unter (2).
$R_f$-Wert: 0,69 (Kieselgel; Essigester/Ethanol = 9:1)

(12) 5-Amino-6-methylamino-nicotinsäure-[N-(2-methoxycarbonyl-ethyl)-amid]
Man verfährt wie unter (2).
$R_f$-Wert: 0,77 (Reversed-Phase-Platte RP18; Methanol/5%ige Natriumchloridlösung = 6:4)

(13) 3-Amino-4-methylamino-benzoesaure-(2-methoxycarbonylpyrrolidid)
Man verfährt wie unter (2).

$R_f$-Wert: 0,41 (Kieselgel; Essigester/Ethanol = 9:1)
(14) 3-Amino-4-methylamino-benzoesäure-(3-methoxycarbonylpiperidid)
Man verfährt wie unter (2).
$R_f$-Wert: 0,48 (Kieselgel; Essigester/Ethanol = 7:3)

Beispiel VII

2-(4-Cyan-phenyl)-5(6)-nitro-benzimidazol

21,9 g 3,4-Diamino-nitrobenzol werden bei Raumtemperatur portionsweise unter Rühren in 400 ml Phosphoroxychlorid eingetragen. Dann gibt man 23,7 g 4-Cyan-benzoylchlorid zu. Man erwärmt zunächst 30 Minuten auf 90°C und dann 6 Stunden auf 100°C. Der größe Teil des überschüssigen Phosphoroxychlorids wird im Vakuum abgedampft, der Rückstand mit Wasser behandelt und der gebildete Niederschlag in Essigester/Wasser aufgenommen. Man stellt mit Natriumbicarbonat alkalisch und engt die organische Phase schließlich ein.
Ausbeute: 4,8 g (13 % der Theorie),
Schmelzpunkt: über 200°C
$R_f$-Wert: 0,74 (Kieselgel; Essigester/Ethanol = 50:2)

Beispiel VIII

4-Amino-3-nitro-phenoxy-essigsäure-ethylester

25 g 4-Amino-3-nitro-phenol werden in 250 ml Dimethylformamid gelöst und unter Eiskühlung portionsweise mit 18,5 g Kalium-tert.butylat versetzt. Man rührt noch 1,5 Stunden nach, tropft dann unter Kühlung mit Wasser 18,5 ml Bromessigsäure-ethylester zu und rührt 16 Stunden bei Raumtemperatur nach. Das Lösungsmittel wird im Vakuum abgedampft und der Rückstand mit einem Gemisch aus 500 ml Essigester, 250 ml Tetrahydrofuran, 750 ml Wasser aufgenommen, und die organische Phase mit gesättigter Kochsalzlösung gewaschen. Die wäßrigen Phasen werden mit Essigester extrahiert, die organischen Phasen vereinigt und eingedampft und der Rückstand säulenchromatographisch (neutrales Aluminiumoxid; Elutionsmittel: Methylenchlorid) gereinigt.
Ausbeute: 29 g (75 % der Theorie),
Schmelzpunkt: 110-113°C

Beispiel IX

3-Amino-4-[2-(4-benzyl-piperazino)-ethylamino]-benzoesäure-[N-(2-methoxycarbonyl-ethyl)-amid]

6,3 g 4-[2-(4-Benzyl-piperazino)-ethylamino]-3-nitro-benzoesäure-[N-(2-methoxycarbonyl-ethyl)-amid]-dihydrochlorid werden auf dem Dampfbad in einer Mischung aus 100 ml Eisessig und 25 ml Wasser gelöst und innerhalb von 10 Minuten mit 4 g Eisenpulver versetzt. Man erhitzt weitere 30 Minuten, filtriert über Kohle und engt das Filtrat ein. Der Rückstand wird in 100 ml Tetrahydrofuran aufgenommen und über eine Kieselgelsäule gegeben, wobei man mit Tetrahydrofuran nacheluiert. Die Fraktionen, die das Produkt enthalten, werden zur Trockne eingedampft und mit Wasser verrieben. Der kristalline Rückstand wird abfiltriert, mit Wasser, Methanol und Ether gewaschen. Das Produkt wird ohne weitere Reinigung weiter verarbeitet.
Ausbeute: 1,2 g (20 % der Theorie),
Schmelzpunkt: 132-134°C (Zers.)
$R_f$-Wert: 0,63 (Kieselgel; Methylenchlorid/Methanol = 8:2)

Beispiel X

4-[2-(4-Benzyl-piperazino)-ethylamino]-3-nitro-benzoesäure-[N-(2-methoxycarbonyl-ethyl)-amid]-dihydrochlorid

9,2 g 4-[2-(4-Benzyl-piperazino)-ethylamino]-3-nitro-benzoesäure-[N-(2-carboxy-ethyl)-amid] werden in 1,5 l Methanol unter Erwärmen suspendiert, auf -20°C abgekühlt, mit 1,7 ml Thionylchlorid versetzt, 2 Stunden bei -10°C gehalten und dann 16 Stunden bei Raumtemperatur gerührt. Man engt im Vakuum auf

150 ml ein, filtriert das Kristallisat ab und wäscht mit Methanol und Ether nach.
Ausbeute: 6,5 g (64 % der Theorie),
Schmelzpunkt: 225-227 ° C (Zers.)
$R_f$-Wert: 0,43 (Kieselgel; Methylenchlorid/Methanol/konz. Ammoniak = 9:1:0,1)
Analog werden folgende Verbindungen erhalten:

(1) 3-Nitro-4-(3-thiomorpholino-propylamino)-benzoesäure-[N-(2-methoxycarbonyl-ethyl)-amid]-hydrochlorid
Man arbeitet in Methanol unter Zusatz von etherischer Salzsäure bei Raumtemperatur.
$R_f$-Wert: 0,59 (Kieselgel; Methylenchlorid/Methanol/konz. Ammoniak = 19:1:0,1, nach zweifacher Entwicklung)

(2) 4-(3-Amino-propylamino)-3-nitro-benzoesäure-[N-(2-methoxycarbonyl-ethyl)-amid]-hydrochlorid
Man arbeitet wie unter (1).
Schmelzpunkt: 178-180 ° C (Zers.)
$R_f$-Wert: 0,41 (Kieselgel; n-Butanol/Eisessig/Wasser = 4:1:1)

(3) 4-Methylamino-3-nitro-benzoesäure-[N-(2-methoxycarbonylethyl)-amid]
Man arbeitet wie unter (1).
Schmelzpunkt: 125-127 ° C
$R_f$-Wert: 0,35 (Kieselgel; Essigester/Ethanol = 50:1)

(4) 4-[2-(3,4-Dimethoxy-phenyl)-ethylamino]-3-nitro-benzoesäure-[N-(2-methoxycarbonyl-ethyl)-amid]
Man arbeitet wie unter (1).
Schmelzpunkt: 112-115 ° C
$R_f$-Wert: 0,71 (Kieselgel; Essigester/Ethanol = 50:2)

(5) 3-Nitro-4-(3-pyridylmethylamino)-benzoesäure-[N-(2-methoxycarbonyl-ethyl)-amid]
Man arbeitet wie unter (1).
$R_f$-Wert: 0,59 (Reversed Phase Platte RP 18; Methanol/5%ige Natriumchloridlösung = 6:4)

(6) 3-Nitro-4-n-tetradecylamino-benzoesäure-[N-(2-methoxycarbonyl-ethyl)-amid]
Man arbeitet wie unter (1).
$R_f$-Wert: 0,78 (Kieselgel; Essigester/Ethanol = 50:2)

(7) 4-(3-Methoxycarbonyl-propylamino)-3-nitro-benzoesäure-[N-(2-methoxycarbonyl-ethyl)-amid]
Man arbeitet wie unter (1).
$R_f$-Wert: 0,80 (Kieselgel; Methylenchlorid/Methanol = 9:1)

(8) 3-Amino-2-benzylamino-benzoesäure-[N-(2-methoxycarbonylethyl)-amid]
Man arbeitet wie unter (1).
$R_f$-Wert: 0,69 (Kieselgel; Methylenchlorid/Methanol = 9:1)

(9) 3-Nitro-4-(4-phenyl-butylamino)-benzoesäure-[N-(2-methoxycarbonyl-ethyl)-amid]
Man arbeitet wie unter (1).
$R_f$-Wert: 0,84 (Kieselgel; Essigester/Ethanol = 9:1)

(10) 2,4-bis-Methylamino-5-nitro-benzoesäure-[N-(2-methoxycarbonyl-ethyl)-amid]-hydrochlorid
Man arbeitet wie unter (1).
$R_f$-Wert: 0,51 (Kieselgel; Methylenchlorid/Methanol = 19:1)

(11) 3-Amino-4-[2-(4-amino-3,5-dibrom-phenyl)-ethylamino]-benzoesäure-[N-(2-methoxycarbonyl-ethyl)-amid]-hydrochlorid
Man arbeitet wie unter (1).
Schmelzpunkt: 135-140 ° C (Zers.)
$R_f$-Wert: 0,73 (Kieselgel; Essigester/Ethanol = 8:2)

(12) 4-[(3,3-Diphenyl-propyl)-amino]-3-nitro-benzoesäure-[N-(2-methoxycarbonyl-ethyl)-amid]
Man arbeitet wie unter (1).
$R_f$-Wert: 0,86 (Kieselgel; Essigester/Ethanol = 9:1)

(13) 6-Methylamino-5-nitro-nicotinsäure-[N-(2-methoxycarbonyl-ethyl)-amid]
Man arbeitet wie unter (1).
$R_f$-Wert: 0,30 (Reversed-Phase-Platte RP18; Methanol/5%ige Natriumchloridlösung = 6:4)

(14) 4-Methylamino-3-nitro-benzoesäure-(2-methoxycarbonylpyrrolidid)
Man arbeitet wie unter (1).
$R_f$-Wert: 0,57 (Kieselgel; Essigester/Ethanol = 9:1)

(15) 4-Methylamino-3-nitro-benzoesäure-(3-methoxycarbonylpiperidid)
Man arbeitet wie unter (1).
$R_f$-Wert: 0,67 (Kieselgel; Essigester/Ethanol = 9:1)

Beispiel XI

4-[2-(4-Benzyl-piperazino)-ethylamino]-3-nitro-benzoesäure-[N-(2-carboxy-ethyl)-amid]

8,2 g 4-Chlor-3-nitro-benzoesäure-[N-(2-carboxy-ethyl)-amid] (hergestellt aus 4-Chlor-3-nitro-benzoylchlorid und β-Alaninmethylester und anschließende Verseifung mit 48%iger Bromwasserstoffsäure), 6,6 g 2-(4-Benzyl-piperazino)-ethylamin (hergestellt aus 1-Benzyl-piperazin und 2-Brom-ethylamin-hydrobromid in Ethanol unter Zusatz von Kalium-tert.butylat), 4,2 ml Triethylamin und 25 ml Wasser werden 8 Stunden auf dem Dampfbad erhitzt. Man dampft ein und reinigt säulenchromatographisch (Kieselgel; Methylenchlorid/Methanol/Eisessig = 8:2:0,1 bis 2:1:0,1).
Ausbeute: 9,2 g (67 % der Theorie),
$R_f$-Wert: 0,47 (Kieselgel; Methylenchlorid/Methanol/konz. Ammoniak = 8:2:0,1)
Analog werden folgende Verbindungen erhalten:

(1) 3-Nitro-4-(3-thiomorpholino-propylamino)-benzoesäure-[N-(2-carboxy-ethyl)-amid]
Schmelzpunkt: 207-211 °C (Zers.)
$R_f$-Wert: 0,33 (Kieselgel; Methylenchlorid/Methanol/konz. Ammoniak = 8:2:0,1, nach viermaliger Entwicklung)

(2) 4-(3-Amino-propylamino)-3-nitro-benzoesäure-[N-(2-carboxy-ethyl)-amid]
Man arbeitet ohne Hilfsbase aber mit der doppelten molaren Menge an 1,3-Diaminopropan
Schmelzpunkt: 136 °C (Zers., sintert ab 118 °C)
$R_f$-Wert: 0,35 (Kieselgel; Isopropanol/Wasser/konz. Ammoniak = 7:2:1)

(3) 4-Methylamino-3-nitro-benzoesäure-[N-(2-carboxy-ethyl)-amid]
Man arbeitet mit 40%iger Methylaminlösung als Lösungsmittel und erwärmt 8 Stunden auf 50 °C in der Bombe.
Schmelzpunkt: 187-190 °C
$R_f$-Wert: 0,22 (Kieselgel; Essigester/Ethanol = 50:1)

(4) 4-[2-(3,4-Dimethoxy-phenyl)-ethylamino]-3-nitro-benzoesäure-[N-(2-carboxy-ethyl)-amid]-hydrochlorid
Man arbeitet ohne Lösungsmittel und Hilfsbase bei 90 °C Badtemperatur.
Schmelzpunkt: 105-107 °C
$R_f$-Wert: 0,35 (Kieselgel; Essigester/Ethanol/Eisessig = 9:1:0,01)

(5) 3-Nitro-4-(3-pyridylmethylamino)-benzoesäure-[N-(2-carboxy-ethyl)-amid]
Man arbeitet ohne Hilfsbase mit der dreifachen Menge an 3-Picolylamin.
Schmelzpunkt: 110 °C (sintert ab 95 °C)
$R_f$-Wert: 0,64 (Reversed Phase Platte RP18; Methanol/5%ige Natriumchloridlösung = 6:4)

(6) 3-Nitro-4-n-tetradecylamino-benzoesäure-[N-(2-carboxyethyl)-amid]
Man arbeitet ohne Lösungsmittel.
$R_f$-Wert: 0,59 (Kieselgel; Essigester/Ethanol/Eisessig = 9:1:0,02)

(7) 4-(3-Carboxy-propylamino)-3-nitro-benzoesäure-[N-(2-carboxy-ethyl)-amid]
Man arbeitet in Wasser unter Zusatz von Kaliumhydrogencarbonat.
$R_f$-Wert: 0,42 (Kieselgel; Methylenchlorid/Methanol = 9:1)

(8) 4-Benzylamino-3-nitro-benzoesäure-[N-(2-carboxy-ethyl)-amid]
Man arbeitet ohne Lösungsmittel.
$R_f$-Wert: 0,76 (Kieselgel; Methylenchlorid/Methanol = 9:1)

(9) 3-Nitro-4-(4-phenyl-butylamino)-benzoesäure-[N-(2-carboxy-ethyl)-amid]
Man arbeitet ohne Lösungsmittel mit N-Ethyl-diisopropylamin als Hilfsbase.
$R_f$-Wert: 0,42 (Kieselgel; Essigester/Ethanol/Eisessig = 9:1:0,02)

(10) 2,4-bis-Methylamino-5-nitro-benzoesäure-[N-(2-carboxyethyl)-amid]-methylaminsalz
Man arbeitet wie unter (3) mit 12-stündiger Reaktionsdauer, als Ausgangsmaterial dient 4-Chlor-2-methoxy-5-nitro-benzoesäure-[N-(2-carboxy-ethyl)-amid]
Schmelzpunkt: 180-189 °C (Zers.)
$R_f$-Wert: 0,62 (Kieselgel; Methylenchlorid/Methanol/Eisessig = 19:1:0,1)

(11) 4-[2-(4-Amino-3,5-dibrom-phenyl)-ethylamino]-3-nitrobenzoesäure-[N-(2-carboxy-ethyl)-amid]
Man arbeitet wie unter (7).
Schmelzpunkt: 110-115 °C
$R_f$-Wert: 0,65 (Kieselgel; Essigester/Ethanol/Eisessig = 7:3:0,02)

(12) 4-[(3,3-Diphenyl-propyl)-amino]-3-nitro-benzoesäure-[N-(2-carboxy-ethyl)-amid]
Man arbeitet wie unter (9).
Schmelzpunkt: 185 °C (sintert ab 165 °C)

$R_f$-Wert: 0,59 (Kieselgel; Essigester/Ethanol/Eisessig = 9:1:0,02)
(13) 4-Methylamino-3-nitro-benzoesäure-(2-carboxy-pyrrolidid) Man arbeitet wie unter (3).
$R_f$-Wert: 0,12 (Kieselgel; Essigester/Ethanol = 9:1)
(14) 4-Methylamino-3-nitro-benzoesäure-(3-carboxy-piperidid) Man arbeitet wie unter (3).
$R_f$-Wert: 0,33 (Kieselgel; Essigester/Ethanol = 9:1)

Beispiel XII

3-Amino-4-benzylamino-benzoesäure-[N-(2-carboxy-ethyl)-amid]

4,0 g 4-Benzylamino-3-nitro-benzoesäure-[N-(2-carboxy-ethyl)-amid] werden in 40 ml 5%iger Natronlauge heiß gelöst und portionsweise mit 5,0 g Natriumdithionit versetzt. Nachdem sich die Lösung entfärbt hat, rührt man noch 15 Minuten nach, läßt abkühlen und filtriert den ausgefallenen Niederschlag ab. Das Filtrat wird mit 2N Salzsäure schwach sauer gestellt, wobei das Produkt ausfällt. Es wird abgesaugt und mit Wasser ge- waschen.
Ausbeute: 1,6 g (52 % der Theorie),
$R_f$-Wert: 0,26 (Kieselgel; Methylenchlorid/Methanol = 9:1)
Analog wird folgende Verbindung erhalten:
(1) 3-Amino-4-[2-(4-amino-3,5-dibrom-phenyl)-ethylamino]-benzoesäure-[N-(2-carboxy-ethyl)-amid]-hydrochlorid
Schmelzpunkt: 150-155 °C
$R_f$-Wert: 0,22 (Kieselgel; Essigester/Ethanol = 8:2)

Beispiel XIII

4-Chlor-2-methoxy-5-nitro-benzoesäure-[N-(2-carboxy-ethyl)-amid]

Man verfährt analog Beispiel 17.
Schmelzpunkt: 170-172 °C
$R_f$-Wert: 0,38 (Kieselgel; Methylenchlorid/Methanol/Eisessig = 30:1:0,1, nach zweimaliger Entwicklung)

Beispiel 1

2-(4-Amidino-phenyl)-5-[(3-carboxy-propyl)-aminocarbonyl]-1-methyl-benzimidazol

Eine Mischung aus 2,0 g 2-(4-Amidino-phenyl)-5-[(3-methoxycarbonyl-propyl)-aminocarbonyl]-1-methyl-benzimidazol, 20 ml Methanol und 15,8 ml 1N Natronlauge wird 16 Stunden bei Raumtemperatur gerührt. Man dampft im Vakuum ein, nimmt mit Wasser auf und stumpft durch Zusatz von Ammoniumchlorid ab. Der ausgefallene Niederschlag wird abfiltriert.
Ausbeute: 1,3 g (68 % der Theorie),
Schmelzpunkt: über 215 °C
$R_f$-Wert: 0,75 (Reversed-Phase-Platte RP18; Methanol/5%ige wäßrige Natriumchloridlösung = 6:4)
Analog werden folgende Verbindungen erhalten:
(1) 2-(4-Amidino-phenyl)-5(6)-[(2-carboxy-ethyl)-carbonylamino]-benzimidazol
Schmelzpunkt: über 200 °C
$R_f$-Wert: 0,63 (Reversed-Phase-Platte RP18; Methanol/5%ige wäßrige Natriumchloridlösung = 6:4)

| | | | | | | |
|---|---|---|---|---|---|---|
| Ber. x 3 $H_2O$: | C | 53,46 | H | 5,73 | N | 17,31 |
| Gef.: | | 53,67 | | 5,54 | | 17,29 |

(2) 2-(4-Amidino-phenyl)-5(6)-[(2-carboxy-ethyl)-aminocarbonyl]-benzimidazol-hydrochlorid
Man verwendet Lithiumhydroxid
$R_f$-Wert: 0,26 (Kieselgel; Methylenchlorid/Methanol = 7:3)
(3) 2-(4-Amidino-phenyl)-5-[(2-carboxy-ethyl)-aminocarbonyl]-1-methyl-benzimidazol
Schmelzpunkt: 247-249 °C
$R_f$-Wert: 0,70 (Reversed-Phase-Platte RP18; Methanol/5%ige wäßrige Natriumchloridlösung = 6:4)
(4) 2-(4-Amidino-phenyl)-5(6)-[(3-carboxy-propyl)-carbonylamino]-benzimidazol

(5) 2-(4-Aminomethyl-phenyl)-5-[(3-carboxy-propyl)-aminocarbonyl]-1-methyl-benzimidazol
(6) 2-(4-Aminomethyl-phenyl)-5(6)-[(2-carboxy-ethyl)-aminocarbonyl]-benzimidazol
(7) 2-(4-Aminomethyl-phenyl)-5-[(2-carboxy-ethyl)-aminocarbonyl]-1-methyl-benzimidazol
(8) 2-(4-Amidino-phenyl)-6-[(2-carboxy-ethyl)-aminocarbonyl]-1-methyl-benzimidazol
(9) 2-(4-Amidino-phenyl)-5-[(3-carboxy-propyl)-carbonyl]-1-methyl-benzimidazol
(10) 2-(4-Amidino-phenyl)-5-[(4-carboxy-butyl)-carbonyl]-1-methyl-benzimidazol
(11) 2-[4-(2-Amino-ethyl)-phenyl]-5-[(3-carboxy-propyl)-carbonyl]-1-methyl-benzimidazol
(12) 2-(4-Guanidinomethyl-phenyl)-5-[(2-carboxy-ethyl)-carbonyl]-1-methyl-benzimidazol
(13) 5-[(3-Carboxy-propyl)-aminocarbonyl]-2-(3-guanidino-phenyl)-1-methyl-benzimidazol
(14) 2-[4-(2-Amino-ethyl)-phenyl]-5-[(2-carboxy-ethyl)-carbonyl]-1-methyl-benzimidazol
(15) 2-(4-Amidino-phenyl)-5(6)-[(2-carboxy-ethyl)-thio]-benzimidazol
(16) 2-(4-Amidino-phenyl)-5(6)-[(2-carboxy-ethyl)-sulfinyl]-benzimidazol
(17) 2-(4-Amidino-phenyl)-5(6)-[(2-carboxy-ethyl)-sulfonyl]-benzimidazol
(18) 2-(4-Amidino-phenyl)-5(6)-[(3-carboxy-propyl)-thio]-benzimidazol
(19) 2-(4-Amidino-phenyl)-5(6)-[(3-carboxy-propyl)-sulfinyl]-benzimidazol
(20) 2-(4-Amidino-phenyl)-5(6)-[(3-carboxy-propyl)-sulfonyl]-benzimidazol
(21) 2-(4-Amidino-phenyl)-5(6)-[(3-carboxy-propyl)-amino]-benzimidazol
(22) 5(6)-[N-Acetyl-N-(3-carboxy-propyl)-amino]-2-(4-amidinophenyl)-benzimidazol
(23) 2-(4-Amidino-phenyl)-5(6)-[(3-carboxy-propyl)-oxy]-benzimidazol
(24) 2-(4-Amidino-phenyl)-5(6)-(4-carboxy-butyl)-benzimidazol
(25) 2-(4-Amidino-phenyl)-5(6)-[(2-carboxy-ethyl)-aminomethyl]-benzimidazol
(26) 2-(4-Amidino-phenyl)-5(6)-[(2-carboxy-ethyl)-thiomethyl]-benzimidazol
(27) 2-(4-Amidino-phenyl)-5(6)-[(2-carboxy-ethyl)-sulfinylmethyl]-benzimidazol
(28) 2-(4-Amidino-phenyl)-5(6)-[(2-carboxy-ethyl)-sulfonylmethyl]-benzimidazol
(29) 5(6)-[N-Acetyl-N-(2-carboxy-ethyl)-aminomethyl]-2-(4-amidino-phenyl)-benzimidazol
(30) 2-(4-Amidino-phenyl)-5(6)-(carboxymethylcarbonyl-aminomethyl)-benzimidazol
(31) 2-(4-Amidino-phenyl)-5(6)-(4-carboxy-1-buten-1-yl)-benzimidazol
(32) 2-(4-Amidino-phenyl)-6-[(2-carboxy-ethyl)-aminocarbonyl]-3-methyl-imidazo[4,5-b]pyridin
$R_f$-Wert: 0,74 (Reversed Phase Platte RP18; Methanol/5%ige Natriumchloridlösung = 6:4)
(33) 2-(4-Amidino-phenyl)-6-[(2-carboxy-ethyl)-aminocarbonyl]-imidazo[4,5-b]pyridin
(34) 2-(4-Amidino-phenyl)-6-[(2-carboxy-ethyl)-aminocarbonyl]-imidazo[1,2-a]pyridin
(35) 2-(4-Amidino-phenyl)-6-[(2-carboxy-ethyl)-aminocarbonyl]-3-methyl-imidazo[4,5-c]pyridin
(36) 2-(4-Aminomethyl-phenyl)-6-[(2-carboxy-ethyl)-aminocarbonyl]-3-methyl-imidazo[4,5-b]pyridin
(37) 2-(4-Amidino-phenyl)-6-[(2-carboxy-ethyl)-aminocarbonyl]-imidazo[4,5-c]pyridazin
(38) 2-(4-Amidino-phenyl)-5-[(2-carboxy-ethyl)-aminocarbonyl]-imidazo[4,5-b]pyrazin
(39) 8-(4-Amidino-phenyl)-2-[(2-carboxy-ethyl)-aminocarbonyl]-9-methyl-purin
(40) 8-(4-Amidino-phenyl)-2-[(2-carboxy-ethyl)-aminocarbonyl]-6-chlor-9-methyl-purin
(41) 8-(4-Amidino-phenyl)-2-[(2-carboxy-ethyl)-aminocarbonyl]-6-methoxy-9-methyl-purin
(42) 8-(4-Amidino-phenyl)-2-[(2-carboxy-ethyl)-aminocarbonyl]-6-dimethylamino-9-methyl-purin
(43) 8-(4-Amidino-phenyl)-6-amino-2-[(2-carboxy-ethyl)-aminocarbonyl]-9-methyl-purin
(44) 8-(4-Amidino-phenyl)-2-[(2-carboxy-ethyl)-aminocarbonyl]-9-methyl-6-piperidino-purin
(45) 8-(4-Amidino-phenyl)-2-[(2-carboxy-ethyl)-aminocarbonyl]-9-methyl-hypoxanthin
(46) 2-(4-Amidino-phenyl)-6-[(2-carboxy-ethyl)-aminocarbonyl]-imidazo[1,2-a]pyrimidin
(47) 2-(4-Amidino-phenyl)-5-[N-(2-carboxy-ethyl)-methylaminocarbonyl]-1-methyl-benzimidazol
(48) 2-(4-Amidino-phenyl)-5-[(2-carboxy-ethyl)-aminocarbonyl]-7-fluor-1-methyl-benzimidazol
(49) 2-(4-Amidino-phenyl)-6-[(2-carboxy-ethyl)-aminocarbonyl]-4-chlor-benzimidazol
(50) 2-(4-Amidino-phenyl)-4-brom-6-[(2-carboxy-ethyl)-aminocarbonyl]-benzimidazol
(51) 2-(4-Amidino-phenyl)-5-[(2-carboxy-ethyl)-aminocarbonyl]-6-hydroxy-1-methyl-benzimidazol
(52) 2-(4-Amidino-phenyl)-5-[(2-carboxy-ethyl)-aminocarbonyl]-6-methoxy-1-methyl-benzimidazol
(53) 2-(4-Amidino-phenyl)-5-[(2-carboxy-ethyl)-aminocarbonyl]-1-methyl-6-propyloxy-benzimidazol
(54) 2-(4-Amidino-phenyl)-6-[(2-carboxy-ethyl)-aminocarbonyl]-4-methyl-benzimidazol
(55) 2-(4-Amidino-phenyl)-5-[(2-carboxy-ethyl)-aminocarbonyl]-1-methyl-6-methansulfonylamino-benzimidazol
(56) 6-Acetylamino-2-(4-amidino-phenyl)-5-[(2-carboxy-ethyl)-aminocarbonyl]-1-methyl-benzimidazol
(57) 2-(4-Amidino-phenyl)-5-[(2-carboxy-ethyl)-aminocarbonyl]-6-dimethylamino-1-methyl-benzimidazol
(58) 2-(4-Amidino-phenyl)-1-n-butyl-5-[(2-carboxy-ethyl)-aminocarbonyl]-benzimidazol
(59) 2-(4-Amidino-phenyl)-5-[(2-carboxy-ethyl)-aminocarbonyl]-1-n-decyl-benzimidazol
(60) 2-(4-Amidino-phenyl)-5-[(2-carboxy-ethyl)-aminocarbonyl]-1-cyclopropyl-benzimidazol

(61) 2-(4-Amidino-phenyl)-5-[(2-carboxy-ethyl)-aminocarbonyl]-1-cyclohexyl-benzimidazol
(62) 2-(4-Amidino-phenyl)-1-benzyl-5-[(2-carboxy-ethyl)-aminocarbonyl]-benzimidazol-hydrochlorid $R_f$-Wert: 0,25 (Kieselgel; Methylenchlorid/Methanol = 8:2)
(63) 2-(4-Amidino-phenyl)-5-[(2-carboxy-ethyl)-aminocarbonyl]-1-(3-phenyl-propyl)-benzimidazol
(64) 2-(4-Amidino-phenyl)-5-[(2-carboxy-ethyl)-aminocarbonyl]-1-[2-(3,4-dimethoxy-phenyl)-ethyl]-benzimidazol
(65) 2-(4-Amidino-phenyl)-5-[(2-carboxy-ethyl)-aminocarbonyl]-1-[2-(2-pyridyl)-ethyl]-benzimidazol
(66) 2-(4-Amidino-phenyl)-5-[(2-carboxy-ethyl)-aminocarbonyl]-1-(3-pyridyl-methyl)-benzimidazol
(67) 2-(4-Amidino-phenyl)-5-[(2-carboxy-ethyl)-aminocarbonyl]-1-(4-pyridyl-methyl)-benzimidazol
(68) 2-(4-Amidino-phenyl)-5-[(2-carboxy-ethyl)-aminocarbonyl]-1-[3-(1-imidazolyl)-propyl]-benzimidazol
(69) 2-(4-Amidino-phenyl)-5-[(2-carboxy-ethyl)-aminocarbonyl]-1-[2-(4-imidazolyl)-ethyl]-benzimidazol
(70) 2-(4-Amidino-phenyl)-5-[(2-carboxy-ethyl)-aminocarbonyl]-1-(2-piperazino-ethyl)-benzimidazol
(71) 2-(4-Amidino-phenyl)-5-[(2-carboxy-ethyl)-aminocarbonyl]-1-(2-piperidino-ethyl)-benzimidazol
(72) 2-(4-Amidino-phenyl)-5-[(2-carboxy-ethyl)-aminocarbonyl]-1-(3-morpholino-propyl)-benzimidazol
(73) 2-(4-Amidino-phenyl)-5-[(2-carboxy-ethyl)-aminocarbonyl]-1-(3-thiomorpholino-propyl)-benzimidazol
Man verwendet Lithiumhydroxid in Tetrahydrofuran/Wasser
$R_f$-Wert: 0,06 (Kieselgel; Methylenchlorid/Methanol/konz. Ammoniak = 2:1:0,1 nach zweifacher Entwicklung)
(74) 2-(4-Amidino-phenyl)-5-[(2-carboxy-ethyl)-aminocarbonyl]-1-[3-(S-oxido-thiomorpholino)-propyl]-benzimidazol
Man verwendet Lithiumhydroxid in Tetrahydrofuran/Wasser
$R_f$-Wert: 0,28 (Kieselgel; n-Butanol/Eisessig/Wasser = 4:1:2 nach dreifacher Entwicklung)
(75) 2-(4-Amidino-phenyl)-5-[(2-carboxy-ethyl)-aminocarbonyl]-1-[3-(S,S-dioxido-thiomorpholino)-propyl]-benzimidazol
Man verwendet Lithiumhydroxid in Tetrahydrofuran/Wasser
$R_f$-Wert: 0,14 (Kieselgel; Isopropanol/Wasser/konz. Ammoniak = 7:2:1 nach zweimaliger Entwicklung)
(76) 2-(4-Amidino-phenyl)-1-(3-amino-propyl)-5-[(2-carboxyethyl)-aminocarbonyl]-benzimidazol-diacetat
Man verwendet Lithiumhydroxid in Tetrahydrofuran/Wasser
Schmelzpunkt: ab 110 °C (Zers.)
$R_f$-Wert: 0,13 (Kieselgel; Isopropanol/Wasser/konz. Ammoniak = 7:2:1)
(77) 2-(4-Amidino-phenyl)-5-[(2-carboxy-ethyl)-aminocarbonyl]-1-(2-hydroxy-ethyl)-benzimidazol
(78) 2-(4-Amidino-phenyl)-5-[(2-carboxy-ethyl)-aminocarbonyl]-1-(2-methoxy-ethyl)-benzimidazol
(79) 2-(4-Amidino-phenyl)-5-[(2-carboxy-ethyl)-aminocarbonyl]-1-methyl-benzimidazol-3-oxid
Schmelzpunkt: 263-265 °C (Zers.)
$R_f$-Wert: 0,79 (Reversed Phase Platte RP18; Methanol/5%ige Natriumchloridlösung = 6:4)
(80) 1-Allyl-2-(4-amidino-phenyl)-5-[(2-carboxy-ethyl)-aminocarbonyl]-benzimidazol
(81) 2-(4-Amidino-phenyl)-5-[(2-carboxy-ethyl)-aminocarbonyl]-1-phenyl-benzimidazol
(82) 2-(4-Amidino-phenyl)-5-[(2-carboxy-ethyl)-aminocarbonyl]-1-(4-chlor-phenyl)-benzimidazol
(83) 2-(4-Amidino-phenyl)-5-[(2-carboxy-ethyl)-aminocarbonyl]-1-(4-methoxy-phenyl)-benzimidazol
(84) 2-(4-Amidino-phenyl)-5-[(2-carboxy-ethyl)-aminocarbonyl]-1-(3-methyl-phenyl)-benzimidazol
(85) 2-(4-Amidino-phenyl)-5-[(2-carboxy-ethyl)-aminocarbonyl]-1-(3-pyridyl)-benzimidazol
(86) 2-(4-Amidino-phenyl)-5-[(2-carboxy-ethyl)-aminocarbonyl]-1-(3-methylsulfenyl-phenyl)-benzimidazol
(87) 2-(4-Amidino-phenyl)-5-[(2-carboxy-ethyl)-aminocarbonyl]-1-(3-methylsulfinyl-phenyl)-benzimidazol
(88) 2-(4-Amidino-phenyl)-5-[(2-carboxy-ethyl)-aminocarbonyl]-1-(3-methylsulfonyl-phenyl)-benzimidazol
(89) 2-(4-Amidino-phenyl)-5-[(2-carboxy-ethyl)-aminocarbonyl]-1-carboxymethyl-benzimidazol
(90) 2-(4-Amidino-phenyl)-1-(2-carboxy-ethyl)-5-[(2-carboxyethyl)-aminocarbonyl]-benzimidazol
(91) 2-(4-Amidino-phenyl)-1-(2-aminocarbonyl-ethyl)-5-[(2-carboxy-ethyl)-aminocarbonyl]-benzimidazol
(92) 2-(4-Amidino-phenyl)-5-[(2-carboxy-ethyl)-aminocarbonyl]-1-(2-dimethylaminocarbonyl-ethyl)-benzimidazol
(93) 2-(4-Amidino-phenyl)-5-[(2-carboxy-ethyl)-aminocarbonyl]-1-(2-morpholinocarbonyl-ethyl)-benzimidazol
(94) 2-(4-Amidino-phenyl)-5-[(2-carboxy-ethyl)-aminocarbonyl]-1-(2-thiomorpholinocarbonyl-ethyl)-benzimidazol
(95) 2-(4-Amidino-phenyl)-5-[(2-carboxy-ethyl)-aminocarbonyl]-1-[2-(S-oxido-thiomorpholinocarbonyl)-ethyl]-benzimidazol
(96) 2-(4-Amidino-phenyl)-5-[(2-carboxy-ethyl)-aminocarbonyl]-1-[2-(S,S-dioxido-thiomorpholinocarbonyl)-ethyl]-benzimidazol

(97) 2-(4-Amidino-phenyl)-5-[(2-carboxy-ethyl)-aminocarbonyl]-1-[2-(carboxymethyl-aminocarbonyl)-ethyl]-benzimidazol
(98) 2-(4-Aminomethyl-cyclohexyl)-5-[(2-carboxy-ethyl)-aminocarbonyl]-1-methyl-benzimidazol
(99) 2-(1-Amidino-4-piperidinyl)-5-[(2-carboxy-ethyl)-aminocarbonyl]-1-methyl-benzimidazol
(100) 2-(5-Amidino-2-pyridyl)-5-[(2-carboxy-ethyl)-aminocarbonyl]-1-methyl-benzimidazol
(101) 2-(4-Amidino-2-fluor-phenyl)-5-[(2-carboxy-ethyl)-aminocarbonyl]-1-methyl-benzimidazol
(102) 2-(4-Amidino-2-chlor-phenyl)-5(6)-[(2-carboxy-ethyl)-aminocarbonyl]-benzimidazol
(103) 2-(4-Amidino-2-methoxy-phenyl)-5-[(2-carboxy-ethyl)-aminocarbonyl]-1-methyl-benzimidazol
(104) 2-(4-Amidino-2-methyl-phenyl)-5-[(2-carboxy-ethyl)-aminocarbonyl]-1-methyl-benzimidazol
(105) 2-(4-Amidino-phenyl)-5-(4-carboxy-1-piperidinyl)-3-methyl-imidazo[4,5-b]pyridin
(106) 2-(4-Amidino-phenyl)-5-(4-carboxymethyl-1-piperidinyl)-3-methyl-imidazo[4,5-b]pyridin
(107) 2-(4-Amidino-phenyl)-5-(4-carboxy-cyclohexylmethylamino)-3-methyl-imidazo[4,5-b]pyridin
(108) 2-(4-Amidino-phenyl)-5-[(2-carboxy-ethyl)-aminocarbonyl]-benzoxazol
(109) 2-(4-Amidino-phenyl)-6-[(2-carboxy-ethyl)-aminocarbonyl]-benzthiazol
(110) 2-(4-Amidino-phenyl)-5-[(2-carboxy-ethyl)-aminocarbonyl]-oxazolo[5,4-b]pyridin
(111) 2-(4-Amidino-phenyl)-5-(N-carboxymethyl-methylaminocarbonylmethyl)-5H-imidazo[4,5-c]pyridin-4-on
(112) 8-(4-Amidino-phenyl)-1-(N-carboxymethyl-methylaminocarbonylmethyl)-hypoxanthin
(113) 8-(4-Amidino-phenyl)-1-(N-carboxymethyl-methylaminocarbonylmethyl)-xanthin
(114) 2-(4-Amidino-phenyl)-6-[(2-carboxy-ethyl)-aminocarbonyl]-3-methyl-imidazo[1,2-a]pyridin
(115) 2-(4-Amidino-phenyl)-5-[(2-carboxy-ethyl)-aminocarbonyl]-1-(2-pyrrolidino-ethyl)-benzimidazol
(116) 2-(4-Amidino-phenyl)-5-[(2-carboxy-ethyl)-aminocarbonyl]-1-(3-methylsulfenyl-propyl)-benzimidazol
(117) 2-(4-Amidino-phenyl)-5-[(2-carboxy-ethyl)-aminocarbonyl]-1-(3-methylsulfinyl-propyl)-benzimidazol
(118) 2-(4-Amidino-phenyl)-5-[(2-carboxy-ethyl)-aminocarbonyl]-1-(3-methylsulfonyl-propyl)-benzimidazol
(119) 2-(4-Amidino-1-piperazinyl)-5-[(2-carboxy-ethyl)-aminocarbonyl]-1-methyl-benzimidazol
(120) 2-(4-Amidino-phenyl)-5-[(2-carboxy-ethyl)-aminocarbonyl]-1-propargyl-benzimidazol
(121) 2-(4-Amidino-phenyl)-6-[N-(3-carboxy-propyl)-methansulfonylamino]-3-methyl-imidazo[4,5-b]pyridin
(122) 2-(4-Amidino-phenyl)-5-[(2-carboxy-ethyl)-aminosulfonyl]-1-methyl-benzimidazol
(123) 2-(4-Amidino-phenyl)-5-(carboxymethyl-aminosulfonyl)-1-methyl-benzimidazol
(124) 2-(4-Amidino-phenyl)-5-[N-[(4-carboxy-cyclohexyl)-methyl]-N-methyl-amino]-3-methyl-imidazo[4,5-b]pyridin
(125) 2-(4-Amidino-phenyl)-5-(4-carboxymethyl-1-piperazinyl)-3-methyl-imidazo[4,5-b]pyridin
(126) 2-(4-Amidino-phenyl)-5-(4-carboxymethyl-3-oxo-1-piperazinyl)-3-methyl-imidazo[4,5-b]pyridin
(127) 5-[(2-Carboxy-ethyl)-aminocarbonyl]-1-methyl-2-(4-methylamidino-phenyl)-benzimidazol
(128) 2-(n-Butylamidino-phenyl)-5-[(2-carboxy-ethyl)-aminocarbonyl]-1-methyl-benzimidazol
(129) 5-[(2-Carboxy-ethyl)-aminocarbonyl]-1-methyl-2-(4-methylaminomethyl-phenyl)-benzimidazol
(130) 2-(4-Amidino-phenyl)-5-[(4-carboxy-cyclohexyl)-amino]-3-methyl-imidazo[4,5-b]pyridin
(131) 2-(4-Amidino-phenyl)-5-[N-(2-carboxy-ethyl)-methylaminosulfonyl]-1-methyl-benzimidazol
(132) 2-(4-Amino-cyclohexyl)-5-[(2-carboxy-ethyl)-aminocarbonyl]-1-methyl-benzimidazol
(133) 8-(4-Amidino-phenyl)-1-(N-carboxymethyl-methylaminocarbonylmethyl)-3-methyl-xanthin
(134) 2-(4-Amidino-phenyl)-1-(6-amino-hexyl)-5-[(2-carboxyethyl)-aminocarbonyl]-benzimidazol
(135) 2-[(4-Amidino-phenyl)-oxymethyl]-5(6)-carboxymethoxybenzimidazol
$R_f$-Wert: 0,07 (Kieselgel; n-Butanol/Eisessig/Wasser = 4:1:1, zweifach entwickelt)

| | | | | | | |
|---|---|---|---|---|---|---|
| Ber.: | C | 60,00 | H | 4,74 | N | 16,46 |
| Gef.: | | 59,60 | | 4,82 | | 16,52 |

(136) 2-(4-Amidino-phenyl)-1-[2-(4-benzyl-piperazino)-ethyl]-5-[(2-carboxy-ethyl)-aminocarbonyl]-benzimidazol
Man verwendet Lithiumhydroxid in Tetrahydrofuran/Wasser
Schmelzpunkt: 82 ° C (Zers.)
$R_f$-Wert: 0,51 (Kieselgel; Isopropanol/Wasser/konz. Ammoniak = 7:2:1)
(137) 2-(4-Aminomethyl-phenyl)-5-[(2-carboxy-ethyl)-aminocarbonyl]-1-(3-thiomorpholino-propyl)-benzimidazol
Man verwendet Lithiumhydroxid in Tetrahydrofuran/Wasser
$R_f$-Wert: 0,50 (Reversed Phase Platte RP18; Methanol/5%ige Natriumchloridlösung = 6:10)

(138) 2-(4-Amidino-phenyl)-5-[(2-carboxy-ethyl)-aminocarbonyl]-1-(3-carboxy-propyl)-benzimidazol
$R_f$-Wert: 0,10 (Kieselgel; Methylenchlorid/Methanol = 8:2)
(139) 2-(4-Amidino-phenyl)-5-[(2-carboxy-ethyl)-aminocarbonyl]-1-methyl-6-methylamino-benzimidazol
Man verwendet Lithiumhydroxid.
Schmelzpunkt: 265-266 ° C (Zers.)
$R_f$-Wert: 0,35 (Kieselgel; n-Butanol/Eisessig/Wasser = 4:1:2)
(140) 2-(4-Amidino-phenyl)-5-[(2-carboxy-ethyl)-aminocarbonyl]-6-(N-carboxymethyl-methylamino)-1-methyl-benzimidazol
Man verwendet Lithiumhydroxid.
$R_f$-Wert: 0,62 (Reversed Phase Platte RP18; Methanol/5%ige Natriumchloridlösung 6:4, nach zweimaliger Entwicklung)
(141) 2-(4-Amidino-phenyl)-5-[(2-carboxy-ethyl)-aminocarbonyl]-1-(3,3-diphenyl-propyl)-benzimidazol
(142) 2-[4-(2-Amino-2-propyl)-phenyl]-5-[(2-carboxy-ethyl)-aminocarbonyl]-1-methyl-benzimidazol
(143) 2-(1-Amino-5-indanyl)-5-[(2-carboxy-ethyl)-aminocarbonyl]-1-methyl-benzimidazol
(144) 2-(1-Amino-1,2,3,4-tetrahydro-6-naphthyl)-5-[(2-carboxy-ethyl)-aminocarbonyl]-1-methyl-benzimidazol
(145) 2-[4-(1-Amino-cyclopropyl)-phenyl]-5-[(2-carboxyethyl)-aminocarbonyl]-1-methyl-benzimidazol
(146) 2-[4-(1-Amino-cyclopentyl)-phenyl)-5-[(2-carboxyethyl)-aminocarbonyl]-1-methyl-benzimidazol
(147) 2-(4-Amidino-phenyl)-5-[(2-carboxy-2-methyl-propyl)-aminocarbonyl]-1-methyl-benzimidazol
(148) 2-(4-Amidino-phenyl)-5-[(2-carboxy-propyl)-aminocarbonyl]-1-methyl-benzimidazol
(149) 2-(4-Amidino-phenyl)-5-[(2-carboxy-4-phenyl-butyl)-aminocarbonyl]-1-methyl-benzimidazol
(150) 8-(4-Amidino-phenyl)-2-[(2-carboxy-ethyl)-aminocarbonyl]-7-methyl-purin
(151) 8-(4-Amidino-phenyl)-2-[(2-carboxy-ethyl)-aminocarbonyl]-6-methoxy-7-methyl-purin
(152) 8-(4-Amidino-phenyl)-2-[(2-carboxy-ethyl)-aminocarbonyl]-6-dimethylamino-7-methyl-purin
(153) 8-(4-Amidino-phenyl)-6-amino-2-[(2-carboxy-ethyl)-aminocarbonyl]-7-methyl-purin
(154) 8-(4-Amidino-phenyl)-2-[(2-carboxy-ethyl)-aminocarbonyl]-7-methyl-6-piperidino-purin
(155) 8-(4-Amidino-phenyl)-2-[(2-carboxy-ethyl)-aminocarbonyl]-7-methyl-hypoxanthin
(156) 2-(4-Amidino-phenyl)-7-[(2-carboxy-ethyl)-aminocarbonyl]-imidazo[1,2-a]pyridin
(157) 2-(4-Amidino-phenyl)-7-[(2-carboxy-ethyl)-aminocarbonyl]-imidazo[1,2-a]pyrimidin
(158) 2-(4-Amidino-phenyl)-7-[(2-carboxy-ethyl)-aminocarbonyl]-imidazo[1,2-c]pyrimidin
(159) 2-(4-Amidino-phenyl)-5-[(2-carboxy-ethyl)-aminocarbonyl]-benzthiazol
(160) 8-(4-Amidino-phenyl)-1-(4-carboxy-butyl)-3,9-dimethylxanthin
Schmelzpunkt: 263-265 ° C (Zers.)
$R_f$-Wert: 0,48 (Kieselgel; n-Butanol/Eisessig/Wasser = 4:1:1,5)
(161) 2-(4-Amidino-phenyl)-7-[(2-carboxy-ethyl)-aminocarbonyl]-3-methyl-imidazol[1,2-a]pyridin
(162) 2-(4-Amidino-phenyl)-5-[(2-carboxy-ethyl)-aminocarbonyl]-1-methyl-6-(3-phenyl-propyloxy)-benzimidazol
(163) 2-(4-Amidino-phenyl)-5-[(2-carboxy-ethyl)-aminocarbonyl]-1-(4-methyl-benzyl)-benzimidazol
(164) 2-(4-Amidino-phenyl)-5-[(2-carboxy-ethyl)-aminocarbonyl]-1-[2-(3,5-dichlor-4-hydroxy-phenyl)-ethyl]-benzimidazol
(165) 2-(4-Amidino-phenyl)-5-[(2-carboxy-ethyl)-aminocarbonyl]-1-[(2-phenyl-ethyl)-aminocarbonylmethyl]-benzimidazol

Beispiel 2

2-(4-Amidino-phenyl)-5-[(3-methoxycarbonyl-propyl)-aminocarbonyl]-1-methyl-benzimidazol-hydrochlorid

4,2 g 4-[4-[N-(4-Amidino-benzoyl)-methylamino]-3-nitro-benzoylamino]-buttersäure-methylester werden in 100 ml Methanol gelöst, mit 10 ml etherischer Salzsäure und 0,5 g 10%iger Palladiumkohle versetzt und bei Raumtemperatur mit Wasserstoff von 5 bar Druck 22 Stunden behandelt. Man setzt weitere 0,3 g des Katalysators zu und läßt eine weitere Stunde reagieren. Der Katalysator wird abfiltriert, das Filtrat eingedampft und der Rückstand mit einer Mischung aus 100 ml Essigester und 10 ml Methanol eine Stunde bei Raumtemperatur verrührt, wobei die Substanz in kristalliner Form anfällt.
Ausbeute: 3,7 g (100 % der Theorie),
Schmelzpunkt: über 200 ° C
$R_f$-Wert: 0,65 (Reversed-Phase-Platte RP18; Methanol/5%ige Natriumchloridlösung = 6:4)
Analog werden folgende Verbindungen erhalten:

(1) 2-(4-Amidino-phenyl)-5-[(2-methoxycarbonyl-ethyl)-aminocarbonyl]-1-methyl-benzimidazol
$R_f$-Wert: 0,59 (Reversed-Phase-Platte RP18; Methanol/5%ige Natriumchloridlösung = 6:4)
(2) 2-(4-Amidino-phenyl)-5-[(2-ethoxycarbonyl-ethyl)-aminocarbonyl]-1-methyl-benzimidazol
(3) 2-(4-Amidino-phenyl)-5-[N-(2-methoxycarbonyl-ethyl)-N-methyl-aminocarbonyl]-1-methyl-benzimidazol
(4) 2-(4-Amidino-phenyl)-5-[(2-methoxycarbonyl-ethyl)-aminocarbonyl]-1-methyl-benzimidazol-3-oxid
Man arbeitet ohne Zusatz von Salzsäure
$R_f$-Wert: 0;63 (Reversed-Phase-Platte RP18; Methanol/5%ige Natriumchloridlösung = 6:4)

Beispiel 3

2-[(4-Amidino-phenyl)-oxymethyl]-5(6)-methoxycarbonylmethoxy-benzimidazol-hydrochlorid

2,4 g 2-[(4-Cyan-phenyl)-oxymethyl]-5(6)-methoxycarbonylmethoxy-benzimidazol werden in 300 ml Methanol suspendiert. In die Mischung leitet man eine Stunde lang bei 0-10°C Salzsäuregas ein und rührt 4 Stunden bei 15-20°C nach. Das Methanol wird im Vakuum abgedampft, der Rückstand mit 75 ml Methanol versetzt und dieses wiederum im Vakuum abdestilliert. Der Rückstand wird in 300 ml Methanol suspendiert, wonach man unter Rühren portionsweise 16,3 g Ammoniumcarbonat zufügt und weitere 16 Stunden nachrührt. Die Reaktionsmischung wird mit einer Mischung aus 3 Teilen Methanol und einem Teil konzentrierter Salzsäure auf pH4 gebracht. Man dampft zur Trockene ein und reinigt den Rückstand über Kieselgel (Elutionsmittel: Methylenchlorid/Methanol = 3:1 bis 0:1).
Ausbeute: 0,9 g (32 % der Theorie),
Schmelzpunkt: 250°C (Zers.)
$R_f$-Wert: 0,64 (Kieselgel; Methylenchlorid/Methanol/Eisessig = 3:1:0,1)

| | | | | | | | | |
|---|---|---|---|---|---|---|---|---|
| Ber. x $H_2O$ x HCl: | C | 52,87 | H | 5,18 | N | 13,70 | Cl | 8,67 |
| Gef.: | | 53,07 | | 5,02 | | 13,81 | | 8,80 |

Analog werden folgende Verbindungen erhalten:
(1) 2-(4-Amidino-phenyl)-5(6)-[(2-methoxycarbonyl-ethyl)-carbonylamino]-benzimidazol
Schmelzpunkt über 200°C
$R_f$-Wert: 0,11 (Kieselgel; Essigester/Ethanol = 7:3)
(2) 2-(4-Amidino-phenyl)-5(6)-[(2-methoxycarbonyl-ethyl)-aminocarbonyl]-benzimidazol
(3) 2-(4-Amidino-phenyl)-5-[(2-methoxycarbonyl-ethyl)-aminocarbonyl]-1-methyl-benzimidazol
(4) 2-(4-Amidino-phenyl)-5-[(2-ethoxycarbonyl-ethyl)-aminocarbonyl]-1-methyl-benzimidazol Man verwendet ethanolische Salzsäure.
(5) 2-(4-Amidino-phenyl)-5(6)-[(3-methoxycarbonyl-propyl)-carbonylamino]-benzimidazol
(6) 2-(4-Amidino-phenyl)-6-[(2-methoxycarbonyl-ethyl)-aminocarbonyl]-1-methyl-benzimidazol
(7) 2-(4-Amidino-phenyl)-5-[(3-methoxycarbonyl-propyl)-carbonyl]-1-methyl-benzimidazol
(8) 2-(4-Amidino-phenyl)-5-[(4-methoxycarbonyl-butyl)-carbonyl]-1-methyl-benzimidazol
(9) 2-(4-Amidino-phenyl)-5(6)-(2-methoxycarbonyl-ethylthio)-benzimidazol
(10) 2-(4-Amidino-phenyl)-5(6)-(3-methoxycarbonyl-propylthio)-benzimidazol
(11) 2-(4-Amidino-phenyl)-5(6)-[(3-methoxycarbonyl-propyl)-amino]-benzimidazol
(12) 5(6)-[N-Acetyl-N-(3-methoxycarbonyl-propyl)-amino]-2-(4-amidino-phenyl)-benzimidazol
(13) 2-(4-Amidino-phenyl)-5(6)-[(3-methoxycarbonyl-propyl)-oxy]-benzimidazol
(14) 2-(4-Amidino-phenyl)-5(6)-(4-methoxycarbonyl-butyl)-benzimidazol
(15) 2-(4-Amidino-phenyl)-5(6)-[(2-methoxycarbonyl-ethyl)-aminomethyl]-benzimidazol
(16) 2-(4-Amidino-phenyl)-5(6)-[(2-methoxycarbonyl-ethyl)-thiomethyl]-benzimidazol
(17) 2-(4-Amidino-phenyl)-5(6)-(methoxycarbonylmethylcarbonyl-aminomethyl)-benzimidazol
(18) 2-(4-Amidino-phenyl)-5(6)-(4-methoxycarbonyl-1-butenl-yl)-benzimidazol
(19) 2-(4-Amidino-phenyl)-6-[(2-methoxycarbonyl-ethyl)-aminocarbonyl]-3-methyl-imidazo[4,5-b]pyridin
$R_f$-Wert: 0,49 (Reversed Phase Platte RP18; Methanol/5%ige Natriumchloridlösung = 6:4)
(20) 2-(4-Amidino-phenyl)-6-[(2-methoxycarbonyl-ethyl)-aminocarbonyl]-imidazo[4,5-b]pyridin
(21) 2-(4-Amidino-phenyl)-6-[(2-methoxycarbonyl-ethyl)-aminocarbonyl]-imidazo[1,2-a]pyridin
(22) 2-(4-Amidino-phenyl)-6-[(2-methoxycarbonyl-ethyl)-aminocarbonyl]-3-methyl-imidazo[4,5-c]pyridin
(23) 2-(4-Amidino-phenyl)-6-[(2-methoxycarbonyl-ethyl)-aminocarbonyl]-imidazo[4,5-c]pyridazin
(24) 2-(4-Amidino-phenyl)-5-[(2-methoxycarbonyl-ethyl)-aminocarbonyl]-imidazo[4,5-b]pyrazin
(25) 8-(4-Amidino-phenyl)-2-[(2-methoxycarbonyl-ethyl)-aminocarbonyl]-9-methyl-purin

(26) 8-(4-Amidino-phenyl)-6-chlor-2-[(2-methoxycarbonylethyl)-aminocarbonyl]-9-methyl-purin
(27) 8-(4-Amidino-phenyl)-6-methoxy-2-[(2-methoxycarbonylethyl)-aminocarbonyl]-9-methyl-purin
(28) 8-(4-Amidino-phenyl)-6-dimethylamino-2-[(2-methoxycarbonyl-ethyl)-aminocarbonyl]-9-methyl-purin
(29) 8-(4-Amidino-phenyl)-6-amino-2-[(2-methoxycarbonylethyl)-aminocarbonyl]-9-methyl-purin
(30) 8-(4-Amidino-phenyl)-2-[(2-methoxycarbonyl-ethyl)-aminocarbonyl]-9-methyl-6-piperidino-purin
(31) 8-(4-Amidino-phenyl)-2-[(2-methoxycarbonyl-ethyl)-aminocarbonyl]-9-methyl-hypoxanthin
(32) 2-(4-Amidino-phenyl)-6-[(2-methoxycarbonyl-ethyl)-aminocarbonyl]-imidazo[1,2-a]pyrimidin
(33) 2-(4-Amidino-phenyl)-5-[N-(2-methoxycarbonyl-ethyl)-methylaminocarbonyl]-1-methyl-benzimidazol
(34) 2-(4-Amidino-phenyl)-7-fluor-5-[(2-methoxycarbonylethyl)-aminocarbonyl]-1-methyl-benzimidazol
(35) 2-(4-Amidino-phenyl)-4-chlor-6-[(2-methoxycarbonylethyl)-aminocarbonyl]-benzimidazol
(36) 2-(4-Amidino-phenyl)-4-brom-6-[(2-methoxycarbonylethyl)-aminocarbonyl]-benzimidazol
(37) 2-(4-Amidino-phenyl)-6-hydroxy-5-[(2-methoxycarbonylethyl)-aminocarbonyl]-1-methyl-benzimidazol
(38) 2-(4-Amidino-phenyl)-6-methoxy-5-[(2-methoxycarbonylethyl)-aminocarbonyl]-1-methyl-benzimidazol
(39) 2-(4-Amidino-phenyl)-5-[(2-methoxycarbonyl-ethyl)-aminocarbonyl]-1-methyl-6-propyloxy-benzimidazol
(40) 2-(4-Amidino-phenyl)-6-[(2-methoxycarbonyl-ethyl)-aminocarbonyl]-4-methyl-benzimidazol
(41) 2-(4-Amidino-phenyl)-5-[(2-methoxycarbonyl-ethyl)-aminocarbonyl]-1-methyl-6-methansulfonylamino-benzimidazol
(42) 6-Acetylamino-2-(4-amidino-phenyl)-5-[(2-methoxycarbonyl-ethyl)-aminocarbonyl]-1-methyl-benzimidazol
(43) 2-(4-Amidino-phenyl)-6-dimethylamino-5-[(2-methoxycarbonyl-ethyl)-aminocarbonyl]-1-methyl-benzimidazol
(44) 2-(4-Amidino-phenyl)-1-n-butyl-5-[(2-methoxycarbonylethyl)-aminocarbonyl]-benzimidazol
(45) 2-(4-Amidino-phenyl)-1-n-decyl-5-[(2-methoxycarbonylethyl)-aminocarbonyl]-benzimidazol
(46) 2-(4-Amidino-phenyl)-1-cyclopropyl-5-[(2-methoxycarbonyl-ethyl)-aminocarbonyl]-benzimidazol
(47) 2-(4-Amidino-phenyl)-1-cyclohexyl-5-[(2-methoxycarbonyl-ethyl)-aminocarbonyl]-benzimidazol
(48) 2-(4-Amidino-phenyl)-1-benzyl-5-[(2-methoxycarbonylethyl)-aminocarbonyl]-benzimidazol-hydrochlorid
$R_f$-Wert: 0,22 (Kieselgel; Methylenchlorid/Methanol = 7:3)
(49) 2-(4-Amidino-phenyl)-5-[(2-methoxycarbonyl-ethyl)-aminocarbonyl]-1-(3-phenyl-propyl)-benzimidazol
(50) 2-(4-Amidino-phenyl)-1-[2-(3,4-dimethoxy-phenyl)-ethyl]-5-[(2-methoxycarbonyl-ethyl)-aminocarbonyl]-benzimidazolhydrochlorid
$R_f$-Wert: 0,20 (Kieselgel; Essigester/Ethanol = 7:3)
(51) 2-(4-Amidino-phenyl)-5-[(2-methoxycarbonyl-ethyl)-aminocarbonyl]-1-[2-(2-pyridyl)-ethyl]-benzimidazol
(52) 2-(4-Amidino-phenyl)-5-[(2-methoxycarbonyl-ethyl)-aminocarbonyl]-1-(3-pyridylmethyl)-benzimidazol
$R_f$-Wert: 0,60 (Reversed Phase Platte RP18; Methanol/5%ige Natriumchloridlösung = 6:4)
(53) 2-(4-Amidino-phenyl)-5-[(2-methoxycarbonyl-ethyl)-aminocarbonyl]-1-(4-pyridylmethyl)-benzimidazol
(54) 2-(4-Amidino-phenyl)-1-[3-(1-imidazolyl)-propyl]-5-[(2-methoxycarbonyl-ethyl)-aminocarbonyl]-benzimidazol
(55) 2-(4-Amidino-phenyl)-1-[2-(4-imidazolyl)-ethyl]-5-[(2-methoxycarbonyl-ethyl)-aminocarbonyl]-benzimidazol
(56) 2-(4-Amidino-phenyl)-5-[(2-methoxycarbonyl-ethyl)-aminocarbonyl]-1-(2-piperazino-ethyl]-benzimidazol
(57) 2-(4-Amidino-phenyl)-5-[(2-methoxycarbonyl-ethyl)-aminocarbonyl]-1-(2-piperidino-ethyl)-benzimidazol
(58) 2-(4-Amidino-phenyl)-5-[(2-methoxycarbonyl-ethyl)-aminocarbonyl]-1-(3-morpholino-propyl)-benzimidazol
(59) 2-(4-Amidino-phenyl)-5-[(2-methoxycarbonyl-ethyl)-aminocarbonyl]-1-(3-thiomorpholino-propyl)-benzimidazol
$R_f$-Wert: 0,18 (Kieselgel; Methylenchlorid/Methanol/konz. Ammoniak = 8:2:0,1)
(60) 2-(4-Amidino-phenyl)-1-(3-amino-propyl)-5-[(2-methoxycarbonyl-ethyl)-aminocarbonyl]-benzimidazol
$R_f$-Wert: 0,29 (Kieselgel; n-Butanol/Eisessig/Wasser = 4:1:1, nach dreifacher Entwicklung)
(61) 2-(4-Amidino-phenyl)-1-(2-hydroxy-ethyl)-5-[(2-methoxycarbonyl-ethyl)-aminocarbonyl]-benzimidazol
(62) 2-(4-Amidino-phenyl)-5-[(2-methoxycarbonyl-ethyl)-aminocarbonyl]-1-(2-methoxy-ethyl)-benzimidazol
(63) 1-Allyl-2-(4-amidino-phenyl)-5-[(2-methoxycarbonylethyl)-aminocarbonyl]-benzimidazol
(64) 2-(4-Amidino-phenyl)-5-[(2-methoxycarbonyl-ethyl)-aminocarbonyl]-1-phenyl-benzimidazol
(65) 2-(4-Amidino-phenyl)-1-(4-chlor-phenyl)-5-[(2-methoxycarbonyl-ethyl)-aminocarbonyl]-benzimidazol

(66) 2-(4-Amidino-phenyl)-5-[(2-methoxycarbonyl-ethyl)-aminocarbonyl]-1-(4-methoxy-phenyl)-benzimidazol

(67) 2-(4-Amidino-phenyl)-5-[(2-methoxycarbonyl-ethyl)-aminocarbonyl]-1-(3-methyl-phenyl)-benzimidazol

(68) 2-(4-Amidino-phenyl)-5-[(2-methoxycarbonyl-ethyl)-aminocarbonyl]-1-(3-pyridyl)-benzimidazol

(69) 2-(4-Amidino-phenyl)-5-[(2-methoxycarbonyl-ethyl)-aminocarbonyl]-1-(3-methylsulfenyl-phenyl)-benzimidazol

(70) 2-(4-Amidino-phenyl)-5-[(2-methoxycarbonyl-ethyl)-aminocarbonyl]-1-methoxycarbonylmethyl-benzimidazol

(71) 2-(4-Amidino-phenyl)-1-(2-methoxycarbonyl-ethyl)-5-[(2-methoxycarbonyl-ethyl)-aminocarbonyl]-benzimidazol

(72) 2-(4-Amidino-phenyl)-1-(2-aminocarbonyl-ethyl)-5-[(2-methoxycarbonyl-ethyl)-aminocarbonyl]-benzimidazol

(73) 2-(4-Amidino-phenyl)-1-(2-dimethylaminocarbonyl-ethyl)-5-[(2-methoxycarbonyl-ethyl)-aminocarbonyl]-benzimidazol

(74) 2-(4-Amidino-phenyl)-5-[(2-methoxycarbonyl-ethyl)-aminocarbonyl]-1-(2-morpholinocarbonyl-ethyl)-benzimidazol

(75) 2-(4-Amidino-phenyl)-5-[(2-methoxycarbonyl-ethyl)-aminocarbonyl]-1-(2-thiomorpholinocarbonyl-ethyl)-benzimidazol

(76) 2-(4-Amidino-phenyl)-5-[(2-methoxycarbonyl-ethyl)-aminocarbonyl]-1-[2-(methoxycarbonylmethyl-aminocarbonyl)-ethyl]-benzimidazol

(77) 2-(5-Amidino-2-pyridyl)-5-[(2-methoxycarbonyl-ethyl)-aminocarbonyl]-1-methyl-benzimidazol

(78) 2-(4-Amidino-2-fluor-phenyl)-5-[(2-methoxycarbonylethyl)-aminocarbonyl]-1-methyl-benzimidazol

(79) 2-(4-Amidino-2-chlor-phenyl)-5(6)-[(2-methoxycarbonylethyl)-aminocarbonyl]-benzimidazol

(80) 2-(4-Amidino-2-methoxy-phenyl)-5-[(2-methoxycarbonylethyl)-aminocarbonyl]-1-methyl-benzimidazol

(81) 2-(4-Amidino-2-methyl-phenyl)-5-[(2-methoxycarbonylethyl)-aminocarbonyl]-1-methyl-benzimidazol

(82) 2-(4-Amidino-phenyl)-5-(2-methoxycarbonyl-1-piperidinyl)-3-methyl-imidazo[4,5-b]pyridin

(83) 2-(4-Amidino-phenyl)-5-(4-methoxycarbonylmethyl-1-piperidinyl)-3-methyl-imidazo[4,5-b]pyridin

(84) 2-(4-Amidino-phenyl)-5-(4-methoxycarbonyl-cyclohexylmethylamino)-3-methyl-imidazo[4,5-b]pyridin

(85) 2-(4-Amidino-phenyl)-5-[(4-methoxycarbonyl-ethyl)-aminocarbonyl]-benzoxazol

(86) 2-(4-Amidino-phenyl)-6-[(2-methoxycarbonyl-ethyl)-aminocarbonyl]-benzthiazol

(87) 2-(4-Amidino-phenyl)-5-[(2-methoxycarbonyl-ethyl)-aminocarbonyl]-oxazolo[5,4-b]pyridin

(88) 2-(4-Amidino-phenyl)-5-(N-methoxycarbonylmethyl-methylamino-carbonylmethyl)-5H-imidazo[4,5-c]-pyridin-4-on

(89) 8-(4-Amidino-phenyl)-1-(N-methoxycarbonylmethyl-methylamino-carbonylmethyl)-hypoxanthin

(90) 8-(4-Amidino-phenyl)-1-(N-methoxycarbonylmethyl-methylamino-carbonylmethyl)-xanthin

(91) 2-(4-Amidino-phenyl)-6-[(2-methoxycarbonyl-ethyl)-aminocarbonyl]-3-methyl-imidazo[1,2-a]pyridin

(92) 2-(4-Amidino-phenyl)-5-[(2-methoxycarbonyl-ethyl)-aminocarbonyl]-1-(2-pyrrolidino-ethyl]-benzimidazol

(93) 2-(4-Amidino-phenyl)-1-methyl-5-[[2-(5-tetrazolyl)-ethyl]-aminocarbonyl]-benzimidazol

(94) 2-(4-Amidino-phenyl)-1-methyl-5-[(2-phosphono-ethyl)-aminocarbonyl]-benzimidazol

(95) 2-(4-Amidino-phenyl)-1-methyl-5-[[2-(O-methyl-phosphono)-ethyl]-aminocarbonyl]-benzimidazol

(96) 2-(4-Amidino-phenyl)-1-methyl-5-[(2-sulfo-ethyl)-aminocarbonyl]-benzimidazol

(97) 2-(4-Amidino-phenyl)-5-[(2-methoxycarbonyl-ethyl)-aminocarbonyl]-1-(3-methylsulfenyl-propyl)-benzimidazol

(98) 2-(4-Amidino-phenyl)-5-[(2-methoxycarbonyl-ethyl)-aminocarbonyl]-1-propargyl-benzimidazol

(99) 2-(4-Amidino-phenyl)-6-[N-methansulfonyl-N-(3-methoxycarbonyl-propyl)-amino]-3-methyl-imidazol-[4,5-b]pyridin

(100) 2-(4-Amidino-phenyl)-5-[(2-methoxycarbonyl-ethyl)-aminosulfonyl]-1-methyl-benzimidazol

(101) 2-(4-Amidino-phenyl)-5-(methoxycarbonylmethylaminosulfonyl)-1-methyl-benzimidazol

(102) 2-(4-Amidino-phenyl)-5-[N-(4-methoxycarbonyl-cyclohexyl)-methyl-methylamino]-3-methyl-imidazo-[4,5-b]pyridin

(103) 2-(4-Amidino-phenyl)-5-(4-methoxycarbonylmethyl-1-piperazinyl)-3-methyl-imidazo[4,5-b]pyridin

(104) 2-(4-Amidino-phenyl)-5-(4-methoxycarbonylmethyl-3-oxo-1-piperazinyl)-3-methyl-imidazo[4,5-b]-pyridin

(105) 5-[(2-Methoxycarbonyl-ethyl)-aminocarbonyl]-1-methyl-2-(4-methylamidino-phenyl)-benzimidazol
Man verwendet konzentrierte wäßrige Methylaminlösung.

(106) 2-(n-Butylamidino-phenyl)-5-[(2-methoxycarbonyl-ethyl)-aminocarbonyl]-1-methyl-benzimidazol
Man verwendet n-Butylamin.

(107) 2-(4-Amidino-phenyl)-5-[(4-methoxycarbonyl-cyclohexyl)-amino]-3-methyl-imidazo[4,5-b]pyridin
(108) 2-(4-Amidino-phenyl)-5-[N-(2-methoxycarbonyl-ethyl)-methylaminosulfonyl]-1-methyl-benzimidazol
(109) 8-(4-Amidino-phenyl)-1-(N-methoxycarbonylmethyl-methylaminocarbonylmethyl)-3-methyl-xanthin
(110) 2-(4-Amidino-phenyl)-1-(6-amino-hexyl)-5-[(2-methoxycarbonyl-ethyl)-aminocarbonyl]-benzimidazol
(111) 2-(4-Amidino-phenyl)-5(6)-[(2-methoxycarbonyl-ethyl)-sulfonyl]-benzimidazol
(112) 2-(4-Amidino-phenyl)-5(6)-[(3-methoxycarbonyl-propyl)-sulfonyl]-benzimidazol
(113) 2-(4-Amidino-phenyl)-5(6)-[(2-methoxycarbonyl-ethyl)-sulfonylmethyl]-benzimidazol
(114) 2-(4-Amidino-phenyl)-1-[3-(S,S-dioxido-thiomorpholino)-propyl]-5-[(2-methoxycarbonyl-ethyl)-aminocarbonyl]-benzimidazol
(115) 2-(4-Amidino-phenyl)-5-[(2-methoxycarbonyl-ethyl)-aminocarbonyl]-1-(3-methylsulfonyl-phenyl)-benzimidazol
(116) 2-(4-Amidino-phenyl)-1-[2-(S,S-dioxido-thiomorpholinocarbonyl)-ethyl]-5-[(2-methoxycarbonyl-ethyl)-aminocarbonyl]-benzimidazol
(117) 2-(4-Amidino-phenyl)-5-[(2-methoxycarbonyl-ethyl)-aminocarbonyl]-1-(3-methylsulfonyl-propyl)-benzimidazol
(118) 2-(4-Amidino-phenyl)-1-[2-(4-benzyl-piperazino)-ethyl]-5-[(2-methoxycarbonyl-ethyl)-aminocarbonyl]-benzimidazol
$R_f$-Wert: 0,49 (Kieselgel; Methylenchlorid/Methanol = 8:2)
(119) 2-(4-Amidino-phenyl)-5-[(2-methoxycarbonyl-ethyl)-aminocarbonyl]-1-n-tetradecyl-benzimidazol
$R_f$-Wert: 0,04 (Kieselgel; Essigester/Ethanol = 8:2)
(120) 2-(4-Amidino-phenyl)-5-[(2-methoxycarbonyl-ethyl)-aminocarbonyl]-1-(3-methoxycarbonyl-propyl)-benzimidazolhydrochlorid
$R_f$-Wert: 0,45 (Kieselgel; Methylenchlorid/Methanol = 8:2)
(121) 2-(4-Amidino-phenyl)-1-(3-carboxy-propyl)-5-[(2-methoxycarbonyl-ethyl)-aminocarbonyl]-benzimidazol
Entsteht als Nebenprodukt bei (120)
$R_f$-Wert: 0,19 (Kieselgel; Methylenchlorid/Methanol = 8:2)
(122) 2-(4-Amidino-phenyl)-5-[(2-methoxycarbonyl-ethyl)-aminocarbonyl]-1-(4-phenyl-butyl)-benzimidazol
Schmelzpunkt: 184-186°C
$R_f$-Wert: 0,13 (Reversed Phase Platte RP18; Methanol/5%ige Natriumchloridlösung = 6:4)
(123) 2-(4-Amidino-phenyl)-5-[(2-methoxycarbonyl-ethyl)-aminocarbonyl]-1-methyl-6-methylamino-benzimidazol-hydrochloridhydrogenacetat
Schmelzpunkt: 260-268°C (Zers.)
$R_f$-Wert: 0,24 (Kieselgel; Methylenchlorid/Methanol/konz. Ammoniak = 8:2:0,1)
(124) 2-(4-Amidino-phenyl)-5-[(2-methoxycarbonyl-ethyl)-aminocarbonyl]-6-(N-methoxycarbonylmethyl-methylamino)-1-methyl-benzimidazol-hydrochlorid
$R_f$-Wert: 0,42 (Kieselgel; Methylenchlorid/Methanol/konz. Ammoniak = 5:2:0,2)
(125) 2-(4-Amidino-phenyl)-1-[2-(4-amino-3,5-dibrom-phenyl)-ethyl]-5-[(2-methoxycarbonyl-ethyl)-aminocarbonyl]-benzimidazol
Schmelzpunkt: 185°C (sintert ab 165°C)
$R_f$-Wert: 0,43 (Reversed Phase Platte RP18; Methanol/5%ige Natriumchloridlösung = 6:4)
(126) 2-(4-Amidino-phenyl)-1-(3,3-diphenyl-propyl)-5-[(2-methoxycarbonyl-ethyl)-aminocarbonyl]-benzimidazol
Schmelzpunkt: über 200°C,
$R_f$-Wert: 0,11 (Reversed Phase Platte RP18; Methanol/5%ige Natriumchloridlösung = 6:4)
(127) 2-(4-Amidino-phenyl)-5-[(2-methoxycarbonyl-2-methylpropyl)-aminocarbonyl]-1-methyl-benzimidazol
(128) 2-(4-Amidino-phenyl)-5-[(2-methoxycarbonyl-propyl)-aminocarbonyl]-1-methyl-benzimidazol
(129) 2-(4-Amidino-phenyl)-5-[(2-methoxycarbonyl-4-phenylbutyl)-aminocarbonyl]-1-methyl-benzimidazol
(130) 2-(4-Amidino-phenyl)-6-(N-isobutyloxycarbonyl-N-methyl-amino)-5-[(2-methoxycarbonyl-ethyl)-aminocarbonyl]-1-methyl-benzimidazol
Schmelzpunkt: 104-106°C (Zers.)
$R_f$-Wert: 0,76 (Kieselgel; Methylenchlorid/Methanol/konz. Ammoniak = 3:1:0,1)
(131) 8-(4-Amidino-phenyl)-2-[(2-methoxycarbonyl-ethyl)-aminocarbonyl]-7-methyl-purin
(132) 8-(4-Amidino-phenyl)-6-methoxy-2-[(2-methoxycarbonylethyl)-aminocarbonyl]-7-methyl-purin
(133) 8-(4-Amidino-phenyl)-6-dimethylamino-2-[(2-methoxycarbonyl-ethyl)-aminocarbonyl]-7-methyl-purin
(134) 8-(4-Amidino-phenyl)-6-amino-2-[(2-methoxycarbonylethyl)-aminocarbonyl]-7-methyl-purin
(135) 8-(4-Amidino-phenyl)-2-[(2-methoxycarbonyl-ethyl)-aminocarbonyl]-7-methyl-6-piperidino-purin

(136) 8-(4-Amidino-phenyl)-2-[(2-methoxycarbonyl-ethyl)-aminocarbonyl]-7-methyl-hypoxanthin
(137) 2-(4-Amidino-phenyl)-7-[(2-methoxycarbonyl-ethyl)-aminocarbonyl]-imidazo[1,2-a]pyridin
(138) 2-(4-Amidino-phenyl)-7-[(2-methoxycarbonyl-ethyl)-aminocarbonyl]-imidazo[1,2-a]pyrimidin
(139) 2-(4-Amidino-phenyl)-7-[(2-methoxycarbonyl-ethyl)-aminocarbonyl]-imidazo[1,2-c]pyrimidin
(140) 2-(4-Amidino-phenyl)-5-[(2-methoxycarbonyl-ethyl)-aminocarbonyl]-benzthiazol
(141) 8-(4-Amidino-phenyl)-3,9-dimethyl-1-(4-methoxycarbonylbutyl)-xanthin-hydrochlorid-hydroacetat
Schmelzpunkt: 224-226 ° C (Zers.)
$R_f$-Wert: 0,58 (Kieselgel; n-Butanol/Eisessig/Wasser = 4:1:1,5)
(142) 2-(4-Amidino-phenyl)-7-[(2-methoxycarbonyl-ethyl)-aminocarbonyl]-3-methyl-imidazo[1,2-a]pyridin
(143) 2-(4-Amidino-phenyl)-5-[(2-methoxycarbonyl-ethyl)-aminocarbonyl]-1-methyl-6-(3-phenyl-propyloxy)-benzimidazol
(144) 2-(4-Amidino-phenyl)-5-[(2-methoxycarbonyl-ethyl)-aminocarbonyl]-1-(4-methyl-benzyl)-benzimidazol
(145) 2-(4-Amidino-phenyl)-1-[2-(3,5-dichlor-4-hydroxy-phenyl)-ethyl]-5-[(2-methoxycarbonyl-ethyl)-aminocarbonyl]-benzimidazol
(146) 2-(4-Amidino-phenyl)-5-[(2-methoxycarbonyl-ethyl)-aminocarbonyl]-1-[(2-phenyl-ethyl)-aminocarbonylmethyl]-benzimidazol
(147) 2-(4-Amidino-phenyl)-5-[(3-methoxycarbonyl-piperidino)-carbonyl]-1-methyl-benzimidazol
$R_f$-Wert: 0,61 (Reversed Phase Platte RP18; Methanol/5%ige Natriumchloridlösung = 6:4)
(148) 2-(4-Amidino-phenyl)-5-[(3-methoxycarbonyl-pyrrolidino)-carbonyl]-1-methyl-benzimidazol
(149) 2-(4-Amidino-phenyl)-5-[(2-methoxycarbonyl-pyrrolidino)-carbonyl]-1-methyl-benzimidazol
$R_f$-Wert: 0,26 (Reversed Phase Platte RP18; Methanol/5%ige Natriumchloridlösung = 6:4)

## Beispiel 4

### 2-(4-Cyan-phenyl)-5(6)-[(2-methoxycarbonyl-ethyl)-carbonylamino]-benzimidazol

Zu einer Mischung aus 1,5 g 5(6)-Amino-2-(4-cyan-phenyl)-benzimidazol, 0,84 g N-Ethyl-diisopropylamin, 20 ml Methylenchlorid und 0,05 g 4-Dimethylamino-pyridin werden 0,98 g Bernsteinsäure-methylesterchlorid zugetropft. Man läßt 16 Stunden bei Raumtemperatur stehen und fügt dann schwach angesäuertes Wasser, das sehr wenig Methanol enthält zu. Das erhaltene Kristallisat wird in einer Mischung aus Essigester, Tetrahydrofuran und Methanol aufgenommen, mit 0,1 N Salzsäure verrührt, die organischen Lösungsmittel im Vakuum abdestilliert und der erhaltene Niederschlag abfiltriert.
Ausbeute: 1,4 g (63 % der Theorie),
Schmelzpunkt: 154-156 ° C

## Beispiel 5

### 2-(4-Amidino-phenyl)-5-[(2-methoxycarbonyl-ethyl)-aminocarbonyl]-1-[3-(S-oxido-thiomorpholino)-propyl]-benzimidazolhydrochlorid

2,2 g 2-(4-Amidino-phenyl)-5-[(2-methoxycarbonyl-ethyl)-aminocarbonyl]-1-(3-thiomorpholino-propyl)-benzimidazol-hydrochlorid werden in 45 ml Eisessig gelöst und unter Kühlung mit Eiswasser mit einer Lösung von 0,39 ml 30%igem Wasserstoffperoxid in 5 ml Eisessig versetzt. Man rührt 4 Stunden unter Eiskühlung und 16 Stunden bei Raumtemperatur nach, dampft das Lösungsmittel im Vakuum ab und reinigt den Rückstand säulenchromatographisch (Kieselgel; Methylenchlorid/Methanol/Wasser = 1:1:0 bis 1:1:0,1).
Ausbeute: 0,6 g (26 % der Theorie),
$R_f$-Wert: 0,46 (Kieselgel; Methylenchlorid/Methanol/konz. Ammoniak = 1:1:0,1)
Analog werden folgende Verbindungen erhalten:
(1) 2-(4-Amidino-phenyl)-5(6)-[(2-methoxycarbonyl-ethyl)-sulfinyl]-benzimidazol
(2) 2-(4-Amidino-phenyl)-5(6)-[(3-methoxycarbonyl-propyl)-sulfinyl]-benzimidazol
(3) 2-(4-Amidino-phenyl)-5(6)-[(2-methoxycarbonyl-ethyl)-sulfinylmethyl]-benzimidazol
(4) 2-(4-Amidino-phenyl)-5-[(2-methoxycarbonyl-ethyl)-aminocarbonyl]-1-(3-methylsulfinyl-phenyl)-benzimidazol
(5) 2-(4-Amidino-phenyl)-5-[(2-methoxycarbonyl-ethyl)-aminocarbonyl]-1-(3-methylsulfinyl-propyl)-benzimidazol
(6) 2-(4-Amidino-phenyl)-1-[3-(S,S-dioxido-thiomorpholino)-propyl]-5-[(2-methoxycarbonyl-ethyl)-aminocarbonyl]-benzimidazol-hydrochlorid

Man arbeitet mit der zehnfachen Menge Wasserstoffperoxid unter 7-stündigem Erwärmen auf 50 °C
$R_f$-Wert: 0,26 (Kieselgel; Butanol/Eisessig/Wasser = 4:1:2, nach zweimaliger Entwicklung)
(7) 2-(4-Amidino-phenyl)-5-[(2-methoxycarbonyl-ethyl)-aminocarbonyl]-1-[2-(S-oxido-thiomorpholinocarbonyl)-ethyl]-benzimidazol

Beispiel 6

2-(4-Aminomethyl-phenyl)-1-(3-amino-propyl)-5-[(2-methoxycarbonyl-ethyl)-aminocarbonyl]-benzimidazol

3 g 1-(3-Amino-propyl)-2-(4-cyan-phenyl)-5-[(2-methoxycarbonyl-ethyl)-aminocarbonyl]-benzimidazol werden in einer Mischung aus 50 ml Methanol und 10 ml methanolischer Salzsäure mit Wasserstoff von 5 bar in Gegenwart von 1 g 10%iger Palladiumkohle 20 Stunden bei Raumtemperatur behandelt. Nach Abfiltrieren des Katalysators wird im Vakuum zur Trockne eingeengt und der Rückstand durch Chromatographie an Kieselgel gereinigt (Elutionsmittel: Methanol/2N Ammoniak = 5:1,5 bis 5:2).
Ausbeute: 1,5 g (55 % der Theorie),
$R_f$-Wert: 0,24 (Kieselgel; Methanol/2N Ammoniak = 5:1,5 nach zweimaliger Entwicklung)
Analog werden folgende Verbindungen erhalten:
(1) 2-(4-Aminomethyl-phenyl)-5(6)-[(2-methoxycarbonyl-ethyl)-aminocarbonyl]-benzimidazol-hydrochlorid
(2) 2-(4-Aminomethyl-phenyl)-5-[(2-methoxycarbonyl-ethyl)-aminocarbonyl]-1-(3-thiomorpholino-propyl)-benzimidazol
$R_f$-Wert: 0,40 (Kieselgel; Methylenchlorid/Methanol/konz. Ammoniak = 8:2:0,1)
(3) 2-(4-Aminomethyl-phenyl)-5-[(3-methoxycarbonyl-propyl)-aminocarbonyl]-1-methyl-benzimidazol
(4) 2-(4-Aminomethyl-phenyl)-5-[(2-methoxycarbonyl-ethyl)-aminocarbonyl]-1-methyl-benzimidazol-dihydrochlorid
Schmelzpunkt: sintert ab 185 °C
$R_f$-Wert: 0,57 (Reversed Phase Platte RP18; Methanol/5%ige Natriumchloridlösung = 6:4)
(5) 2-(4-Aminomethyl-phenyl)-6-[(2-methoxycarbonyl-ethyl)-aminocarbonyl]-imidazo[4,5-b]pyridin
(6) 2-(4-Aminomethyl-phenyl)-1-[2-(3,4-dimethoxy-phenyl)-ethyl]-5-[(2-methoxycarbonyl-ethyl)-aminocarbonyl]-benzimidazol-dihydrochlorid
$R_f$-Wert: 0,03 (Kieselgel; Essigester/Ethanol/konz. Ammoniak = 8:2:0,02)
(7) 2-(4-Aminomethyl-phenyl)-5-[(2-methoxycarbonyl-ethyl)-aminocarbonyl]-1-n-tetradecyl-benzimidazol-dihydrochlorid
$R_f$-Wert: 0,03 (Kieselgel; Essigester/Ethanol/konz. Ammoniak = 50:2:0,02)

Beispiel 7

2-(4-Aminomethyl-cyclohexyl)-5-[(2-carboxy-ethyl)-aminocarbonyl]-1-methyl-benzimidazol

Herstellung aus 5-[(2-carboxy-ethyl)-aminocarbonyl]-1-methyl-2-(4-phthalimidomethyl-cyclohexyl)-benzimidazol durch 20-stündiges Behandeln mit 40%iger wäßriger Methylaminlösung bei Raumtemperatur.

Analog werden folgende Verbindungen erhalten:
(1) 2-[4-(2-Amino-ethyl)-phenyl]-5-[(3-carboxy-propyl)-carbonyl]-1-methyl-benzimidazol
(2) 2-[4-(2-Amino-ethyl)-phenyl]-5-[(2-carboxy-ethyl)-carbonyl]-1-methyl-benzimidazol
(3) 2-(4-Amino-cyclohexyl)-5-[(2-carboxy-ethyl)-aminocarbonyl]-1-methyl-benzimidazol

Beispiel 8

2-(4-Aminomethyl-cyclohexyl)-5-[(2-methoxycarbonyl-ethyl)aminocarbonyl]-1-methyl-benzimidazol-dihydrochlorid

Herstellung aus 2-(4-Aminomethyl-cyclohexyl)-5-[(2-carboxyethyl)-aminocarbonyl]-1-methyl-benzimidazol durch Behandeln mit gesättigter methanolischer Salzsäure bei Raumtemperatur.

Analog werden folgende Verbindungen erhalten:
(1) 2-[4-(2-Amino-ethyl)-phenyl]-5-[(3-methoxycarbonyl-propyl)-carbonyl]-1-methyl-benzimidazol-dihydrochlorid
(2) 2-[4-(2-Amino-ethyl)-phenyl]-5-[(2-methoxycarbonylethyl)-carbonyl]-1-methyl-benzimidazol-dihydrochlorid

(3) 5-[(2-Methoxycarbonyl-ethyl)-aminocarbonyl]-1-methyl-2-(4-methylaminomethyl-phenyl)-benzimidazol-dihydrochlorid

(4) 2-(4-Amino-cyclohexyl)-5-[(2-methoxycarbonyl-ethyl)-aminocarbonyl]-1-methyl-benzimidazol-dihydrochlorid

Beispiel 9

5-[(2-Carboxy-ethyl)-aminocarbonyl]-1-methyl-2-(4-methylaminomethyl-phenyl)-benzimidazol

Herstellung aus 5-[(2-Carboxy-ethyl)-aminocarbonyl]-1-methyl-2-[4-(N-trifluoracetyl-methylaminomethyl)-phenyl]-benzimidazol durch Behandeln mit 2 N Natronlauge.

Beispiel 10

2-(1-Amidino-4-piperidinyl)-5-[(2-methoxycarbonyl-ethyl)-aminocarbonyl]-1-methyl-benzimidazol

Herstellung aus 5-[(2-Methoxycarbonyl-ethyl)-aminocarbonyl]-1-methyl-2-(4-piperidinyl)-benzimidazol und S-Ethylisothioharnstoff-hydrobromid durch vierstündiges Erwärmen auf 100 ° C in Dimethylformamid in Gegenwart von Natriumcarbonat.

Analog werden folgende Verbindungen erhalten:

(1) 2-(4-Guanidinomethyl-phenyl)-5-[(2-methoxycarbonylethyl)-carbonyl]-1-methyl-benzimidazol

(2) 2-(4-Amidino-1-piperazinyl)-5-[(2-methoxycarbonylethyl)-aminocarbonyl]-1-methyl-benzimidazol

Beispiel 11

2-(3-Guanidino-phenyl)-5-[(3-methoxycarbonyl-propyl)-aminocarbonyl]-1-methyl-benzimidazol

Herstellung aus 2-(3-Amino-phenyl)-5-[(3-methoxycarbonylpropyl)-aminocarbonyl]-1-methyl-benzimidazol-hydrochlorid durch dreistündiges Rückflußkoche mit Cyanamid in Dioxan.

Beispiel 12

2-(4-Methoxycarbonylamidino-phenyl)-5-[(2-methoxycarbonylethyl)-aminocarbonyl]-1-methyl-benzimidazol

0,26 g 2-(4-Amidino-phenyl)-5-[(2-methoxycarbonyl-ethyl)-aminocarbonyl]-1-methyl-benzimidazol-hydrochlorid werden in 50 ml Methylenchlorid suspendiert und unter Eiskühlung mit 0,5 ml N-Ethyl-diisopropylamin und 0,067 ml Chlorameisensäure-methylester versetzt. Man rührt bei Raumtemperatur, setzt nach einer halben Stunde weitere 0,5 ml N-Ethyl-diisopropylamin und 0,03 ml Chlorameisensäure-methylester und nach einer weiteren halben Stunde noch 0,1 ml N-Ethyl-diisopropylamin sowie 0,03 ml Chlorameisensäure-methylester zu. Man rührt eine halbe Stunde weiter, dampft im Vakuum ein und reinigt säulenchromatographisch (Kieselgel; Elutionsmittel: Essigester/Ethanol = 100:2,5 bis 100:12,5).

Ausbeute: 0,12 g (40 % der Theorie),

$R_f$-Wert: 0,41 (Kieselgel; Essigester/Ethanol = 8:2)

Analog werden folgende Verbindungen erhalten:

(1) 2-(4-Isobutyloxycarbonylamidino-phenyl)-5-[(2-methoxycarbonyl-ethyl)-aminocarbonyl]-1-methyl-benzimidazol

(2) 5-[(2-Ethoxycarbonyl-ethyl)-aminocarbonyl]-2-(4-methoxycarbonylamidino-phenyl)-1-methyl-benzimidazol

(3) 5-[(2-Isobutyloxycarbonyl-ethyl)-aminocarbonyl]-2-(4-methoxycarbonylamidino-phenyl)-1-(4-phenyl-butyl)-benzimidazol

(4) 2-(4-Acetoxymethyloxycarbonylamidino-phenyl)-5-[(2-methoxycarbonyl-ethyl)-aminocarbonyl]-1-(4-phenyl-butyl)-benzimidazol

(5) 2-(4-Cyan-phenyl)-6-(N-isobutyloxycarbonyl-N-methylamino)-5-[(2-methoxycarbonyl-ethyl)-aminocarbonyl]-1-methylbenzimidazol

$R_f$-Wert: 0,63 (Kieselgel; Methylenchlorid/Methanol/Eisessig = 25:1:0,1)

Beispiel 13

2-(4-Amidino-phenyl)-1-[2-(3,4-dimethoxy-phenyl)-ethyl]-5-[(2-isopropyloxycarbonyl-ethyl)-aminocarbonyl]-benzimidazoldihydrochlorid

Zu 50 ml eiskalter gesättigter isopropanolischer Salzsäure fügt man 0,6 g 2-(4-Amidino-phenyl)-1-[2-(3,4-dimethoxy-phenyl)-ethyl]-5-[(2-methoxycarbonyl-ethyl)-aminocarbonyl]-benzimidazol-hydrochlorid, überschichtet mit Petrolether und rührt vorsichtig bis der größte Teil des Festproduktes in Lösung gegangen ist. Man läßt 16 Stunden bei Raumtemperatur stehen, filtriert vom vorhandenen Niederschlag ab, dampft das Filtrat im Vakuum ein und verrührt den Rückstand mit Tetrahydrofuran.
Ausbeute: 0,5 g (76 % der Theorie),
Schmelzpunkt: 210 ° C (sintert ab 180 ° C)
Analog wird folgende Verbindung erhalten:

(1) 2-(4-Amidino-phenyl)-5-[(2-n-butyloxycarbonyl-ethyl)-aminocarbonyl]-1-methyl-benzimidazol-dihydrochlorid

(2) 2-(4-Amidino-phenyl)-5-[(2-isopropyloxycarbonyl-ethyl)-aminocarbonyl]-1-(4-phenyl-butyl)-benzimidazol-dihydrochlorid

(3) 2-(4-Amidino-phenyl)-5-[(2-cyclohexyloxycarbonyl-ethyl)-aminocarbonyl]-1-(4-phenyl-butyl)-benzimidazol-dihydrochlorid $R_f$-Wert: 0,69 (Kieselgel; n-Butanol/Eisessig/Wasser = 3:1:1)

(4) 2-(4-Amidino-phenyl)-5-[(2-n-hexyloxycarbonyl-ethyl)-aminocarbonyl]-1-(4-phenyl-butyl)-benzimidazol-dihydrochlorid

(5) 2-(4-Amidino-phenyl)-5-[(2-isobutyloxycarbonyl-ethyl)-aminocarbonyl]-1-(4-phenyl-butyl)-benzimidazol-dihydrochlorid

(6) 2-(4-Amidino-phenyl)-5-[(2-cyclohexylmethyloxycarbonylethyl)-aminocarbonyl]-1-(4-phenyl-butyl)-benzimidazol-dihydrochlorid
$R_f$-Wert: 0,67 (Kieselgel; n-Butanol/Eisessig/Wasser = 3:1:1)

(7) 2-(4-Amidino-phenyl)-5-[[2-(2-indanyloxycarbonyl)-ethyl]-aminocarbonyl]-1-methyl-benzimidazol-dihydrochlorid
Man geht von der entsprechenden Carbonsäure aus und erwärmt in Gegenwart von Chlorwasserstoff

(8) 2-(4-Amidino-phenyl)-1-methyl-5-[[2-[(1-naphthyl)-methyloxycarbonyl]-ethyl]-aminocarbonyl]-benzimidazol-dihydrochlorid
Man verfährt wie unter (7)

(9) 2-(4-Amidino-phenyl)-5-[(2-cinnamyloxycarbonyl-ethyl)-aminocarbonyl]-1-methyl-benzimidazol-dihydrochlorid
Man verfährt wie unter (7)

(10) 2-(4-Amidino-phenyl)-1-methyl-5-[[2-[(2-norbornyl)-methyloxycarbonyl]-ethyl]-aminocarbonyl]-benzimidazol-dihydrochlorid
$R_f$-Wert: 0,75 (Kieselgel; Methylenchlorid/Methanol = 2:1)

(11) 2-(4-Amidino-phenyl)-1-methyl-5-[[2-(2-norbornyloxycarbonyl)-ethyl]-aminocarbonyl]-benzimidazol-dihydrochlorid
Man verfährt wie unter (7)

(12) 2-(4-Amidino-phenyl)-1-methyl-5-[[2-[(5-phenyl-butyl)-oxycarbonyl]-ethyl]-aminocarbonyl]-benzimidazol-dihydrochlorid
Man verfährt wie unter (7)

(13) 2-(4-Amidino-phenyl)-1-methyl-5-[[2-[4-phenyl-butyl)-oxycarbonyl]-ethyl]-aminocarbonyl]-benzimidazol-dihydrochlorid
Man verfährt wie unter (7)

(14) 2-(4-Amidino-phenyl)-5-[(2-cyclooctyloxycarbonyl-ethyl)-aminocarbonyl]-1-methyl-benzimidazol-dihydrochlorid
$R_f$-Wert: 0,67 (Kieselgel; n-Butanol/Eisessig/Wasser = 3:1:1)

(15) 2-(4-Amidino-phenyl)-1-(4-phenyl-butyl)-5-[[2-[(2-phenyl-ethyl)-oxycarbonyl]-aminocarbonyl]-benzimidazol-dihydrochlorid
$R_f$-Wert: 0,78 (Kieselgel; n-Butanol/Eisessig/Wasser = 3:1:1)

(16) 2-(4-Amidino-phenyl)-1-[2-(4-amino-3,5-dibrom-phenyl)-ethyl]-5-[(2-isopropyloxycarbonyl-ethyl)-aminocarbonyl]-benzimidazol-dihydrochlorid
$R_f$-Wert: 0,18 (Reversed-Phase-Platte RP18; Methanol/5%ige Natriumchloridlösung = 6:4)

(17) 2-(4-Amidino-phenyl)-1-[2-(2-brom-4,5-dimethoxy-phenyl)-ethyl]-5-[[2-(2-norbornyloxycarbonyl-ethyl)-aminocarbonyl]-benzimidazol-di-hydrogenacetat

Man erhitzt 2-(4-Amidino-phenyl)-5-[(2-carboxy-ethyl)-aminocarbonyl]-1-[2-(3,4-dimethoxy-phenyl)-ethyl]-benzimidazol-dihydrobromid zwei Stunden mit Thionylchlorid und setzt das gebildete Säurechlorid mit Norborneol um. Die Reinigung geschieht durch Chromatographie an Kieselgel (Elutionsmittel: n-Butanol/Eisessig/Wasser = 3:1:1)
$R_f$-Wert: 0,72 (Kieselgel; n-Butanol/Eisessig/Wasser = 3:1:1)
(18) 2-(4-Amidino-phenyl)-1-[2-(2-brom-4,5-dimethoxy-phenyl)-ethyl]-5-[[2-(2-indanyloxycarbonyl)-ethyl]-aminocarbonyl]-benzimidazol-di-hydrogenacetat
Man verfährt analog (17)
$R_f$-Wert: 0,65 (Kieselgel; n-Butanol/Eisessig/Wasser = 3:1:1)

Beispiel 14

2-[(4-Cyan-phenyl)-oxymethyl]-5(6)-ethoxycarbonylmethoxybenzimidazol

4,5 g 3-Amino-4-[(4-cyan-phenyl)-oxymethylcarbonylamino]-phenoxy-essigsäure-ethylester werden geschmolzen und 15 Minuten bei 200 ° C gehalten. Das Produkt wird durch Säulenchromatographie, zunächst über Kieselgel (Elutionsmittel; Methylenchlorid/Methanol/konz. Ammoniak = 30:1:0,1) und anschliessend über neutrales Aluminiumoxid (Elutionsmittel: Methylenchlorid/Methanol = 100:1) gereinigt und aus einer 1:2-Mischung von Methylenchlorid und Ether kristallisiert.
Ausbeute: 2,5 g (58 % der Theorie),
Schmelzpunkt: 111-112 ° C
$R_f$-Wert: 0,22 (Kieselgel; Methylenchlorid/Methanol/konz. Ammoniak = 30:1:0,1)
Analog werden folgende Verbindungen erhalten:
(1) 1-[2-(4-Benzyl-piperazino)-ethyl]-2-(4-cyan-phenyl)-5-[2-methoxycarbonyl-ethyl)-aminocarbonyl]-benzimidazol
$R_f$-Wert: 0,58 (Kieselgel; Methylenchlorid/Methanol/konz.
Ammoniak = 19:1:0,1, nach dreifacher Entwicklung)
(2) 1-(3-Amino-propyl)-2-(4-cyan-phenyl)-5-[(2-methoxycarbonyl-ethyl)-aminocarbonyl]-benzimidazol-hydrochlorid
3-Amino-4-(3-amino-propylamino)-benzoesäure-[N-(2-methoxycarbonyl-ethyl)-amid]-hydrochlorid-hydrogenacetat wird zunächst durch Behandeln mit 4-Cyan-benzoylchlorid in Chlorbenzol bei 140 ° C Badtemperatur acyliert und dabei gleichzeitig das Chlorbenzol abdestilliert. Den Rückstand erhitzt man 10 Minuten auf 190 ° C.
Schmelzpunkt: 252 ° C (Zers., sintert ab 115 ° C)
$R_f$-Wert: 0,54 (Kieselgel; Methylenchlorid/Methanol/konz. Ammoniak = 9:1:0,2, nach zweimaliger Entwicklung)
(3) 2-(4-Cyan-phenyl)-5-[(2-methoxycarbonyl-ethyl)-aminocarbonyl]-1-methyl-6-methylamino-benzimidazol
Man erhitzt 35 Minuten.
Schmelzpunkt: 203-205 ° C
$R_f$-Wert: 0,68 (Kieselgel; Methylenchlorid/Methanol/konz. Ammoniak = 30:1:0,1, nach dreifacher Entwicklung)
(4) 8-(4-Cyan-phenyl)-3,9-dimethyl-xanthin 5-Amino-3-methyl-4-methylamino-pyrimidin-2,6-dion wird zunächst in Gegenwart von 4-Dimethylamino-pyridin in Tetrahydrofuran mit 4-Cyan-benzoylchlorid acyliert und der Eindampfrückstand 45 Minuten auf 200-205 ° C erhitzt.
$R_f$-Wert: 0,79 (Kieselgel; Methylenchlorid/Methanol/Eisessig = 9:1:0,1 nach zweifacher Entwicklung)

Beispiel 15

2-(4-Amidino-phenyl)-5-[(2-carboxy-ethyl)-aminocarbonyl]-1-(2-piperazino-ethyl)-benzimidazol-hydrogenacetat

0,30 g 2-(4-Amidino-phenyl)-1-[2-(4-benzyl-piperazino)-ethyl]-5-[(2-carboxy-ethyl)-aminocarbonyl]-benzimidazol werden in 100 ml Eisessig gelöst und in Gegenwart von 1,8 g Palladiumoxid 36 Stunden bei Raumtemperatur mit Wasserstoff von 5 bar behandelt. Nach Abfiltrieren des Katalysators wird im Vakuum eingedampft und der Rückstand mit Aceton und dann Ether digeriert.
Ausbeute: 0,27 g (92 % der Theorie),
Schmelzpunkt: 80 ° C (Zers.)

$R_f$-Wert: 0,14 (Kieselgel; Isopropanol/Wasser/konz. Ammoniak = 7:2:1, nach zweifacher Entwicklung)
Analog wird folgende Verbindung erhalten:

(1) 2-(4-Amidino-phenyl)-5(6)-[(2-methoxycarbonyl-ethyl)-aminocarbonyl]-benzimidazol-hydrochlorid
Man arbeitet in Methanol mit 10%iger Palladiumkohle.
$R_f$-Wert: 0,65 (Kieselgel; Methylenchlorid/Methanol = 3:1)

(2) 2-(4-Amidino-phenyl)-1-(4-phenyl-butyl)-5-[(2-pivaloyloxymethyloxycarbonyl-ethyl)-aminocarbonyl]-benzimidazol

(3) 2-(4-Amidino-phenyl)-5-[[2-[(1-ethoxycarbonyloxy-ethyl)-oxycarbonyl]-ethyl]-aminocarbonyl]-1-(4-phenyl-butyl)-benzimidazol

(4) 2-(4-Amidino-phenyl)-5-[(2-cyclohexyloxycarbonyloxymethyloxycarbonyl-ethyl)-aminocarbonyl]-1-(4-phenyl-butyl)-benzimidazol

(5) 5-[(2-Acetoxymethyloxycarbonyl-ethyl)-aminocarbonyl]-2-(4-amidino-phenyl)-1-(4-phenyl-butyl)-benzimidazol

(6) 2-(4-Amidino-phenyl)-5-[(1,2-bis-carboxy-ethyl)-aminocarbonyl]-1-methyl-benzimidazol

(7) 2-(4-Amidino-phenyl)-5-[[2-carboxy-1-[(1-carboxy-2-phenyl-ethyl)-aminocarbonyl]-ethyl]-aminocarbonyl]-1-methyl-benzimidazol

(8) 2-(4-Amidino-phenyl)-5-[[2-carboxy-1-[(1-carboxy-2-methyl-propyl)-aminocarbonyl]-ethyl]-aminocarbonyl]-1-methylbenzimidazol

(9) 2-(4-Amidino-phenyl)-5-[[2-carboxy-1-[[1-carboxy-2-(4-hydroxy-phenyl)-ethyl]-aminocarbonyl]-ethyl]-aminocarbonyl]-1-methyl-benzimidazol

(10) 2-(4-Amidino-phenyl)-5-[[2-carboxy-1-[[1-carboxy-2-(4-methoxy-phenyl)-ethyl]-aminocarbonyl]-ethyl]-aminocarbonyl]-1-methyl-benzimidazol

(11) 2-(4-Amidino-phenyl)-5-[[2-carboxy-1-[[2-(4-methoxyphenyl)-ethyl]-aminocarbonyl]-ethyl]-aminocarbonyl]-1-methyl-benzimidazol

## Beispiel 16

### 2-(4-Cyan-phenyl)-5-[(2-methoxycarbonyl-ethyl)-aminocarbonyl]-1-(3-thiomorpholino-propyl)-benzimidazol

48,6 g 3-Amino-4-(3-thiomorpholino-propylamino)-benzoesäure-[N-(2-methoxycarbonyl-ethyl)-amid]-hydrochlorid werden unter Erwärmen in 500 ml Eisessig gelöst, mit 19,5 g 4-Cyan-benzoylchlorid versetzt, eine Stunde auf dem Dampfbad und anschließend 2 Stunden bei 120°C Badtemperatur erhitzt. Man dampft im Vakuum ein und reinigt den Rückstand durch Chromatographie an Kieselgel (Elutionsmittel: Methylenchlorid/Methanol/konz. Ammoniak = 9:1:0 bis 8:2:0,2).
Ausbeute: 33,7 g (58 % der Theorie),
$R_f$-Wert: 0,84 (Kieselgel; Methylenchlorid/Methanol/konz. Ammoniak = 19:1:0,1, nach dreifacher Entwicklung)
Analog werden folgende Verbindungen erhalten:

(1) 2-(4-Cyan-phenyl)-1-[2-(3,4-dimethoxy-phenyl)-ethyl]-5-[(2-methoxycarbonyl-ethyl)-aminocarbonyl]-benzimidazol
Die Acylierung mit 4-Cyan-benzoylchlorid wird zunächst in Methylenchlorid unter Zusatz von N-Ethyl-diisopropylamin durchgeführt. Nach Abdampfen des Lösungsmittels nimmt man in Eisessig auf und erwärmt 1,5 Stunden auf 100°C.
$R_f$-Wert: 0,36 (Kieselgel; Essigester/Ethanol/konz. Ammoniak = 50:2:0,1)

(2) 2-(4-Cyan-phenyl)-5-[(2-methoxycarbonyl-ethyl)-aminocarbonyl]-1-(3-pyridylmethyl)-benzimidazol
Man verfährt wie unter (1) mit 3 Stunden Erhitzen in Eisessig
$R_f$-Wert: 0,40 (Kieselgel; Essigester/Ethanol = 8:2)

(3) 2-(4-Cyan-phenyl)-5-[(2-methoxycarbonyl-ethyl)-aminocarbonyl]-1-n-tetradecyl-benzimidazol
Man verfährt wie unter (1)
$R_f$-Wert: 0,60 (Kieselgel; Essigester/Ethanol/konz. Ammoniak = 50:2:0,02)

(4) 2-(4-Cyan-phenyl)-5-[(2-methoxycarbonyl-ethyl)-aminocarbonyl]-1-(3-methoxycarbonyl-propyl)-benzimidazol
Man verfährt wie unter (1) mit Triethylamin als Hilfsbase.
Als Lösungsmittel dient ein 3:1-Gemisch aus Tetrahydrofuran und Methylenchlorid.
$R_f$-Wert: 0,68 (Kieselgel; Methylenchlorid/Methanol = 37:3)

(5) 1-Benzyl-2-(4-cyan-phenyl)-5-[(2-methoxycarbonyl-ethyl)-aminocarbonyl]-benzimidazol
Man verfährt wie unter (4).
$R_f$-Wert: 0,77 (Kieselgel; Methylenchlorid/Methanol = 9:1)

(6) 2-(4-Cyan-phenyl)-5-[(2-methoxycarbonyl-ethyl)-aminocarbonyl]-1-(4-phenyl-butyl)-benzimidazol
Man verfährt wie unter (1).
$R_f$-Wert: 0,61 (Kieselgel; Essigester/Ethanol = 50:2)
(7) 1-[2-(4-Amino-3,5-dibrom-phenyl)-ethyl]-2-(4-cyan-phenyl)-5-[(2-methoxycarbonyl-ethyl)-aminocarbonyl]-benzimidazol
Man verfährt wie unter (1).
Schmelzpunkt: 168-170 ° C,
$R_f$-Wert: 0,66 (Kieselgel; Essigester/Ethanol = 8:2)
(8) 2-(4-Cyan-phenyl)-1-(3,3-diphenyl-propyl)-5-[(2-methoxycarbonyl-ethyl)-aminocarbonyl]-benzimidazol
Schmelzpunkt: 157-159 ° C,
$R_f$-Wert: 0,65 (Kieselgel; Essigester/Ethanol = 9:1)
(9) 2-(4-Cyan-phenyl)-5-[(2-methoxycarbonyl-pyrrolidino)-carbonyl]-1-methyl-benzimidazol
Man verfährt wie unter (1)
Schmelzpunkt: 174-176 ° C,
$R_f$-Wert: 0,36 (Kieselgel; Essigester/Ethanol = 9:1)
(10) 2-(4-Cyan-phenyl)-5-[(3-methoxycarbonyl-piperidino)-carbonyl]-1-methyl-benzimidazol
Man verfährt wie unter (1)
Schmelzpunkt: 185-186 ° C,
$R_f$-Wert: 0,43 (Kieselgel; Essigester/Ethanol = 9:1)
(11) 2-(4-Cyan-phenyl)-6-[(2-methoxycarbonyl-ethyl)-aminocarbonyl]-3-methyl-imidazo[4,5-b]pyridin
Man verfährt wie unter (1)
Schmelzpunkt: 195-197 ° C,
$R_f$-Wert: 0,51 (Kieselgel; Essigester/Ethanol = 9:1)

Beispiel 17

2-(4-Aminomethyl-phenyl)-1-(3-amino-propyl)-5-[(2-carboxyethyl)-aminocarbonyl]-benzimidazol-hydrogenacetat

0,8 g 2-(4-Aminomethyl-phenyl)-1-(3-amino-propyl)-5-[(2-methoxycarbonyl-ethyl)-aminocarbonyl]-benzimidazol werden 20 Stunden bei Raumtemperatur in 10 ml 48%iger Bromwasserstoffsäure stehen gelassen. Man engt im Vakuum ein und reinigt den Ruckstand durch Chromatographie an Kieselgel (Elutionsmittel: n-Butanol/Eisessig/Wasser = 4:1:1 bis 4:2:2).
Ausbeute: 0,6 g (67 % der Theorie),
$R_f$-Wert: 0,66 (Reversed Phase Platte RP18; Methanol/5%ige Natriumchloridlösung/konz. Bromwasserstoffsäure = 6:10:0,01)
Analog werden folgende Verbindungen erhalten:
(1) 2-(4-Aminomethyl-phenyl)-5-[(2-carboxy-ethyl)-aminocarbonyl]-1-methyl-benzimidazol-dihydrobromid
Schmelzpunkt: sintert ab 220 ° C
$R_f$-Wert: 0,87 (Reversed Phase Platte RP18; Methanol/5%ige Natriumchloridlösung = 6:4)
(2) 2-(4-Amidino-phenyl)-5-[(2-carboxy-ethyl)-aminocarbonyl]-1-[2-(3,4-dimethoxy-phenyl)-ethyl]-benzimidazol-dihydrobromid
$R_f$-Wert: 0,72 (Reversed Phase Platte RP18; Methanol/5%ige Natriumchloridlösung = 6:4)
(3) 2-(4-Amidino-phenyl)-5-[(2-carboxy-ethyl)-aminocarbonyl]-1-(3-pyridylmethyl)-benzimidazol-dihydrobromid
$R_f$-Wert: 0,73 (Reversed Phase Platte RP18; Methanol/5%ige Natriumchloridlösung = 6:4)
(4) 2-(4-Aminomethyl-phenyl)-5-[(2-carboxy-ethyl)-aminocarbonyl]-1-n-tetradecyl-benzimidazol-dihydrobromid
$R_f$-Wert: 0,05 (Reversed Phase Platte RP18; Methanol/5%ige Natriumchloridlösung = 6:4)
(5) 2-(4-Aminomethyl-phenyl)-5-[(2-carboxy-ethyl)-aminocarbonyl]-1-[2-(3,4-dimethoxy-phenyl)-ethyl]-benzimidazol-dihydrobromid
$R_f$-Wert: 0,67 (Reversed Phase Platte RP18; Methanol/5%ige Natriumchloridlösung = 6:4)
(6) 2-(4-Amidino-phenyl)-5-[(2-carboxy-ethyl)-aminocarbonyl]-1-n-tetradecyl-benzimidazol-dihydrobromid
$R_f$-Wert: 0,01 (Reversed Phase Platte RP18; Methanol/5%ige Natriumchloridlösung = 6:4)
(7) 2-(4-Amidino-phenyl)-5-[(2-carboxy-ethyl)-aminocarbonyl]-1-(4-phenyl-butyl)-benzimidazol-dihydrobromid
$R_f$-Wert: 0,08 (Reversed Phase Platte RP18; Methanol/5%ige Natriumchloridlösung = 6:4)

(8) 2-(4-Amidino-phenyl)-1-[2-(4-amino-3,5-dibrom-phenyl)-ethyl]-5-[(2-carboxy-ethyl)-aminocarbonyl]-benzimidazol-dihydrobromid
Schmelzpunkt: über 225 ° C
$R_f$-Wert: 0,57 (Reversed Phase Platte RP18; Methanol/5%ige Natriumchloridlösung = 6:4)
(9) 2-(4-Amidino-phenyl)-5-[(2-carboxy-ethyl)-aminocarbonyl]-1-(3,3-diphenyl-propyl)-benzimidazol-dihydrobromid
$R_f$-Wert: 0,71 (Kieselgel; n-Butanol/Eisessig/Wasser = 3:1:1)
(10) 2-(4-Amidino-phenyl)-5-[(3-carboxy-piperidino)-carbonyl]-1-methyl-benzimidazol-dihydrobromid
$R_f$-Wert: 0,49 (Reversed Phase Platte RP18; Methanol/5%ige Natriumchloridlösung = 6:4)
(11) 2-(4-Amidino-phenyl)-5-[(3-carboxy-pyrrolidino)-carbonyl]-1-methyl-benzimidazol-dihydrobromid
(12) 2-(4-Amidino-phenyl)-5-[(2-carboxy-pyrrolidino)-carbonyl]-1-methyl-benzimidazol-dihydrobromid
$R_f$-Wert: 0,46 (Reversed Phase Platte RP18; Methanol/5%ige Natriumchloridlösung = 6:4)
(13) 2-(4-Amidino-phenyl)-6-[(2-carboxy-ethyl)-aminocarbonyl]-3-methyl-imidazo[4,5-b]pyridin-dihydrobromid
$R_f$-Wert: 0,74 (Reversed Phase Platte RP18; Methanol/5%ige Natriumchloridlösung = 6:4)

Beispiel 18

2-(4-Cyan-phenyl)-5-[(2-methoxycarbonyl-ethyl)-aminocarbonyl]-1-methyl-benzimidazol

4,7 g 3-Amino-4-methylamino-benzoesäure-[N-(2-methoxycarbonyl-ethyl)-amid] werden zunächst bei Raumtemperatur mit 40 ml Phosphoroxychlorid verrührt, dann mit 3,1 g 4-Cyan-benzoylchlorid versetzt und 8 Stunden auf 90 ° C erhitzt. Man destilliert das Phosphoroxychlorid im Vakuum ab, rührt den Rückstand bei 30 ° C in 500 ml Wasser ein und neutralisiert durch portionsweise Zugabe von Natriumbicarbonat. Man saugt den Niederschlag ab und extrahiert mit Essigester. Die Essigesterphasen werden eingedampft, der Rückstand mit dem Niederschlag vereinigt und durch Chromatographie an Kieselgel gereinigt (Elutionsmittel: Essigester/Ethanol = 100:1 bis 100:5). Das erhaltene Produkt wird zur endgültigen Reinigung noch mit Essigester ausgekocht.
Ausbeute: 1,86 g (27 % der Theorie),
$R_f$-Wert: 0,51 (Kieselgel; Essigester/Ethanol = 9:1)

Beispiel 19

2-(4-Cyan-phenyl)-5-[(2-methoxycarbonyl-ethyl)-aminocarbonyl]-6-(N-methoxycarbonylmethyl-N-methyl-amino]-1-methyl-benzimidazol

5,0 g 2-(4-Cyan-phenyl)-5-[(2-methoxycarbonyl-ethyl)-aminocarbonyl]-1-methyl-6-methylamino-benzimidazol werden in 20 ml Dimethylformamid in der Wärme gelöst und 2,3 ml N-Ethyl-diisopropylamin zugefügt. Man tropft 1,2 ml Bromessigsäure-methylester zu und erhitzt 5 Stunden auf dem Dampfbad. Man versetzt mit weiteren 1,2 ml N-Ethyl-diisopropylamin und 0,6 ml Bromessigsäure-methylester und erhitzt nochmals 3 Stunden. Man dampft das Lösungsmittel im Vakuum ab, verrührt 2 Stunden mit 50 ml Wasser bei Raumtemperatur, saugt ab und verreibt den getrockneten Rückstand mit Essigester.
Ausbeute: 3,2 g (54 % der Theorie),
Schmelzpunkt: 174-176 ° C
$R_f$-Wert: 0,34 (Kieselgel; Methylenchlorid/Methanol = 19:1, nach dreimaliger Entwicklung)
Analog wird folgende Verbindung erhalten:
(1) 8-(4-Cyan-phenyl)-3,9-dimethyl-1-(4-ethoxycarbonyl-butyl)-xanthin
Als Lösungsmittel dient Dimethylsulfoxid, als Base Kaliumcarbonat, wobei man 4 Stunden auf 80 ° C erhitzt.
Schmelzpunkt: 148-150 ° C
$R_f$-Wert: 0,33 (Kieselgel; Essigester)

Beispiel 20

2-(4-Benzyloxycarbonylamidino-phenyl)-1-(4-phenyl-butyl)-5-[(2-pivaloyloxymethyloxycarbonyl-ethyl)-aminocarbonyl]-benzimidazol

Herstellung durch Umsetzung von 2-(4-Benzyloxycarbonylamidino-phenyl)-5-[(2-carboxy-ethyl)-aminocarbonyl]-1-(4-phenyl butyl)-benzimidazol mit Pivaloyloxymethylchlorid in Dimethylsulfoxid in Gegenwart von Kaliumcarbonat unter Zusatz von Kaliumiodid bei Raumtemperatur.

Analog werden folgende Verbindungen erhalten:

(1) 2-(4-Benzyloxycarbonylamidino-phenyl)-5-[[2-[(1-ethoxycarbonyloxy-ethyl)-oxycarbonyl]-ethyl]-aminocarbonyl]-1-(4-phenyl-butyl)-benzimidazol

(2) 2-(4-Benzyloxycarbonylamidino-phenyl)-5-[(2-cyclohexyloxycarbonyloxymethyloxycarbonyl-ethyl)-aminocarbonyl]-1-(4-phenyl-butyl)-benzimidazol

(3) 5-[(2-Acetoxymethyloxycarbonyl-ethyl)-aminocarbonyl]-2-(4-benzyloxycarbonylamidino-phenyl)-1-(4-phenyl-butyl)-benzimidazol

Beispiel 21

2-(4-Diethylphosphorylamidino-phenyl)-5-[(2-methoxycarbonylethyl)-aminocarbonyl]-1-(4-phenyl-butyl)-benzimidazol

Herstellung durch Umsetzung von 2-(4-Amidino-phenyl)-5-[(2-methoxycarbonyl-ethyl)-aminocarbonyl]-1-(4-phenyl-butyl)-benzimidazol mit Diethylphosphorylcyanid in Dimethylformamid.

Beispiel 22

2-(4-Benzyloxycarbonylamidino-phenyl)-5-[(1,2-bis-benzyloxycarbonyl-ethyl)-aminocarbonyl]-1-methyl-benzimidazol

Herstellung aus 2-(4-Benzyloxycarbonylamidino-phenyl)-1-methyl-benzimidazol-4-carbonsäure und Asparaginsäure-dibenzylester unter Zusatz von 2-(1H-Benztriazol-1-yl)-1,1,3,3-tetramethyluronium-tetrafluorborat in Tetrahydrofuran.

Analog werden folgende Verbindungen erhalten:

(1) 2-(4-Benzyloxycarbonylamidino-phenyl)-5-[[2-benzyloxycarbonyl-1-[(1-benzyloxycarbonyl-2-phenyl-ethyl)-aminocarbonyl]-ethyl]-aminocarbonyl]-1-methyl-benzimidazol

(2) 2-(4-Benzyloxycarbonylamidino-phenyl)-5-[[2-benzyloxycarbonyl-1-[(1-benzyloxycarbonyl-2-methyl-propyl)-aminocarbonyl]-ethyl]-aminocarbonyl]-1-methyl-benzimidazol

(3) 2-(4-Benzyloxycarbonylamidino-phenyl)-5-[[2-benzyloxycarbonyl-1-[[1-benzyloxycarbonyl-2-(4-benzyloxy-phenyl)-ethyl]-aminocarbonyl]-ethyl]-aminocarbonyl]-1-methyl-benzimidazol

(4) 2-(4-Benzyloxycarbonylamidino-phenyl)-5-[[2-benzyloxycarbonyl-1-[[1-benzyloxycarbonyl-2-(4-methoxy-phenyl)-ethyl]-aminocarbonyl]-ethyl]-aminocarbonyl]-1-methyl-benzimidazol

(5) 5-[[2-Benzyloxycarbonyl-1-[[2-(4-methoxy-phenyl)-ethyl]-aminocarbonyl]-ethyl]-aminocarbonyl]-2-(4-benzyloxycarbonylamidino-phenyl)-1-methyl-benzimidazol

Beispiel 23

2-[4-(1-Amino-cyclopropyl)-phenyl]-5-[(2-methoxycarbonylethyl)-aminocarbonyl]-1-methyl-benzimidazol

Herstellung aus 2-[4-(1-tert.Butyloxycarbonylamino-cyclopropyl)-phenyl]-5-[(2-methoxycarbonyl-ethyl)-aminocarbonyl]-1-methyl-benzimidazol durch Behandeln mit Trifluoressigsäure in Methylenchlorid bei Raumtemperatur.

Analog wird folgende Verbindung erhalten:

(1) 2-[4-(1-Amino-cyclopentyl)-phenyl]-5-[(2-methoxycarbonylethyl)-aminocarbonyl]-1-methyl-benzimidazol

Beispiel 24

2-(1-Amino-5-indanyl)-5-[(2-methoxycarbonyl-ethyl)-aminocarbonyl]-1-methyl-benzimidazol

Herstellung aus 2-(1-Hydroxyimino-5-indanyl)-5-[(2-methoxycarbonyl-ethyl)-aminocarbonyl]-1-methyl-benzimidazol durch Reduktion mit Wasserstoff von 5 bar in einem 50:1-Gemisch aus Methanol und methanolischer Salzsäure bei Raumtemperatur in Gegenwart von 10%iger Palladiumkohle.

Analog wird folgende Verbindung erhalten:

(1) 2-(1-Amino-1,2,3,4-tetrahydro-6-naphthyl)-5-[(2-methoxycarbonyl-ethyl)-aminocarbonyl]-1-methyl-benzimidazol

Beispiel 25

2-[4-(2-Amino-2-propyl)-phenyl]-5-[(2-methoxycarbonyl-ethyl)-aminocarbonyl]-1-methyl-benzimidazol

Herstellung aus 2-[4-(2-Aminocarbonyl-2-propyl)-phenyl]-5-[(2-methoxycarbonyl-ethyl)-aminocarbonyl]-1-methyl-benzimidazol durch Behandeln mit [Bis(trifluoracetoxy)iod]benzol in Acetonitril/Wasser bei Raumtemperatur.

Beispiel 26

2-(4-Amidino-phenyl)-1-methyl-5-[[2-[[2-(2-oxo-1-pyrrolidinyl)-ethyl]-oxycarbonyl]-ethyl]-aminocarbonyl]-benzimidazol

Herstellung aus 2-(4-Amidino-phenyl)-1-methyl-5-[(2-carboxyethyl)-aminocarbonyl]-1-methyl-benzimidazol durch 36-stündiges Erwärmen auf 60 °C in 1-(2-Hydroxy-ethyl)-2-pyrrolidon und überschüssigem Trimethylchlorsilan.

Analog werden folgende Verbindungen erhalten:

(1) 2-(4-Amidino-phenyl)-1-methyl-5-[[2-[(2-phenyl-ethyl)-oxycarbonyl]-ethyl]-aminocarbonyl]-benzimidazol

(2) 2-(4-Amidino-phenyl)-1-(4-phenyl-butyl)-5-[[2-[(3-pyridyl)methyloxycarbonyl]-ethyl]-aminocarbonyl]-benzimidazol

Beispiel 27

Trockenampulle mit 2,5 mg Wirkstoff pro 1 ml

Zusammensetzung:

| | |
|---|---|
| Wirkstoff | 2,5 mg |
| Mannitol | 50,0 mg |
| Wasser für Injektionszwecke ad | 1,0 ml |

Herstellung:

Wirkstoff und Mannitol werden in Wasser gelöst. Nach Abfüllung wird gefriergetrocknet.

Die Auflösung zur gebrauchsfertigen Lösung erfolgt mit Wasser für Injektionszwecke.

Beispiel 28

Trockenampulle mit 35 mg Wirkstoff pro 2 ml

Zusammensetzung:

| | |
|---|---|
| Wirkstoff | 35,0 mg |
| Mannitol | 100,0 mg |
| Wasser für Injektionszwecke ad | 2,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 29

Tablette mit 50 mg Wirkstoff

Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 30

Tablette mit 350 mg Wirkstoff

Zusammensetzung:

| | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 31

Kapseln mit 50 mg Wirkstoff

Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

Beispiel 32

Kapseln mit 350 mg Wirkstoff

Zusammensetzung:

| | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

**Patentansprüche**

**1.** Kondensierte 5-gliedrige Heterocyclen der allgemeinen Formel

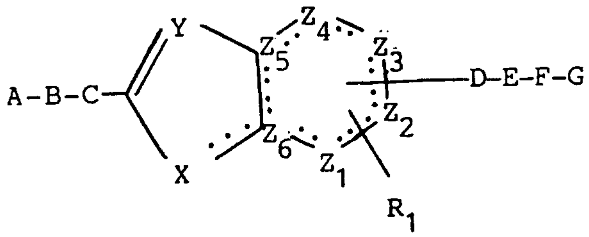

,(I)

in der

$R_1$ ein Wasserstoff-, Fluor-, Chlor- oder Bromatom, eine Alkyl-, Aralkyl-, Aryl-, Heteroaryl-, $R_3O$-, $(R_3)_2N$-, $R_4CO-NR_3$-, Alkylsulfonyl-$NR_3$-, Arylsulfonyl-$NR_3$-, $R_3S$-, $R_3SO$-, $R_3SO_2$- oder $R_5$-Gruppe, wobei

$R_3$ ein Wasserstoffatom, eine Alkylgruppe mit 1 bis 6 Kohlenstoffatomen, eine Aryl-, Heteroaryl-, Aralkyl-, Carboxyalkyl- oder Alkoxycarbonylalkylgruppe,

$R_4$ ein Wasserstoffatom, eine Alkyl- oder Alkoxygruppe mit jeweils 1 bis 6 Kohlenstoffatomen, eine Aryl-, Heteroaryl- oder Aralkylgruppe mit 1 bis 6 Kohlenstoffatomen im Alkylteil und

$R_5$ eine Azetidino-, Pyrrolidino-, Hexamethylenimino- oder Heptamethyleniminogruppe oder eine Piperidinogruppe, in der die Methylengruppe in 4-Stellung durch ein Sauerstoffatom, eine Sulfenyl-, Sulfinyl- oder Sulfonylgruppe oder durch eine durch eine $R_3$-, $R_4CO$-, Alkylsulfonyl- oder Arylsulfonylgruppe substituierte Iminogruppe ersetzt sein kann, wobei $R_3$ und $R_4$ wie vorstehend erwähnt definiert sind, darstellen,

X ein Sauerstoff-, Schwefel- oder Stickstoffatom oder eine -$NR_2$-Gruppe, wobei

$R_2$ ein Wasserstoffatom, eine geradkettige oder verzweigte Alkylgruppe mit 1 bis 15 Kohlenstoffatomen, eine geradkettige oder verzweigte Alkenyl- oder Alkinylgruppe mit jeweils 3 bis 10 Kohlenstoffatomen, wobei die Doppel- oder Dreifachbindung nicht unmittelbar an das Stickstoffatom anschließen kann, eine Cycloalkyl- oder Cycloalkylalkylgruppe mit jeweils 3 bis 7 Kohlenstoffatomen im Cycloalkylteil, eine Aryl- oder Heteroarylgruppe, eine Alkylgruppe mit 2 bis 6 Kohlenstoffatomen, die ab der $\beta$-Position zu dem Stickstoffatom der -$NR_2$-Gruppe durch eine $R_3O$-, $(R_3)_2N$-, $R_4CO-NR_3$-, Alkylsulfonyl-$NR_3$-, Arylsulfonyl-$NR_3$-, Alkylsulfenyl-, Alkylsulfinyl-, Alkylsulfonyl- oder $R_5$-Gruppe substituiert ist, oder eine Alkylgruppe mit 1 bis 6 Kohlenstoffatomen, die durch eine oder zwei Arylgruppen, durch eine Heteroaryl-, $R_6OCO$-, $(R_3)_2NCO$-, $R_5CO$-, $R_3O$-CO-alkylen-$NR_3$-CO-, $(R_3)_2N$-CO-alkylen-$NR_3$-CO- oder $R_5CO$-alkylen-$NR_3$-CO-gruppe substituiert ist, in denen $R_3$ und $R_5$ wie vorstehend definiert sind und $R_6$ ein Wasserstoffatom, eine Alkylgruppe mit 1 bis 6 Kohlenstoffatomen, eine Cycloalkylgruppe mit 5 bis 7 Kohlenstoffatomen oder eine Aralkylgruppe darstellt,

Y eine NO-Gruppe, ein Stickstoffatom oder eine gegebenenfalls durch eine Alkylgruppe substituierte Methingruppe,

$Z_1$, $Z_2$, $Z_3$ und $Z_4$, die gleich oder verschieden sein können, Methingruppen, Kohlenstoffatome, Iminogruppen oder Stickstoffatome, wobei mindestens einer der Reste $Z_1$ bis $Z_4$ ein Kohlenstoffatom enthalten muß und eine oder zwei zu einem Stickstoffatom benachbarte Methingruppen jeweils durch Carbonylgruppen ersetzt sein können,

$Z_5$ und $Z_6$ jeweils ein Kohlenstoffatom oder auch einer der Reste $Z_5$ oder $Z_6$ ein Stickstoffatom und der andere der Reste $Z_5$ oder $Z_6$ ein Kohlenstoffatom,

A eine Cyanogruppe, eine Aminogruppe, eine geradkettige oder verzweigte Aminoalkylgruppe mit 1 bis 4 Kohlenstoffatomen, eine Amidino-, Guanidino- oder Guanidinoalkylgruppe, wobei in den vorstehend erwähnten Amino-, Aminoalkyl-, Amidino-, Guanidino- oder Guanidinoalkylgruppen jeweils an einem der Stickstoffatome ein oder zwei Wasserstoffatome durch eine oder zwei Alkylgruppen mit jeweils 1 bis 4 Kohlenstoffatomen oder ein Wasserstoffatom durch eine Alkoxycarbonylgruppe mit insgesamt 2 bis 5 Kohlenstoffatomen, durch eine Alkenyloxycarbonylgruppe mit insgesamt 4 bis 6 Kohlenstoffatomen, durch eine Alkylcarbonylgruppe mit insgesamt 2 bis 5 Kohlenstoffatomen, durch eine Arylcarbonyl-, Aryloxycarbonyl-, Aralkoxycarbonyl-, Alkanoyloxymethoxycarbonyl-,

Cycloalkanoyloxymethoxycarbonyl-, Aralkanoyloxymethoxycarbonyl-, Aroyloxymethoxycarbonyl-, Phosphono-, Dialkylphosphoryl- oder O-Alkyl-phosphonogruppe ersetzt sein kann, in denen jeweils die Alkanoylteile insgesamt 2 bis 7 Kohlenstoffatome und die Cycloalkanoylteile insgesamt 4 bis 8 Kohlenstoffatome enthalten sowie der Methoxyteil jeweils durch eine Cycloalkylgruppe mit 3 bis 6 Kohlenstoffatomen, durch eine Aralkyl-, Aryl- oder Alkylgruppe oder durch zwei Alkylgruppen, die zusammen mit dem Methylenkohlenstoffatom auch einen 5- oder 6-gliedrigen Ring bilden können, substituiert sein kann, oder, falls B ein cyclisches Imin mit 4 bis 7 Ringgliedern darstellt, auch ein Wasserstoffatom oder eine Alkylgruppe, die jeweils an den Iminostickstoff gebunden sind,

B eine Phenylengruppe, die durch Fluor-, Chlor- oder Bromatome, durch Alkyl-, Hydroxy-, Alkoxy-, Alkylsulfenyl-, Alkylsulfinyl-, Alkylsulfonyl-, Nitro-, Amino-, Alkylamino-, Dialkylamino-, Alkylcarbonylamino-, Alkylsulfonylamino- oder Trifluormethylgruppen mono- oder disubstituiert sein kann, wobei die Substituenten gleich oder verschieden sein können und gleichzeitig eine oder zwei Methingruppen in den vorstehend erwähnten Phenylengruppen jeweils durch ein oder zwei Stickstoffatome ersetzt sein können, oder eine Cycloalkylengruppe mit 3 bis 7 Kohlenstoffatomen, wobei in einem 4- oder 5-gliedrigen Cycloalkylenring ein Ringglied ein Stickstoffatom und in einem 6- oder 7-gliedrigen Cycloalkylenring ein oder zwei Ringglieder jeweils ein Stickstoffatom darstellen können und gleichzeitig die Verbindung mit Kohlenstoffatomen der benachbarten Reste über die gegebenenfalls vorhandenen Stickstoffatome erfolgen kann,

eine Indanylen- oder 1,2,3,4-Tetrahydronaphthylengruppe, in denen jeweils der gesättigte Ring an den Rest A und der aromatische Ring an den Rest C oder an den kondensierten 5-gliedrigen Heterocyclus gebunden ist,

C eine Bindung, eine Alkylen-, Arylen-, -O-alkylen-, -S-alkylen-, -NH-alkylen-, -N(Alkyl)-alkylen-, -Alkylen-NH-, -Alkylen-N(Alkyl)-, -SO-alkylen- oder $-SO_2$-alkylengruppe,

D eine Bindung oder eine Alkylengruppe,

E eine Alkylengruppe mit 1 bis 7 Kohlenstoffatomen, eine Alkenylen- oder Alkinylengruppe mit jeweils 2 bis 7 Kohlenstoffatomen, wobei die Doppel- oder Dreifachbindung nicht direkt an ein Stickstoffatom der $-Z_1-Z_2-Z_3-Z_4$-Gruppe gebunden sein kann, oder, sofern E nicht unmittelbar an ein Stickstoffatom der $-Z_1-Z_2-Z_3-Z_4$-Gruppe gebunden ist, eine -O-, -S-, -SO-, $-SO_2-$, $-NR_3-$, $-N(COR_4)-$, -CO-, $-NR_3-CO-$, $-CO-NR_3-$, $-SO_2-NR_3-$, Alkylsulfonylimino- oder Arylsulfonyliminogruppe, wobei $R_3$ und $R_4$ wie vorstehend erwähnt definiert ist, oder eine Cycloalkylengruppe mit 4 bis 7 Kohlenstoffatomen, wobei in einem 4- oder 5-gliedrigen Cycloalkylenring ein Ringglied ein Stickstoffatom und in einem 6- oder 7-gliedrigen Cycloalkylenring ein oder zwei Ringglieder jeweils ein Stickstoffatom darstellen können und zusätzlich eine zu einem Stickstoffatom benachbarte Methylengruppe durch eine Carbonylgruppe ersetzt sein kann, wobei gleichzeitig die Verbindung mit Kohlenstoffatomen der benachbarten Reste über die gegebenenfalls vorhandenen Stickstoffatome erfolgen kann,

F eine Bindung, eine geradkettige oder verzweigte Alkylengruppe mit 1 bis 6 Kohlenstoffatomen,

eine geradkettige oder verzweigte Alkenylen- oder Alkinylengruppe mit jeweils 2 bis 6 Kohlenstoffatomen, wobei die Doppel- oder Dreifachbindung nicht unmittelbar an ein Heteroatom oder eine Dreifachbindung des Restes E anschließen kann, und die vorstehend erwähnten Alkylen-, Alkenylen- und Alkinylengruppen jeweils durch eine Aryl-, $-COOR_6-$, $-CON(R_3)_2-$ oder $-CO-N(R_3)$-alkyl-Gruppe substituiert sein können, wobei die Reste $R_3$ und $R_6$ wie vorstehend erwähnt definiert sind und der Alkylteil der $-CO-N(R_3)$-alkyl-Gruppe, der 1 bis 6 Kohlenstoffatome enthalten kann, zusätzlich durch die Reste $R_7$ und $R_8$ substituiert sein kann, wobei $R_7$ und $R_8$, die gleich oder verschieden sein können, jeweils ein Wasserstoffatom, eine Aryl- oder eine $-COOR_6$-gruppe darstellen, wobei $R_6$ wie vorstehend erwähnt definiert ist,

eine Cycloalkylen-, Alkylen-cycloalkylen- oder Cycloalkylenalkylen-gruppe mit jeweils 4 bis 6 Kohlenstoffatomen im Cycloalkylenteil, in denen jeweils eine im Ring befindliche CH-Gruppe durch ein Stickstoffatom ersetzt sein und die Verknüpfung zum benachbarten Rest E jeweils auch über das Stickstoffatom erfolgen kann, sofern die Bindung über ein Kohlenstoffatom des Restes E erfolgt, wobei, falls D eine Bindung, E ein Sauerstoffatom und F eine Alkylgruppe darstellen, A keine direkt an einen Phenylring gebundene Amino- oder Acylaminogruppe sein kann, und, für den Fall, daß gleichzeitig noch X ein Schwefelatom und Y ein Stickstoffatom darstellt, die Gruppe A-B-C keine 4-Acetamino-piperazinogruppe sein kann, und

G eine nicht an ein Heteroatom des Restes E gebundene Carbonylgruppe, die durch eine Hydroxygruppe, durch eine Arylalkenyloxygruppe mit 3 bis 4 Kohlenstoffatomen im Alkenylteil, durch eine

Alkoxygruppe mit 1 bis 8 Kohlenstoffatomen, in der ein Alkoxyrest mit 1 bis 5 Kohlenstoffatomen durch eine Arylgruppe oder ein Alkoxyrest mit 1 bis 3 Kohlenstoffatomen in 1-, 2- oder 3-Stellung durch eine Heteroaryl- oder Cycloalkylgruppe mit 4 bis 8 Kohlenstoffatomen oder in 2- oder 3-Stellung durch eine Pyrrolidin-2-on-1-yl-, Morpholino-, Thiomorpholino- oder 1-Oxido-thiomorpholinogruppe substituiert sein kann, durch eine gegebenenfalls durch 1 bis 3 Alkylgruppen substituierte Cycloalkoxygruppe mit 4 bis 8 Kohlenstoffatomen, durch eine gegebenenfalls durch 1 bis 3 Methylgruppen substituierte Benzocycloalkoxy-, Benzocycloalkylalkoxy-, Bicycloalkoxy- oder Bicycloalkylalkoxygruppe mit jeweils 4 bis 8 Kohlenstoffatomen im Cycloalkylteil und 6 bis 8 Kohlenstoffatomen im Bicycloalkylteil, durch eine Alkanoyloxymethoxygruppe mit insgesamt 2 bis 7 Kohlenstoffatomen im Alkanoylteil, durch eine Cycloalkanoyloxymethoxygruppe mit insgesamt 4 bis 8 Kohlenstoffatomen im Cycloalkanoylteil, durch eine Alkoxycarbonyloxymethoxygruppe mit 1 bis 6 Kohlenstoffatomen im Alkylteil, durch eine Cycloalkoxycarbonyloxymethoxygruppe mit 3 bis 7 Kohlenstoffatomen im Cycloalkylteil, durch eine Aroyloxymethoxy-, Aralkanoyloxymethoxy-, Aryloxycarbonyloxymethoxy- oder Aralkoxycarbonyloxymethoxygruppe, in denen der Methoxyteil jeweils durch eine Alkylgruppe mit 1 bis 6 Kohlenstoffatomen, durch eine Cycloalkylgruppe mit 3 bis 7 Kohlenstoffatomen, durch eine Aralkyl- oder Arylgruppe substituiert sein kann, substituiert ist, eine Sulfo-, Phosphono-, O-Alkylphosphono- oder Tetrazol-5-yl-gruppe darstellen, bedeuten,

wobei, soweit nichts anderes erwähnt wurde,

die vorstehend erwähnten Alkyl-, Alkylen- oder Alkoxyteile jeweils 1 bis 3 Kohlenstoffatome enthalten können, sowie unter "eine Arylgruppe" eine gegebenenfalls durch Fluor-, Chlor-, Brom- oder Jodatome, durch Alkyl-, Trifluormethyl-, Nitro-, Amino-, Alkylamino-, Dialkylamino-, Alkanoylamino-, Alkylsulfonylamino-, Aminosulfonyl-, Alkylaminosulfonyl-, Dialkylaminosulfonyl-, Hydroxy-, Alkoxy-, Aralkoxy-, Carboxy-, Alkoxycarbonyl-, Aminocarbonyl-, Alkylaminocarbonyl-, Dialkylaminocarbonyl-, Alkylsulfenyl-, Alkylsulfinyl- oder Alkylsulfonylgruppen mono-, di- oder trisubstituierte Phenylgruppe, wobei die Substituenten gleich oder verschieden sein können, oder eine Naphthylgruppe und

unter "eine Heteroarylgruppe" ein 5-gliedriger heteroaromatischer Ring, der eine Iminogruppe, ein Sauerstoff- oder Schwefelatom, ein bis zwei Stickstoffatome und ein Sauerstoff- oder Schwefelatom oder eine Iminogruppe und ein bis drei Stickstoffatome enthält, oder ein 6-gliedriger heteroaromatischer Ring, der 1, 2 oder 3 Stickstoffatome enthält, wobei an die vorstehend erwähnten Ringe ein Phenylring ankondensiert sein kann und zusätzlich die vorstehend erwähnten Ringe durch ein Fluor-, Chlor- oder Bromatom, durch eine Alkyl-, Alkoxy-, Hydroxy-, Amino-, Dialkylamino-, Alkylcarbonylamino-, Alkylsulfonylamino- oder Trifluormethylgruppe oder durch eine Alkylaminogruppe mit 1 bis 4 Kohlenstoffatomen mono- oder disubstituiert sein können, zu verstehen sind,

deren Tautomere, deren Stereoisomere, deren Gemische und deren Salze.

**2.** Kondensierte 5-gliedrige Heterocyclen der allgemeinen Formel I, in der

$R_1$ ein Wasserstoff-, Fluor-, Chlor- oder Bromatom, eine Alkoxycarbonylamino- oder N-Alkoxycarbonylalkylaminogruppe mit jeweils 1 bis 4 Kohlenstoffatomen im Alkoxyteil, eine Alkyl-, Hydroxy-, Alkoxy-, Phenylalkoxy-, Amino-, Alkylamino-, Dialkylamino-, Pyrrolidino-, Piperidino-, Alkylcarbonylamino- oder Alkylsulfonylaminogruppe, wobei soweit nichts anderes erwähnt wurde, der Alkyl- und Alkoxyteil jeweils 1 bis 3 Kohlenstoffatome enthalten kann, und zusätzlich die Alkylteile der Alkylamino- und Dialkylaminogruppen durch eine Carboxy- oder Alkoxycarbonylgruppe mit 1 bis 3 Kohlenstoffatomen im Alkylteil substituiert sein können,

X ein Sauerstoff-, Schwefel- oder Stickstoffatom oder eine $-NR_2$-Gruppe, wobei

$R_2$ ein Wasserstoffatom, eine geradkettige oder verzweigte Alkylgruppe mit 1 bis 14 Kohlenstoffatomen, eine Alkenyl- oder Alkinylgruppe mit jeweils 3 oder 4 Kohlenstoffatomen, wobei die Doppel- oder Dreifachbindung nicht unmittelbar an das Stickstoffatom anschließen kann,
eine Alkylgruppe mit 1 bis 5 Kohlenstoffatomen, die durch eine Carboxy-, Alkoxycarbonyl-, Aminocarbonyl-, Alkylaminocarbonyl-, Dialkylaminocarbonyl-, Phenylalkylaminocarbonyl-, Pyrrolidinocarbonyl-, Carboxyalkylaminocarbonyl-, Alkoxycarbonylalkylaminocarbonyl-, 2- oder 4-Imidazolyl- oder Pyridylgruppe, durch eine Piperidinocarbonylgruppe, in der die Methylengruppe in 4-Stellung durch eine Sauerstoffatom, durch eine Sulfenyl-, Sulfinyl-, Sulfonyl-, Imino-, Alkylimino- oder Phenylalkyliminogruppe ersetzt sein kann, oder durch eine gegebenenfalls durch Chlor- oder Bromatome, durch Amino-, Hydroxy-, Alkoxy- oder Alkylgruppen mono- oder disubstituierte Phenylgruppe oder durch zwei Phenylgruppen substituiert ist, eine Cycloalkylgruppe mit 3 bis 7 Kohlenstoffatomen, eine Pyridylgruppe, eine gegebenenfalls durch Fluor-, Chlor- oder Bromatome, durch Alkyl-, Alkoxy-, Alkylsulfenyl-, Alkylsulfinyl- oder Alkylsulfonylgruppen mono- oder disubstituierte Phenylgruppe, wobei die Substiuenten gleich oder verschieden sein können,

eine Alkylgruppe mit 2 bis 4 Kohlenstoffatomen, die in 2-, 3- oder 4-Stellung durch eine Hydroxy-, Alkoxy-, Alkylsulfenyl-, Alkylsulfinyl-, Alklylsulfonyl-, 1-Imidazolyl- oder Pyrrolidinogruppe oder durch eine Piperidinogruppe, in der die Methylengruppe in 4-Stellung durch ein Sauerstoffatom, eine Sulfenyl-, Sulfinyl-, Sulfonyl-, Imino-, Alkylimino- oder Phenylalkyliminogruppe ersetzt sein kann, substituiert ist, oder

eine Alkylgruppe mit 2 bis 6 Kohlenstoffatomen, die in 2-, 3-, 4-, 5- oder 6-Stellung durch eine Amino-, Alkylamino- oder Dialkylaminogruppe substituiert ist, wobei, soweit nichts anderes erwähnt wurde, die vorstehend erwähnten Alkyl- und Alkoxyteile jeweils 1 bis 3 Kohlenstoffatome enthalten können,

Y eine NO-Gruppe, ein Stickstoffatom oder eine gegebenenfalls durch eine Alkylgruppe mit 1 bis 3 Kohlenstoffatomen substituierte Methingruppe,

$Z_1$, $Z_2$, $Z_3$ und $Z_4$, die gleich oder verschieden sein können, Methingruppen, Kohlenstoffatome, Iminogruppen oder Stickstoffatome, wobei mindestens einer der Reste $Z_1$ bis $Z_4$ ein Kohlenstoffatom enthalten muß und eine oder zwei zu einem Stickstoffatom benachbarte Methingruppen jeweils durch Carbonylgruppen ersetzt sein können,

$Z_5$ und $Z_6$ jeweils ein Kohlenstoffatom oder auch einer der Reste $Z_5$ oder $Z_6$ ein Stickstoffatom und der andere der Reste $Z_5$ oder $Z_6$ ein Kohlenstoffatom,

A eine Cyanogruppe, eine Aminogruppe, eine geradkettige oder verzweigte Aminoalkylgruppe mit 1 bis 4 Kohlenstoffatomen, eine Amidino-, Guanidino- oder Guanidinoalkylgruppe, wobei in den vorstehend erwähnten Amino-, Aminoalkyl-, Amidino-, Guanidino- oder Guanidinoalkylgruppen jeweils an einem der Stickstoffatome ein Wasserstoffatom durch eine Alkylgruppe mit jeweils 1 bis 4 Kohlenstoffatomen, durch eine Alkoxycarbonylgruppe mit insgesamt 2 bis 5 Kohlenstoffatomen, durch eine Phenylalkoxycarbonyl-, Phenyloxycarbonyloxy- oder Benzoylgruppe oder durch eine Alkanoyloxymethoxycarbonylgruppe, in der der Alkanoylteil insgesamt 2 bis 4 Kohlenstoffatome enthalten kann, oder durch eine Phosphono-, Dialkylphosphoryl- oder O-Alkyl-phosphonogruppe ersetzt sein kann, oder, falls B ein cyclisches Imin darstellt, auch ein Wasserstoffatom oder eine Alkylgruppe mit 1 bis 3 Kohlenstoffatomen, die jeweils an den Iminostickstoff gebunden sind,

B eine Phenylengruppe, die durch Fluor-, Chlor- oder Bromatome, durch Alkyl-, Hydroxy-, Alkoxy-, Alkylsulfenyl-, Alkylsulfinyl-, Alkylsulfonyl-, Nitro-, Amino-, Alkylamino-, Dialkylamino-, Alkylcarbonylamino-, Alkylsulfonylamino- oder Trifluormethylgruppen mono- oder disubstituiert sein kann, wobei die Substituenten gleich oder verschieden sein können sowie die Alkyl- und Alkoxyteile jeweils 1 bis 3 Kohlenstoffatome enthalten können, eine Pyridinylen- oder Cycloalkylengruppe mit 3 bis 6 Kohlenstoffatomen, eine Piperidinylen- oder Piperazinylengruppe, die auch über die Stickstoffatome an Kohlenstoffatome der benachbarten Reste gebunden sein können,

eine Indanylen- oder 1,2,3,4-Tetrahydronaphthylengruppe, in denen jeweils der gesättigte Ring an den Rest A und der aromatische Ring an den Rest C oder den kondensierten 5-gliedrigen Heterocyclus gebunden ist,

C eine Bindung, eine Methylen-, Ethylen-, Phenylen-, -O-methylen-, -S-methylen-, -SO-methylen-, $-SO_2$-methylen- oder -N(Alkyl)-methylen-gruppe, die jeweils mit dem Heteroatom an den Rest B gebunden sind, oder eine -Methylen-N(Alkyl)-gruppe, wobei der Alkylteil jeweils 1 bis 3 Kohlenstoffatome enthalten kann,

D eine Bindung, eine Methylen- oder Ethylengruppe,

E eine Alkylengruppe mit 1 bis 5 Kohlenstoffatomen, eine Alkenylen- oder Alkinylengruppe mit jeweils 3 bis 5 Kohlenstoffatomen, wobei die Doppel- oder Dreifachbindung nicht direkt an ein Stickstoffatom der $-Z_1-Z_2-Z_3-Z_4$-Gruppe gebunden sein kann, oder, sofern E nicht unmittelbar an ein Stickstoffatom der $-Z_1-Z_2-Z_3-Z_4$-Gruppe gebunden ist, eine -O-, -S-, -SO-, $-SO_2-$, -NH-, -N(Alkyl)-, -N(COAlkyl)-, -CO-, -NH-CO-, -N(Alkyl)-CO-, -CO-NH-, -CO-N(Alkyl)-, $-SO_2-NH-$, $-SO_2-N(Alkyl)-$ oder

Alkylsulfonyliminogruppe, wobei der Alkylteil jeweils 1 bis 3 Kohlenstoffatome enthalten kann, eine Cyclohexylengruppe, eine Piperidinylen- oder Piperazinylengruppe, in denen zusätzlich eine zu einem Stickstoffatom benachbarte Methylengruppe durch eine Carbonylgruppe ersetzt sein kann, wobei gleichzeitig die Verbindung mit Kohlenstoffatomen der benachbarten Reste über die Stickstoffatome erfolgen kann,

F eine Bindung, eine Alkylengruppe mit 1 bis 4 Kohlenstoffatomen, die durch eine Phenyl-, Carboxy-, Alkoxycarbonyl-, Phenylalkoxycarbonyl- oder Alkylaminocarbonylgruppe substituiert sein kann, wobei die Alkylaminocarbonylgruppe im Alkylteil, der 1 bis 5 Kohlenstoffatome enthalten kann, durch eine Phenyl-, Hydroxyphenyl-, Methoxyphenyl-, Benzyloxyphenyl-, Carboxy-, Alkoxycarbonyl- oder Phenylalkoxycarbonylgruppe oder zusätzlich durch eine weitere Carboxy-, Alkoxycarbonyl- oder Phenylalkoxycarbonylgruppe substituiert sein kann, eine Cyclohexylen-, Cyclohexylen-alkylen- oder Alkylen-cyclohexylengruppe mit jeweils 1 bis 3 Kohlenstoffatomen in den Alkyl-, Alkylen- und Alkoxyteilen, eine Piperidinylen- oder Pyrrolidinylengruppe, die jeweils auch über das Stickstoffatom an den benachbarten Rest E gebunden sein können, sofern die Bindung über einen Kohlenstoffatom des Restes E erfolgt, wobei, falls D eine Bindung, E ein Sauerstoffatom und F eine Alkylgruppe darstellen, A keine direkt an einen Phenylring gebundene Amino- oder Acylaminogruppe sein kann, und

G eine nicht an ein Heteroatom des Restes E gebundene Carbonylgruppe, die durch eine Hydroxygruppe, durch eine Cinnamyloxygruppe, durch eine Alkoxygruppe mit 1 bis 6 Kohlenstoffatomen, in der ein Alkoxyrest mit 1 bis 5 Kohlenstoffatomen durch eine Phenylgruppe oder ein Alkoxyrest mit 1 bis 3 Kohlenstoffatomen in 1-, 2- oder 3-Stellung durch eine Naphthylgruppe, durch eine Cycloalkylgruppe mit 5 bis 6 Kohlenstoffatomen, durch eine Pyridylgruppe oder in 2- oder 3-Stellung durch eine Pyrrolidin-2-on-1-yl-gruppe substituiert sein kann, durch eine Cycloalkoxygruppe mit 5 bis 8 Kohlenstoffatomen, durch eine Indanyloxy-, 1,2,3,4-Tetrahydronaphthyloxy-, Bicycloheptyloxy- oder Bicycloheptylmethoxygruppe, wobei die Bicycloheptylgruppe jeweils durch 1 bis 3 Methylgruppen substituiert sein kann, durch eine Alkanoyloxymethoxygruppe mit insgesamt 2 bis 5 Kohlenstoffatomen im Alkanoylteil, durch eine Alkoxycarbonyloxymethoxygruppe mit 1 bis 2 Kohlenstoffatomen im Alkylteil oder durch eine Cycloalkoxycarbonyloxymethoxygruppe mit 5 oder 6 Kohlenstoffatomen im Cycloalkylteil, in denen der Methoxyteil jeweils durch eine Alkylgruppe substituiert sein kann, substituiert ist, eine O-Alkylphosphonogruppe mit 1 bis 3 Kohlenstoffatomen, eine Sulfo-, Phosphono- oder Tetrazol-5-ylgruppe darstellen,

deren Tautomere, deren Stereoisomere, deren Gemische und deren Salze.

**3.** Kondensierte 5-gliedrige Heterocyclen der allgemeinen Formel I, in der

$R_1$ ein Wasserstoff-, Fluor-, Chlor- oder Bromatom, eine Alkoxy- oder Phenylalkoxygruppe mit jeweils 1 bis 3 Kohlenstoffatomen im Alkoxyteil, eine Alkoxycarbonylamino- oder N-Alkoxycarbonyl-methylaminogruppe mit jeweils 1 bis 4 Kohlenstoffatomen im Alkoxyteil, eine Methyl-, Hydroxy-, Amino-, Methylamino-, Dimethylamino-, N-Carboxymethyl-amino-, N-Carboxymethyl-methylamino-, N-Methoxycarbonylmethyl-methylamino-, Acetylamino-, Piperidino- oder Methylsulfonylaminogruppe,

X ein Sauerstoff-, Schwefel- oder Stickstoffatom oder eine $-NR_2$-Gruppe, wobei

$R_2$ ein Wasserstoffatom, eine geradkettige oder verzweigte Alkylgruppe mit 1 bis 14 Kohlenstoffatomen, eine Allyl- oder Propargylgruppe,

eine Alkylgruppe mit 1 bis 3 Kohlenstoffatomen, die durch eine Carboxy-, Methoxycarbonyl-, Ethoxycarbonyl-, Aminocarbonyl-, Methylaminocarbonyl-, Dimethylaminocarbonyl-, Benzylaminocarbonyl-, Phenylethylaminocarbonyl-, Carboxymethylaminocarbonyl-, Methoxycarbonylmethylaminocarbonyl- oder Pyridylgruppe oder durch eine Piperidinocarbonylgruppe, in der die Methylengruppe in 4-Stellung durch eine Sauerstoffatom, durch eine Sulfenyl-, Sulfinyl-, Sulfonyl-, Imino- oder Benzyliminogruppe ersetzt sein kann, substituiert ist,

eine Alkylgruppe mit 1 bis 4 Kohlenstoffatomen, die durch eine gegebenenfalls durch Brom- oder Chloratome, durch Amino-, Hydroxy-, Methoxy- oder Methylgruppen mono- oder disubstituierte Phenylgruppe oder durch zwei Phenylgruppen substituiert ist,

eine Cycloalkylgruppe mit 3 bis 6 Kohlenstoffatomen, eine Pyridylgruppe, eine gegebenenfalls durch Chloratome, durch Methyl-, Methoxy-, Methylsulfenyl-, Methylsulfinyl- oder Methylsulfonylgruppen mono- oder disubstituierte Phenylgruppe, wobei die Substituenten gleich oder verschieden sein

können,
eine Alkylgruppe mit 2 oder 3 Kohlenstoffatomen, die in 2- oder 3-Stellung durch eine Hydroxy-, Methoxy-, Methylsulfenyl-, Methylsulfinyl-, Methylsulfonyl-, Imidazolyl- oder Pyrrolidinogruppe oder durch eine Piperidinogruppe, in der die Methylengruppe in 4-Stellung durch ein Sauerstoffatom, durch eine Sulfenyl-, Sulfinyl-, Sulfonyl-, Imino- oder Benzyliminogruppe ersetzt sein kann, substituiert ist, oder
eine Alkylgruppe mit 2 bis 6 Kohlenstoffatomen, die in 2-, 3-, 4-, 5- oder 6-Stellung durch eine Aminogruppe substituiert ist,

Y eine NO-Gruppe, ein Stickstoffatom oder eine gegebenenfalls durch eine Methylgruppe substituierte Methingruppe,

$Z_1$, $Z_2$, $Z_3$ und $Z_4$, die gleich oder verschieden sein können, Methingruppen, Kohlenstoffatome, Iminogruppen oder Stickstoffatome, wobei mindestens einer der Reste $Z_1$ bis $Z_4$ ein Kohlenstoffatom enthalten muß und eine oder zwei zu einem Stickstoffatom benachbarte Methingruppen jeweils durch Carbonylgruppen ersetzt sein können,

$Z_5$ und $Z_6$ jeweils ein Kohlenstoffatom oder auch einer der Reste $Z_5$ oder $Z_6$ ein Stickstoffatom und der andere der Reste $Z_5$ oder $Z_6$ ein Kohlenstoffatom,
A eine Aminogruppe, eine geradkettige oder verzweigte Aminoalkylgruppe mit 1 bis 3 Kohlenstoffatomen, eine Amidino-, Guanidino- oder Guanidinomethylgruppe, wobei in den vorstehend erwähnten Amino-, Aminoalkyl-, Amidino-, Guanidino- oder Guanidinomethylgruppen jeweils an einem der Stickstoffatome ein Wasserstoffatom durch eine Alkylgruppe mit jeweils 1 bis 4 Kohlenstoffatomen, durch eine Alkoxycarbonylgruppe mit insgesamt 2 bis 5 Kohlenstoffatomen, durch eine Benzyloxycarbonyl-, Acetyloxymethoxycarbonyl, Dimethylphosphoryl- oder Diethylphosphorylgruppe ersetzt sein kann,
B eine Phenylengruppe, die durch ein Fluor-, Chlor- oder Bromatom, durch eine Methyl-, Hydroxy- oder Methoxygruppe substituiert sein kann, eine Pyridinylengruppe oder eine Cycloalkylengruppe mit 3 bis 6 Kohlenstoffatomen, eine Indanylen- oder 1,2,3,4-Tetrahydronaphthylengruppe, in denen jeweils der gesättigte Ring an den Rest A und der aromatische Ring an den Rest C oder den kondensierten 5-gliedrigen Heterocyclus gebunden ist, eine Piperidinylen- oder Piperazinylengruppe, die auch über die Stickstoffatome an Kohlenstoffatome der benachbarten Reste gebunden sein können,
C eine Bindung, eine Phenylen- oder eine -O-methylen-Gruppe,
D eine Bindung oder eine Methylengruppe,
E eine Alkylengruppe mit 3 bis 5 Kohlenstoffatomen, eine Alkenylengruppe mit jeweils 3 bis 5 Kohlenstoffatomen, wobei die Doppelbindung nicht direkt an ein Stickstoffatom der $-Z_1-Z_2-Z_3-Z_4-$Gruppe gebunden sein kann, oder, sofern E nicht unmittelbar an ein Stickstoffatom der $-Z_1-Z_2-Z_3-Z_4-$Gruppe gebunden ist, eine -O-, -S-, -SO-, $-SO_2-$, -NH-, -N(Methyl)-, -N(Acetyl)-, $-N(SO_2CH_3)-$, -CO-, -NH-CO-, $-N(CH_3)-CO-$, -CO-NH-, $-CO-N(CH_3)-$, $-SO_2-NH-$ oder $-SO_2-N(CH_3)-$gruppe, eine Piperidinylen-, Piperazinylen- oder Oxo-piperazinylengruppe, wobei die Stickstoffatome auch an Kohlenstoffatome der benachbarten Reste gebunden sein können,
F eine Bindung, eine Alkylengruppe mit 1 bis 4 Kohlenstoffatomen, die durch eine Phenyl-, Carboxy-, Methoxycarbonyl-, Benzyloxycarbonyl-, Phenylethylaminocarbonyl-, (Methoxyphenyl)-ethylaminocarbonyl- oder Alkylaminocarbonylgruppe, wobei die Alkylaminocarbonylgruppe im Alkylteil, der 1 bis 4 Kohlenstoffatome enthalten kann, durch eine Phenyl-, Hydroxyphenyl-, Methoxyphenyl-, Carboxy- oder Benzyloxycarbonylgruppe oder zusätzlich durch eine weitere Carboxy- oder Benzyloxycarbonylgruppen substituiert sein kann, substituiert sein kann,

Eine Piperidinylen- oder Pyrrolidinylengruppe, die jeweils auch über das Stickstoffatom an den benachbarten Rest E gebunden sein können, sofern die Bindung über ein Stickstoffatom des Restes E erfolgt,

eine Cyclohexylen- oder Cyclohexylen-methylengruppe, wobei, falls D eine Bindung, E ein Sauerstoffatom und F eine Alkylgruppe darstellen, A keine direkt an einen Phenylring gebundene Amino- oder Acylaminogruppe sein kann,

G eine nicht an ein Heteroatom des Restes E gebundene Carbonylgruppe, die durch eine Hydroxygruppe, durch eine Alkoxygruppe mit 1 bis 6 Kohlenstoffatomen, die in 1 bis 5-Stellung durch eine Phenylgruppe oder in 1- oder 2-Stellung durch eine Cyclohexyl-, Naphthyl- oder Pyridylgruppe oder in 2-Stellung durch eine Pyrrolidin-2-on-1-yl-gruppe substituiert sein kann, durch eine Cinnamyloxygruppe, durch eine Cycloalkoxygruppe mit 6 bis 8 Kohlenstoffatomen, durch eine Indanyloxy-, Norbornyloxy- oder Norbornylmethyloxygruppe, durch eine Alkanoyloxymethoxygruppe mit insgesamt 2 bis 5 Kohlenstoffatomen im Alkanoylteil, durch eine Alkoxycarbonyloxymethoxygruppe mit 1 bis 3 Kohlenstoffatomen im Alkoxyteil oder durch eine Cyclohexyloxycarbonyloxymethoxygruppe, in denen der Methoxyteil jeweils durch eine Methylgruppe substituiert sein kann, substituiert ist, eine Sulfo-, Phosphono-, O-Methyl-phosphono- oder Tetrazol-5-ylgruppe darstellen,

deren Tautomere, deren Stereoisomere, deren Gemische und deren Salze.

**4.** Kondensierte 5-gliedrige Heterocyclen der allgemeinen Formel I, in der

$R_1$ ein Wasserstoff-, eine Methyl-, Methoxy-, Methylamino-, Dimethylamino-, N-Butyloxycarbonyl-methylamino-, N-Isobutyloxycarbonyl-methylamino-, N-Carboxymethyl-amino-, N-Carboxymethyl-methylamino-, N-Methoxycarbonylmethyl-amino- oder N-Methoxycarbonylmethyl-methylaminogruppe,

X ein Sauerstoff- oder Stickstoffatom oder eine $-NR_2$-Gruppe, wobei

$R_2$ ein Wasserstoffatom, eine geradkettige oder verzweigte Alkylgruppe mit 1 bis 14 Kohlenstoffatomen,
eine Alkylgruppe mit 1 bis 3 Kohlenstoffatomen, die durch eine Carboxy- oder Methoxycarbonylgruppe substituiert ist,
eine Alkylgruppe mit 1 bis 4 Kohlenstoffatomen, die durch eine gegebenenfalls durch Bromatome, Amino- oder Methoxygruppen mono- oder disubstituierte Phenylgruppe durch zwei Phenylgruppen oder durch eine Pyridylgruppe substituiert ist,
eine Alkylgruppe mit 2 oder 3 Kohlenstoffatomen, die in 2- oder 3-Stellung durch eine Amino- oder Piperidinogruppe, in der die Methylengruppe in 4-Stellung durch eine Imino-, Benzylimino-, Sulfenyl-, Sulfinyl- oder Sulfonylgruppe ersetzt ist, substituiert ist,

Y eine NO-Gruppe, ein Stickstoffatom oder eine Methingruppe,

$Z_1$, $Z_2$, $Z_3$, und $Z_4$, die gleich oder verschieden sein können, Methingruppen, Kohlenstoffatome, Iminogruppen oder Stickstoffatome, wobei mindestens zwei der Reste $Z_1$ bis $Z_4$ ein Kohlenstoffatom enthalten müssen und eine oder zwei zu einem Stickstoffatom benachbarte Methingruppen jeweils durch Carbonylgruppen ersetzt sein können,

$Z_5$ und $Z_6$ jeweils ein Kohlenstoffatom oder auch einer der Reste $Z_5$ oder $Z_6$ ein Stickstoffatom und der andere der Reste $Z_5$ oder $Z_6$ ein Kohlenstoffatom,
A eine Aminomethyl- oder Amidinogruppe, in denen jeweils an einem der Stickstoffatome ein Wasserstoffatom durch eine Methoxycarbonylgruppe ersetzt sein kann,
B eine Phenylengruppe,
C eine Bindung oder eine -O-methylen-Gruppe,
D eine Bindung,
E eine Alkylengruppe mit 3 oder 4 Kohlenstoffatomen oder, sofern E nicht unmittelbar an ein Stickstoffatom der $-Z_1-Z_2-Z_3-Z_4$-Gruppe gebunden ist, eine -O-, -NH-CO- oder -CO-NH-gruppe,
F eine Bindung oder eine Alkylengruppe mit 1 bis 3 Kohlenstoffatomen und
G eine Carboxygruppe, eine Alkoxycarbonylgruppe mit insgesamt 2 bis 5 Kohlenstoffatomen oder eine Cyclohexyloxygruppe darstellen,

deren Tautomere, deren Stereoisomere, deren Gemische und deren Salze.

**5.** Folgende kondensierte 5-gliedrige Heterocyclen der allgemeinen Formel I:
(a) 2-(4-Amidino-phenyl)-5-[(2-carboxy-ethyl)-aminocarbonyl]-1-methyl-benzimidazol,
(b) 2-(4-Amidino-phenyl)-1-benzyl-5-[(2-carboxy-ethyl)-aminocarbonyl]-benzimidazol,

(c) 2-(4-Amidino-phenyl)-5-[(2-carboxy-ethyl)-aminocarbonyl]-1-(3-thiomorpholino-propyl)-benzimidazol,
(d) 2-(4-Amidino-phenyl)-5-[(2-carboxy-ethyl)-aminocarbonyl]-1-[3-(S-oxido-thiomorpholino)-propyl]-benzimidazol,
(e) 2-(4-Amidino-phenyl)-5-[(2-carboxy-ethyl)-aminocarbonyl]-1-[3-(S,S-dioxido-thiomorpholino)-propyl]-benzimidazol,
(f) 2-(4-Amidino-phenyl)-1-(3-amino-propyl)-5-[(2-carboxyethyl)-aminocarbonyl]-benzimidazol,
(g) 2-(4-Amidino-phenyl)-5-[(2-carboxy-ethyl)-aminocarbonyl]-1-methyl-benzimidazol-3-oxid,
(h) 2-(4-Amidino-phenyl)-1-[2-(4-benzyl-piperazino)-ethyl]-5-[(2-carboxy-ethyl)-aminocarbonyl]-benzimidazol,
(i) 2-(4-Amidino-phenyl)-5-[(2-carboxy-ethyl)-aminocarbonyl]-1-methyl-6-methylamino-benzimidazol,
(j) 2-(4-Amidino-phenyl)-5-[(2-carboxy-ethyl)-aminocarbonyl]-6-(N-carboxymethyl-methylamino)-1-methyl-benzimidazol,
(k) 2-(4-Amidino-phenyl)-5-[(2-carboxy-ethyl)-aminocarbonyl]-1-(2-piperazino-ethyl)-benzimidazol,
(1) 2-(4-Amidino-phenyl)-5-[(2-carboxy-ethyl)-aminocarbonyl]-1-[2-(3,4-dimethoxy-phenyl)-ethyl]-benzimidazol,
(m) 2-(4-Amidino-phenyl)-5-[(2-carboxy-ethyl)-aminocarbonyl]-1-(3-pyridylmethyl)-benzimidazol,
(n) 2-(4-Aminomethyl-phenyl)-5-[(2-carboxy-ethyl)-aminocarbonyl]-1-[2-(3,4-dimethoxy-phenyl)-ethyl]-benzimidazol,
(o) 2-(4-Amidino-phenyl)-5-[(2-carboxy-ethyl)-aminocarbonyl]-1-(4-phenyl-butyl)-benzimidazol,
(p) 2-(4-Amidino-phenyl)-1-[2-(4-amino-3,5-dibrom-phenyl)-ethyl]-5-[(2-carboxy-ethyl)-aminocarbonyl]-benzimidazol,
(q) 2-(4-Amidino-phenyl)-5-[(2-methoxycarbonyl-ethyl)-aminocarbonyl]-1-methyl-benzimidazol,
(r) 2-(4-Amidino-phenyl)-5-[(2-methoxycarbonyl-ethyl)-aminocarbonyl]-1-(3-thiomorpholino-propyl)-benzimidazol,
(s) 2-(4-Amidino-phenyl)-5-[(2-methoxycarbonyl-ethyl)-aminocarbonyl]-1-(4-phenyl-butyl)-benzimidazol,
(t) 2-(4-Amidino-phenyl)-1-[2-(4-amino-3,5-dibrom-phenyl)-ethyl]-5-[(2-methoxycarbonyl-ethyl)-aminocarbonyl]-benzimidazol und
(u) 2-(4-Amidino-phenyl)-1-[2-(3,4-dimethoxy-phenyl)-ethyl]-5-[(2-isopropyloxycarbonyl-ethyl)-aminocarbonyl]-benzimidazol,
(v) 8-(4-Amidino-phenyl)-1-(4-carboxy-butyl)-3,9-dimethylxanthin
(w) 2-(4-Amidino-phenyl)-6-[(2-carboxy-ethyl)-aminocarbonyl]-3-methyl-imidazo[4,5-b]pyridin

deren Tautomere, deren Stereoisomere und deren Salze.

**6.** Physiologisch verträgliche Salze der Verbindungen nach mindestens einem der Ansprüche 1 bis 5 mit anorganischen oder organischen Säuren oder Basen.

**7.** Arzneimittel, enthaltend eine Verbindung nach mindestens einem der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

**8.** Verwendung einer Verbindung nach mindestens einem der Ansprüche 1 bis 6 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

**9.** Verfahren zur Herstellung eines Arzneimittels gemäß Anspruch 7, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

**10.** Verfahren zur Herstellung der cyclischen Iminoderivate gemäß den Ansprüchen 1 bis 6, dadurch gekennzeichnet, daß

a) zur Herstellung von Verbindungen der allgemeinen Formel I, in der G eine Carboxylgruppe darstellt, eine Verbindung der allgemeinen Formel

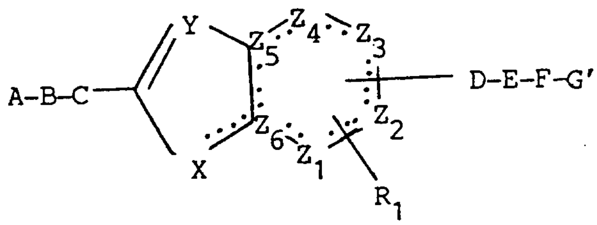

,(II)

in der
A bis F, $R_1$, $Z_1$ bis $Z_6$, X und Y wie in den Ansprüchen 1 bis 5 definiert sind und
G', das an ein Kohlenstoffatom gebunden ist, eine mittels Hydrolyse, Behandlung mit Säuren, Thermolyse oder Hydrogenolyse in eine Carboxylgruppe überführbare Gruppe darstellt, in eine Verbindung der allgemeinen Formel I, in der G eine Carboxylgruppe darstellt, übergeführt wird oder
b) zur Herstellung von Benzimidazolen der allgemeinen Formel I, eine Verbindung der allgemeinen Formel

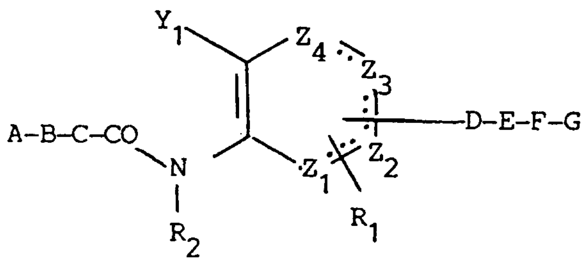

,(III)

in der
A bis G, $R_1$, $R_2$ und $Z_1$ bis $Z_4$ wie in den Ansprüchen 1 bis 5 definiert sind und
$Y_1$ eine Aminogruppe darstellt, cyclisiert wird oder
c) zur Herstellung von Verbindungen der allgemeinen Formel I, in der A eine gegebenenfalls durch eine Alkylgruppe substituierte Amidinogruppe darstellt, eine gegebenenfalls im Reaktionsgemisch gebildete Verbindung der allgemeinen Formel

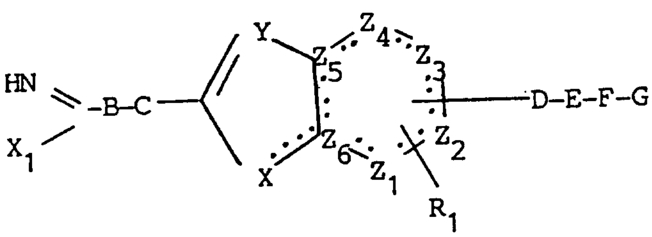

,(IV)

in der
B bis G, $R_1$, $Z_1$ bis $Z_6$, X und Y wie in den Ansprüchen 1 bis 5 definiert sind und
$X_1$ eine Alkoxy-, Aralkoxy-, Alkylthio-, Aralkylthio- oder Aminogruppe darstellt, mit einem Amin der allgemeinen Formel

$R_a$ - $NH_2$ ,(V)

in der
$R_a$ ein Wasserstoffatom oder eine Alkylgruppe mit 1 bis 4 Kohlenstoffatomen bedeutet, oder mit deren Säureadditionssalzen umgesetzt wird oder
d) zur Herstellung von Verbindungen der allgemeinen Formel I, in der A eine Aminoalkylgruppe darstellt, eine Verbindung der allgemeinen Formel

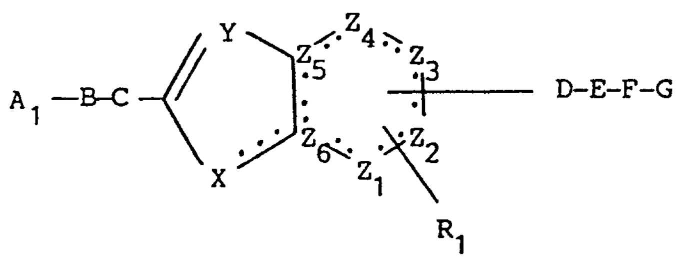

,(VI)

in der
B bis G, $R_1$, $Z_1$ bis $Z_6$, X und Y wie in den Ansprüchen 1 bis 5 definiert sind und
$A_1$ eine Cyano- oder Cyanoalkylgruppe darstellt, reduziert wird oder
e) zur Herstellung von Verbindungen der allgemeinen Formel I, die eine Sulfinylgruppe oder eine Sulfonylgruppe enthalten, die nicht mit einem Stickstoffatom verknüpft ist, eine Verbindung der allgemeinen Formel

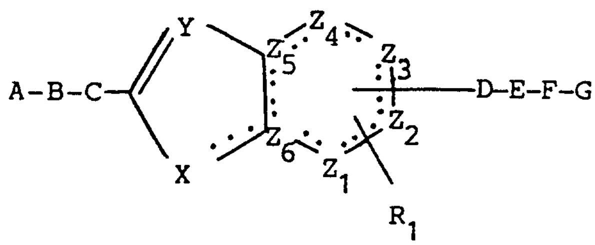

,(VII)

in der
A bis G, $R_1$, $Z_1$ bis $Z_6$, X und Y mit der Maßgabe wie in den Ansprüchen 1 bis 5 definiert sind, daß mindestens einer der Reste $R_1$, B, C, E, G oder X eine Sulfenyl- oder Sulfinylgruppe enthält, die nicht mit einem Stickstoffatom verknüpft ist, oxidiert wird oder
f) zur Herstellung von Verbindungen der allgemeinen Formel I, in der A eine durch eine Alkoxycarbonylgruppe mit insgesamt 2 bis 5 Kohlenstoffatomen, durch eine Arylmethoxycarbonyl-, Arylethoxycarbonyl-, Aryloxycarbonyl-, Alkylcarbonyl-, Aralkylcarbonyl- oder Arylcarbonylgruppe substituierte Amino-, Aminoalkyl-, Amidino-, Guanidino- oder Guanidinoalkylgruppe darstellt, eine Verbindung der allgemeinen Formel

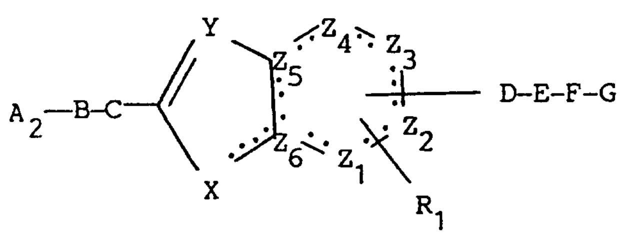

,(VIII)

in der
B bis G, $R_1$, $Z_1$ bis $Z_6$, X und Y wie in den Ansprüchen 1 bis 5 definiert sind und
$A_2$ eine Amino-, Aminoalkyl-, Amidino-, Guanidino- oder Guanidinoalkylgruppe darstellt, mit einer Verbindung der allgemeinen Formel

$X_2$ - $COR_b$ ,(IX)

in der
$R_b$ eine Alkoxygruppe mit 1 bis 4 Kohlenstoffatomen, eine Arylmethoxy-, Arylethoxy-, Aryloxy-, Alkyl-, Aralkyl- oder Arylgruppe und
$X_2$ eine nukleophile Austrittsgruppe darstellen, umgesetzt wird oder

g) zur Herstellung von Verbindungen der allgemeinen Formel I, in der G eine nicht an ein Heteroatom des Restes E gebundene Carbonylgruppe darstellt, die durch eine Cinnamyloxygruppe oder durch eine Alkoxygruppe mit 1 bis 6 Kohlenstoffatomen substituiert ist, in der ein Alkoxyrest mit 1 bis 5 Kohlenstoffatomen durch eine Phenylgruppe oder ein Alkoxyrest mit 1 bis 3 Kohlenstoffatomen in 1-, 2- oder 3-Stellung durch eine Naphthylgruppe, durch eine Cycloalkylgruppe mit 5 bis 6 Kohlenstoffatomen, durch eine Pyridylgruppe oder in 2- oder 3-Stellung durch eine Pyrrolidin-2-on-1-yl-gruppe substituiert sein kann, durch eine Cycloalkoxygruppe mit 5 bis 8 Kohlenstoffatomen, durch eine Indanyloxy-, 1,2,3,4-Tetrahydronaphthyloxy-, Bicycloheptyloxy- oder Bicycloheptylmethoxygruppe, durch eine Alkanoyloxymethoxygruppe mit insgesamt 2 bis 5 Kohlenstoffatomen im Alkanoylteil, durch eine Alkoxycarbonyloxymethoxygruppe mit 1 bis 2 Kohlenstoffatomen im Alkylteil oder durch eine Cycloalkoxycarbonyloxymethoxygruppe mit 5 oder 6 Kohlenstoffatomen im Cycloalkylteil, in denen der Methoxyteil jeweils durch eine Alkylgruppe substituiert sein kann, substituiert ist, eine Verbindung der allgemeinen Formel

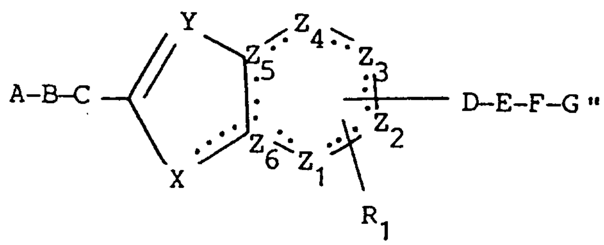 ,(X)

in der
A bis F, $R_1$, $Z_1$ bis $Z_6$, X und Y wie in den Ansprüchen 1 bis 5 definiert sind und
G" eine Carboxy- oder Alkoxycarbonylgruppe darstellt, mit einem Alkohol der allgemeinen Formel

$X_3$ - $R_c$ ,(XI)

in der
$X_3$ eine Hydroxygruppe oder eine nukleophile Austrittsgruppe und
$R_c$ eine Cinnamylgruppe oder eine Alkylgruppe mit 1 bis 6 Kohlenstoffatomen, in der ein Alkylrest mit 1 bis 5 Kohlenstoffatomen durch eine Phenylgruppe oder ein Alkylrest mit 1 bis 3 Kohlenstoffatomen in 1-, 2- oder 3-Stellung durch eine Naphthylgruppe, durch eine Cycloalkylgruppe mit 5 bis 6 Kohlenstoffatomen, durch eine Pyridylgruppe oder in 2- oder 3-Stellung durch eine Pyrrolidin-2-on-1-yl-gruppe substituiert sein kann, eine Cycloalkylgruppe mit 5 bis 8 Kohlenstoffatomen, eine Indanyl-, 1,2,3,4-Tetrahydronaphthyl-, Bicycloheptyl- oder Bicycloheptylmethylgruppe, eine Alkanoyloxymethylgruppe mit insgesamt 2 bis 5 Kohlenstoffatomen im Alkanoylteil, eine Alkoxycarbonyloxymethylgruppe mit 1 bis 2 Kohlenstoffatomen im Alkylteil oder eine Cycloalkoxycarbonyloxymethylgruppe mit 5 oder 6 Kohlenstoffatomen im Cycloalkylteil, in denen der Methylteil jeweils durch eine Alkylgruppe substituiert sein kann, darstellen, umgesetzt wird oder
h) zur Herstellung von Verbindungen der allgemeinen Formel I, in der A eine Guanidinoalkylgruppe oder eine Amidinogruppe darstellt, welche an das Stickstoffatom einer cyclischen Iminogruppe gebunden ist, eine Verbindung der allgemeinen Formel

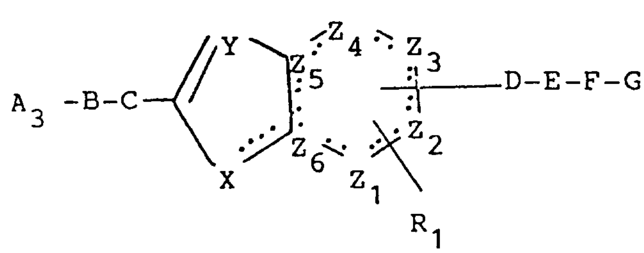 ,(XII)

in der
B bis G, $R_1$, $Z_1$ bis $Z_6$, X und Y wie in den Ansprüchen 1 bis 5 definiert sind und

$A_3$ eine Aminoalkylgruppe darstellt, oder eine Verbindung der allgemeinen Formel

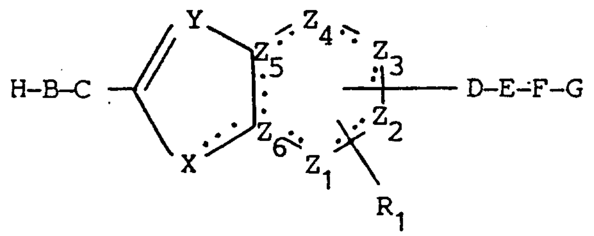

,(XIII)

in der

B bis G, $R_1$, $Z_1$ bis $Z_6$, X und Y mit der Maßgabe wie in den Ansprüchen 1 bis 5 definiert sind, daß der Rest B eine cyclische Iminogruppe enthält, mit einem S-Alkyl-isothioharnstoff umgesetzt wird oder

i) zur Herstellung von Verbindungen der allgemeinen Formel I, in der A eine an einen aromatischen Ring gebundene Guanidinogruppe darstellt, eine Verbindung der allgemeinen Formel

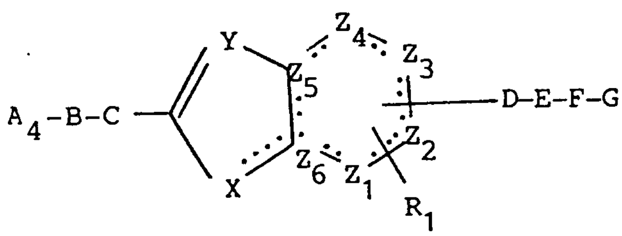

,(XIV)

in der

B bis G, $R_1$, $Z_1$ bis $Z_6$, X und Y mit der Maßgabe wie in den Ansprüchen 1 bis 5 definiert sind, daß B einen aromatischen oder heteroaromatischen Ring darstellt, und

$A_4$ eine Aminogruppe darstellt, mit Cyanamid oder dessen Säureadditionssalz umgesetzt wird oder

j) zur Herstellung von Verbindungen der allgemeinen Formel I, in der D eine Bindung und E eine $-NR_3$-CO-Gruppe darstellt, eine Verbindung der allgemeinen Formel

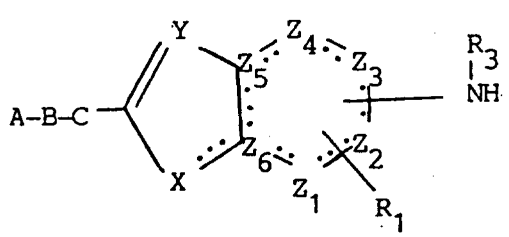

,(XV)

in der

A bis C, $R_1$, $R_3$, $Z_1$ bis $Z_6$, X und Y wie in den Ansprüchen 1 bis 5 definiert sind, mit einer Verbindung der allgemeinen Formel

$X_4$ - CO - F - G ,(XVI)

in der

F und G wie in den Ansprüchen 1 bis 5 definiert sind und

$X_4$ eine nucleophile Austrittsgruppe darstellt, oder mit deren reaktionsfähigen Derivaten umgesetzt wird oder

k) zur Herstellung von Verbindungen der allgemeinen Formel I, in der A eine Amino- oder Aminoalkylgruppe darstellt, eine Verbindung der allgemeinen Formel

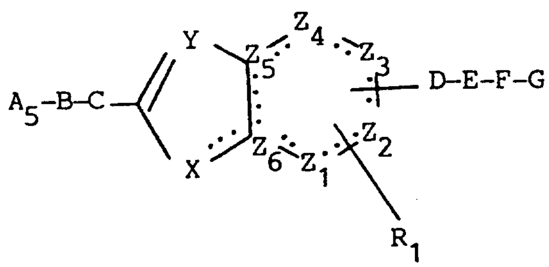

,(XVII)

in der
$R_1$, B bis G, X, Y und $Z_1$ bis $Z_6$ wie in den Ansprüchen 1 bis 5 definiert sind und
$A_5$ eine $H_2N$-CO-T-Gruppe darstellt, in der T eine Bindung oder eine Alkylengruppe mit 1 bis 4 Kohlenstoffatomen darstellt, mit einer Phenyljod(III)verbindung der allgemeinen Formel

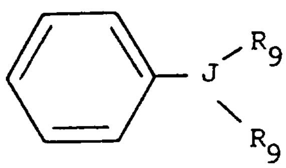

,(XVIII)

in der
$R_9$ jeweils den Acylrest einer organischen Carbonsäure darstellt, umgesetzt wird oder
1) zur Herstellung von Verbindungen der allgemeinen Formel I, in der A eine Aminoalkylgruppe, in der die Aminogruppe nicht an ein quartäres Kohlenstoffatom gebunden ist, oder eine Aminogruppe darstellt, die an eine CH- oder $CH_2$-Gruppe des Restes B oder C gebunden ist, darstellt, eine Verbindung der allgemeinen Formel

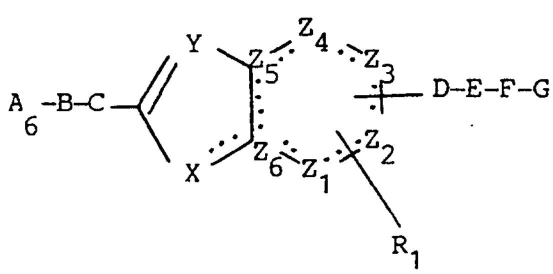

,(XIX)

in der
$R_1$, B bis G, X, Y und $Z_1$ bis $Z_6$ wie in den Ansprüchen 1 bis 5 definiert sind und
$A_5$ eine Hydroxyimino- oder Hydroxyiminoalkylgruppe mit 1 bis 4 Kohlenstoffatomen darstellt, reduziert wird oder
m) zur Herstellung von Verbindungen der allgemeinen Formel I, in der A eine durch eine Dialkylphosphorylgruppe mit jeweils 1 bis 3 Kohlenstoffatomen im Alkylteil substituierte Amino-, Aminoalkyl-, Amidino-, Guanidino- oder Guanidinoalkylgruppe darstellt, eine Verbindung der allgemeinen Formel

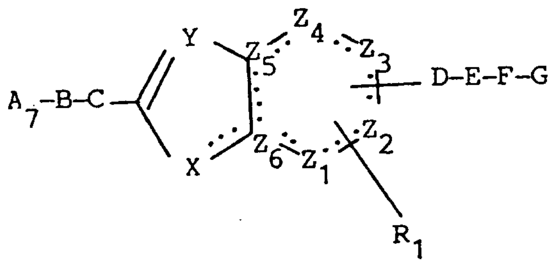

,(XX)

in der
$R_1$, B bis G, X, Y und $Z_1$ bis $Z_6$ wie in den Ansprüchen 1 bis 5 definiert sind und
$A_7$ eine Aminogruppe, eine geradkettige oder verzweigte Aminoalkylgruppe mit 1 bis 4 Kohlenstoffatomen, eine Amidino-, Guanidino- oder Guanidinoalkylgruppe mit 1 bis 3 Kohlenstoffatomen im Alkylteil darstellt, mit einer Verbindung der allgemeinen Formel

$X_5$ - $PO(OR_{10})_2$ ,(XXI)

in der
$R_{10}$ eine Alkylgruppe mit 1 bis 3 Kohlenstoffatomen und
$X_5$ eine nukleophile Austrittsgruppe darstellen, umgesetzt wird oder
n) zur Herstellung von Verbindungen der allgemeinen Formel I, in der E eine $-CO-NR_3$-Gruppe darstellt, eine Verbindung der allgemeinen Formel

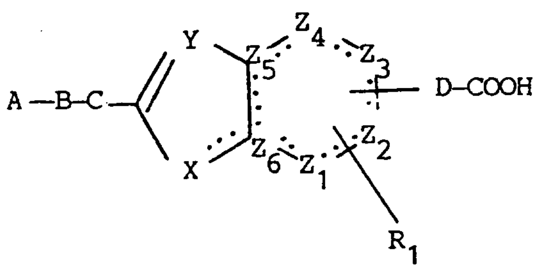

,(XXII)

in der
A bis D, X, Y und $Z_1$ bis $Z_6$ wie in den Ansprüchen 1 bis 5 definiert sind, mit einer Verbindung der allgemeinen Formel

$HNR_3$ - F - G ,(XXIII)

in der
F, G und $R_3$ wie in den Ansprüchen 1 bis 5 definiert sind, umgesetzt wird und

gewünschtenfalls eine so erhaltene Verbindung der allgemeinen Formel I, in der $R_1$ eine der in den Ansprüchen 1 bis 5 erwähnten Amino- oder Aminoalkylgruppen darstellt, mittels Alkylierung in eine entsprechende Alkylamino- oder Dialkylaminoverbindung der allgemeinen Formel I übergeführt wird und erforderlichenfalls anschließend ein während der Umsetzungen zum Schutze von reaktiven Gruppen verwendeter Schutzrest abgespalten wird und/oder

gewünschtenfalls eine so erhaltene Verbindung der allgemeinen Formel I in ihre Stereoisomere aufgetrennt wird und/oder

eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung

EP 92 11 5057
Seite 1

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 5) |
|---|---|---|---|
| A | EP-A-0 267 607 (MARION LABORATORIES INC)<br>--- | | C07D235/18<br>A61K31/415<br>C07D235/12<br>C07D235/22<br>C07D265/14<br>C07D277/66<br>C07D473/00<br>C07D473/08<br>C07D473/30<br>C07D473/34<br>C07D401/06<br>C07D403/06<br>C07D471/04<br>C07D487/04<br>C07D498/04 |
| A | EP-A-0 151 824 (JANSSEN PHARMACEUTICA N.V.)<br>--- | | |
| A | EP-A-0 215 736 (SANDOZ AG)<br>--- | | |
| A | EP-A-0 184 738 (DR. KARL THOMAE GMBH)<br>--- | | |
| A | EP-A-0 203 721 (THE WELLCOME FOUNDATION LIMITED)<br>--- | | |
| A | EP-A-0 258 191 (SANDOZ AG)<br>--- | | |
| A | US-A-3 516 999 (SHIONOGI & CO)<br>--- | | |
| A | US-A-4 563 526 (FORSYTH DENTAL INFIRMARY FOR CHILDREN)<br>--- | | |
| A | US-A-4 581 457 (AMERICAN HOME PRODUCTS CORPORATION)<br>---<br>-/-- | | RECHERCHIERTE SACHGEBIETE (Int. Cl. 5)<br>C07D<br>A61K |

## UNVOLLSTÄNDIGE RECHERCHE

**Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.**
**Vollständig recherchierte Patentansprüche:**
**Unvollständig recherchierte Patentansprüche:**
**Nicht recherchierte Patentansprüche:**
**Grund für die Beschränkung der Recherche:**

Siehe Ergänzungsblatt C

| Recherchenort | Abschlußdatum der Recherche | Prüfer |
|---|---|---|
| DEN HAAG | 14 DEZEMBER 1992 | DE BUYSER I.A.F. |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04E09)

Europäisches Patentamt

# EUROPÄISCHER TEILRECHERCHENBERICHT

Nummer der Anmeldung

EP 92 11 5057
Seite 2

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 5) |
|---|---|---|---|
| A | EP-A-0 287 971 (KYORIN PHARMACEUTICAL CO.,LTD.)<br>--- | | |
| A | EP-A-0 010 063 (CIBA-GEIGY AG)<br>--- | | |
| A | EP-A-0 172 631 (THE BOOTS COMPANY PLC)<br>--- | | |
| A | EP-A-0 412 898 (SCIENCE ET ORGANISATION)<br>--- | | |
| P,A | EP-A-0 470 543 (DR. KARL THOMAE GMBH)<br>----- | | |
| | | | RECHERCHIERTE SACHGEBIETE (Int. Cl. 5) |

EP 92 11 5057 -C-

UNVOLLSTÄNDIGE RECHERCHE

Vollständig recherchierte Patentanspruch : 5
Unvollständig recherchierte Patentansprüche : 1-4,6-10

Grund : Die Abfassung der Ansprüche ist nicht klar und knapp zu fassen (Art. 83-84, EPA) und enthalt eine so grosse Zahl Verbindungen dass eine vollständige Recherche auf ökonomischer Gründe nicht möglich ist (Siehe Richtlinien für die Prüfung im Europaïschen Patentamt, Teil B, Kapittel III,2 (Umfang der Recherche)). Geleitet durch den Sinn der Anfrage und die erfinderische Idee als offenbart in die Beschreibung der vorliegende Anfrage, ist die Recherche gegründet auf die Beispiele.